# EUROPEAN PATENT SPECIFICATION

(11) **EP 3 960 179 B1**
(45) Date of publication and mention of the grant of the patent: **18.03.2026**
(21) Application number: 20799419.5
(22) Date of filing: 29.04.2020
(51) Int. Cl.: A61K 31/506, A61K 31/513, A61P 11/14

(54) **METHOD FOR TREATING COUGH BY USING DIAMINOPYRIMIDINE COMPOUND**
VERFAHREN ZUR BEHANDLUNG VON HUSTEN UNTER VERWENDUNG EINER DIAMINOPYRIMIDINVERBINDUNG
MÉTHODE DE TRAITEMENT DE LA TOUX À L'AIDE D'UN COMPOSÉ DE DIAMINOPYRIMIDINE

(30) Priority: 30.04.2019 WO PCT/CN2019/085180
(43) Date of publication of application: 02.03.2022
(73) Proprietor: Beijing Tide Pharmaceutical Co., Ltd., Beijing 100176 (CN)
(72) Inventor: ZHAO, Yanping, Beijing 100176 (CN); WANG, Hongjun, Beijing 100176 (CN); HUANG, Huai, Beijing 100176 (CN); JIANG, Yuanyuan, Beijing 100176 (CN); LIANG, Huining, Beijing 100176 (CN); AN, Ran, Beijing 100176 (CN); LAN, Zhou, Beijing 100176 (CN); WANG, Jin, Beijing 100176 (CN); ZHOU, Liying, Beijing 100176 (CN); LIU, Yanan, Beijing 100176 (CN)
(74) Representative: Turner, Craig Robert
(86) International application number: PCT/CN2020/087686
(87) International publication number: WO 2020/221274

(56) References cited:
- WO-A1-2017/058645
- WO-A1-2019/085916
- CN-A- 1 930 135
- CN-A- 105 682 659
- CN-A- 108 779 119
- CN-A- 108 834 412
- RAYID ABDULQAWI ET AL: "P2X3 receptor antagonist (AF-219) in refractory chronic cough: a randomised, double-blind, placebo-controlled phase 2 study", THE LANCET, vol. 385, no. 9974, 28 March 2015 (2015-03-28), AMSTERDAM, NL, pages 1198 - 1205, XP055537425, ISSN: 0140-6736, DOI: 10.1016/S0140-6736(14)61255-1

## Description

### FIELD OF THE INVENTION

The present invention belongs to the field of biomedicine, and specifically relates to a method for treating, suppressing or alleviating cough or cough impulse, comprising administering to a subject in need thereof a therapeutically effective amount of a diaminopyrimidine compound or a pharmaceutically acceptable salt, ester, stereoisomer, polymorph, solvate, N-oxide, or isotopically labeled compound thereof.

### BACKGROUND OF THE INVENTION

Cough is a common respiratory symptom, which is caused by inflammation, foreign body, physical or chemical stimuli to tracheal and bronchial mucosae or pleurae. It manifests firstly as closure of glottis, contraction of respiratory muscles, and increase of intrapulmonic pressure, and then as opening of glottis, and air injection from the lung, usually accompanied with sound. A cough plays a protective role in clearing airway foreign bodies and secretions. If cough persists and turns from acute to chronic, it may bring a patient greater pain, such as chest distress, throat itching and/or gasping, etc.

The cause and recurrent attack of cough usually result from combined effects of various complicated factors. The factors causing cough typically include inhaled material, infection, food, climate change, etc. There still lack effective drugs for treating cough caused by various factors at present.

Document CN 108 779 119 discloses P2X3 and/or P2X2/3 diaminopyrimidine receptor antagonists for use in the treatment of cough and cough impulses associated with respiratory diseases.

### SUMMARY OF THE INVENTION

In one aspect, the present invention provides a compound of Formula (I) or a pharmaceutically acceptable salt, ester, stereoisomer, polymorph, solvate, N-oxide, or isotopically labeled compound thereof for use in a method for treating, suppressing or alleviating cough or cough impulse : 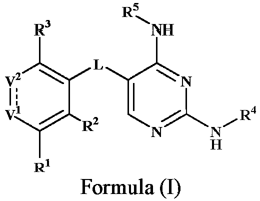 wherein
L is selected from the group consisting of C(=O), CRR', NR, O, S, S=O and S(=O)₂;
V¹ is selected from the group consisting of N, 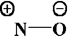 and NR;
V² is selected from the group consisting of CR⁶ and C(=O);
--- represents either a single bond or a double bond, provided that when --- is a single bond, V¹ is NR and V² is C(=O);
R and R' are each independently selected from the group consisting of H, halogen, C₁₋₆ alkyl, C₂₋₆ alkenyl, C₂₋₆ alkynyl, saturated or partially unsaturated C₃₋₁₀ cyclic hydrocarbyl, saturated or partially unsaturated 3- to 10-membered heterocyclyl, C₆₋₁₀ aryl, 5- to 14-membered heteroaryl and C₆₋₁₂ aralkyl, and at most 2 ring members in the cyclic hydrocarbyl and heterocyclyl are C(=O);
R¹, R², R³ and R⁶ are each independently selected from the group consisting of H, halogen, -CN, -NO₂, -NH₂, -OH, -SH, -Se-R, -Si(R)₃, C₁₋₆ alkyl, C₂₋₆ alkenyl, C₂₋₆ alkynyl, saturated or partially unsaturated C₃₋₁₀ cyclic hydrocarbyl, saturated or partially unsaturated 3- to 10-membered heterocyclyl, C₆₋₁₀ aryl, 5- to 14-membered heteroaryl, C₆₋₁₂ aralkyl, C₁₋₆ haloalkyl, -C(=O)R^{a}, -OC(=O)R^{a}, -C(=O)OR^{a}, -OR^{a}, -SR^{a}, -S(=O)R^{a}, -S(=O)₂R^{a}, -S(=O)₂NR^{a}R^{b}, -S(=O)(=NR)R^{a}, -NR^{a}R^{b}, -C(=O)NR^{a}R^{b}, -C(=S)NR^{a}R^{b}, -C(=NR)NR^{a}R^{b}, -NR^{a}-C(=O)R^{b}, -NR^{a}-C(=O)OR⁶, -NR^{a}-S(=O)₂-R^{b}, -NR^{a}-C(=O)-NR^{a}R^{b}, -C₁₋₆ alkylene-NR^{a}R^{b}, -C₁₋₆ alkylene-OR^{a}, -C₁₋₆ alkylene-C(=O)R, -C₁₋₆ alkenylene-OR^{a}, -O-C₁₋₆ alkylene-NR^{a}R^{b} and -P(=O)R^{a}R^{b};
R⁴ and R⁵ are each independently selected from the group consisting of H, -C(=O)OR^{a}, -NR^{a}R^{b}, -NR^{a}-C(=O)R^{b}, -NR^{a}-C(=O)OR^{b}, -C₁₋₆ alkylene-NR^{a}R^{b}, -C₁₋₆ alkylene-OR^{a}, -C₁₋₆ alkylene-O-C₁₋₆ alkylene-OR^{a}, C₁₋₆ alkyl, C₁₋₆ haloalkyl, C₂₋₆ alkenyl, C₂₋₆ alkynyl, saturated or partially unsaturated C₃₋₁₀ cyclic hydrocarbyl, saturated or partially unsaturated 3- to 10-membered heterocyclyl, C₆₋₁₀ aryl, 5- to 14-membered heteroaryl and C₆₋₁₂ aralkyl;
alternatively, R¹ and R⁴ together form -NHCH₂CH₂-O-CH₂CH₂-;
the above alkyl, alkylene, alkenyl, alkynyl, cyclic hydrocarbyl, heterocyclyl, aryl, heteroaryl and aralkyl, at each occurrence, are each optionally substituted with one or more substituents independently selected from the group consisting of halogen, hydroxyl, oxo, amino, cyano, nitro, -Si(R)₃, C₁₋₆ alkyl, saturated or partially unsaturated C₃₋₆ cyclic hydrocarbyl, saturated or partially unsaturated 3- to 10-membered heterocyclyl, C₆₋₁₀ aryl, 5- to 14-membered heteroaryl, C₆₋₁₂ aralkyl, -C(=O)R^{a}, -OC(=O)R^{a}, -C(=O)OR^{a}, -OR^{a}, -SR^{a}, -S(=O)R^{a}, -S(=O)₂R^{a}, -S(=O)₂NR^{a}R^{b}, -NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆ alkylene-NR^{a}R^{b}, -C₁₋₆ alkylene-OR^{a}, -C₁₋₆ alkenylene-OR^{a} and -O-C₁₋₆ alkylene-NR^{a}R^{b}, the alkyl, cyclic hydrocarbyl, heterocyclyl, aryl, heteroaryl and aralkyl are further optionally substituted with one or more substituents independently selected from the group consisting of halogen, hydroxyl, oxo, amino, cyano, nitro, -NR^{a}R^{b}, C₁₋₆ alkyl, -O-C₁₋₆ alkyl, saturated or partially unsaturated C₃₋₆ cyclic hydrocarbyl, saturated or partially unsaturated 3- to 10-membered heterocyclyl, C₆₋₁₀ aryl, 5- to 14-membered heteroaryl and C₆₋₁₂ aralkyl; and
R^{a} and R^{b}, at each occurrence, are each independently selected from the group consisting of H, -OH, C₁₋₆ alkyl, C₂₋₆ alkenyl, C₂₋₆ alkynyl, saturated or partially unsaturated C₃₋₁₀ cyclic hydrocarbyl, saturated or partially unsaturated 3- to 10-membered heterocyclyl, C₆₋₁₀ aryl, 5- to 14-membered heteroaryl and C₆₋₁₂ aralkyl; alternatively, R^{a} and R^{b} together with the atom to which they are attached form a 3- to 12-membered heterocycle or heteroaromatic ring, the above groups are further optionally substituted with one or more substituents independently selected from the group consisting of halogen, hydroxyl, oxo, amino, cyano, nitro, C₁₋₆ alkyl, -O-C₁₋₆ alkyl, saturated or partially unsaturated C₃₋₆ cyclic hydrocarbyl, saturated or partially unsaturated 3- to 10-membered heterocyclyl, C₆₋₁₀ aryl, 5- to 14-membered heteroaryl and C₆₋₁₂ aralkyl.

### BRIEF DESCRIPTION OF THE DRAWINGS

Figure 1 shows the antitussive effect of Compound 2 at doses of 23 mg/kg and 92 mg/kg.
Figure 2 shows the antitussive effect of compound 66 at doses of 46 mg/kg and 92 mg/kg.
Figure 3 shows the antitussive effect of compound 66 at doses of 5.1 mg/kg, 15.3 mg/kg and 46 mg/kg.
Figure 4 shows the effect of compound 66 on the P2X3-mediated current in 1321N1 cells stably transfected with P2X3
Figure 5 shows the concentration-inhibition curve of compound 66 on the P2X3-mediated current in 1321N1 cells stably transfected with P2X3.
Figure 6 shows the effect of compound 66 on the P2X3-mediated current in rat dorsal root ganglion cells
Figure 7 shows the concentration-inhibition curve of compound 66 on the P2X3-mediated current in rat dorsal root ganglion cells.

### DETAILED DESCRIPTION OF THE INVENTION

### Definition

Unless otherwise defined in the context, all technical and scientific terms used herein are intended to have the same meaning as commonly understood by a person skilled in the art. References to techniques employed herein are intended to refer to the techniques as commonly understood in the art, including variations on those techniques or substitutions of equivalent techniques which would be apparent to a person skilled in the art. While it is believed that the following terms will be readily understood by a person skilled in the art, the following definitions are nevertheless put forth to better illustrate the present invention

The terms "contain", "include", "comprise", "have", or "relate to", as well as other variations used herein are inclusive or open-ended, and do not exclude additional, unrecited elements or method steps.

As used herein, the term "alkylene" refers to a saturated divalent hydrocarbyl, preferably refers to a saturated divalent hydrocarbyl having 1, 2, 3, 4, 5 or 6 carbon atoms, e.g., methylene, ethylene, propylene or butylene.

As used herein, the term "alkyl" is defined as a linear or branched saturated aliphatic hydrocarbon. In some embodiments, alkyl has 1-12, e.g., 1-6, carbon atoms. For example, as used herein, the term "C₁₋₆ alkyl" refers to a linear or branched group having 1-6 carbon atoms (such as methyl, ethyl, n-propyl, isopropyl, n-butyl, isobutyl, sec-butyl, *tert-butyl,* n-pentyl, isopentyl, neopentyl, or n-hexyl), which is optionally substituted with one or more (e.g., 1 to 3) suitable substituents such as halogen (in which case the group may be referred to as "haloalkyl") (e.g., CH₂F, CHF₂, CF₃, CCl₃, C₂F₅, C₂Cl₅, CH₂CF₃, CH₂Cl or -CH₂CH₂CF₃ *etc.).* The term "C₁₋₄ alkyl" refers to a linear or branched aliphatic hydrocarbon chain having 1-4 carbon atoms (i.e., methyl, ethyl, n-propyl, isopropyl, n-butyl, isobutyl, sec-butyl or *tert*-butyl)*.*

As used herein, the term "alkenyl" refers to a linear or branched monovalent hydrocarbyl having a double bond and 2-6 carbon atoms ("C₂₋₆ alkenyl"). The alkenyl is e.g., vinyl, 1-propenyl, 2-propenyl, 2-butenyl, 3-butenyl, 2-pentenyl, 3-pentenyl, 4-pentenyl, 2-hexenyl, 3-hexenyl, 4-hexenyl, 5-hexenyl, 2-methyl-2-propenyl and 4-methyl-3-pentenyl. When the compound of the present invention contains an alkenylene group, the compound may exist as the pure E (entgegen) form, the pure Z (zusammen) form, or any mixture thereof.

As used herein, the term "alkynyl" refers to a monovalent hydrocarbyl containing one or more triple bond, and preferably having 2, 3, 4, 5 or 6 carbon atoms, e.g., ethynyl or propynyl.

As used herein, the term "cycloalkyl" refers to a saturated monocyclic or polycyclic (e.g., bicyclic) hydrocarbon ring (e.g., monocyclic, such as cyclopropyl, cyclobutyl, cyclopentyl, cyclohexyl, cycloheptyl, cyclooctyl, or cyclononyl, or bicyclic, including spiro, fused or bridged cyclic system (such as bicyclo[1.1.1]pentyl, bicyclo[2.2.1]heptyl, bicyclo[3.2.1]octyl or bicyclo[5.2.0]nonyl, or decahydronaphthalene *etc*.))*,* which is optionally substituted with one or more (e.g., 1 to 3) suitable substituents. The cycloalkyl has 3 to 15 carbon atoms. For example, the term "C₃₋₆ cycloalkyl" refers to a saturated monocyclic or polycyclic (e.g., bicyclic) hydrocarbon ring having 3 to 6 ring forming carbon atoms (e.g., cyclopropyl, cyclobutyl, cyclopentyl, or cyclohexyl), which is optionally substituted with one or more (e.g., 1 to 3) suitable substituents, e.g., methyl substituted cyclopropyl.

As used herein, the terms "cyclic hydrocarbylene", "cyclic hydrocarbyl" and "hydrocarbon ring" refer to a saturated (i.e., "cycloalkylene" and "cycloalkyl") or unsaturated (i.e., having one or more double and/or triple bonds in the ring) monocyclic or polycyclic hydrocarbon ring having e.g., 3-10 (suitably having 3-8, and more suitably having 3-6) ring carbon atoms, including but not limited to cyclopropyl(ene) (ring), cyclobutyl(ene) (ring), cyclopentyl(ene) (ring), cyclohexyl(ene) (ring), cycloheptyl(ene) (ring), cyclooctyl(ene) (ring), cyclononyl(ene) (ring), cyclohexenyl(ene) (ring), and the like.

As used herein, the terms "heterocyclyl", "heterocyclylene" and "heterocycle" refer to a saturated (i.e., heterocycloalkyl) or partially unsaturated (i.e., having one or more double and/or triple bonds in the ring) cyclic group having e.g., 3-10 (suitably having 3-8, and more suitably having 3-6) ring atoms, wherein at least one ring atom is a heteroatom selected from the group consisting of N, O and S, and the remaining ring atoms are C. For example, "3- to 10-membered heterocyclyl(ene)" of "3- to 10-membered heterocycle" refers to saturated or partially unsaturated heterocyclyl(ene) or heterocycle having 2-9 (e.g., 2, 3, 4, 5, 6, 7, 8 or 9) ring carbon atoms and one or more (e.g., 1, 2, 3, or 4) heteroatoms independently selected from the group consisting of N, O and S. Examples of heterocyclylene, heterocyclyl and heterocycle include, but are not limited to oxiranyl(ene), aziridinyl(ene), azetidinyl(ene), oxetanyl(ene), tetrahydrofuranyl(ene), dioxolinyl(ene), pyrrolidinyl(ene), pyrrolidonyl(ene), imidazolidinyl(ene), pyrazolidinyl(ene), pyrrolinyl(ene), tetrahydropyranyl(ene), piperidinyl(ene), morpholinyl(ene), dithianyl(ene), thiomorpholinyl(ene), piperazinyl(ene) or trithianyl(ene). Said group also encompasses a bicyclic system, including a spiro, fused, or bridged system (e.g., 8-azaspiro[4.5]decane, 3,9-diazaspiro[5.5]undecane, 2-azabicyclo[2.2.2]octane, *etc.).* Heterocyclylene, heterocyclyl and heterocycle may optionally be substituted with one or more (e.g., 1, 2, 3 or 4) suitable substituents.

As used herein, the terms "aryl(ene)" and "aromatic ring" refer to an all-carbon monocyclic or fused-ring polycyclic aromatic group having a conjugated π electron system. For example, as used herein, the terms "C₆₋₁₀ aryl(ene)" and "C₆₋₁₀ aromatic ring" refer to an aromatic group containing 6 to 10 carbon atoms, such as phenyl(ene) (benzene ring) or naphthyl(ene) (naphthalene ring). Aryl(ene) or aromatic ring is optionally substituted with one or more (such as 1 to 3) suitable substituents (e.g., halogen, -OH, -CN, -NO₂, and C₁₋₆ alkyl, *etc.).*

As used herein, the terms "heteroaryl(ene)" and "heteroaromatic ring" refer to a monocyclic, bicyclic or tricyclic aromatic ring system having 5, 6, 8, 9, 10, 11, 12, 13 or 14 ring atoms, particularly 1 or 2 or 3 or 4 or 5 or 6 or 9 or 10 carbon atoms, and containing at least one heteroatom (such as O, N, or S), which can be same to different. Moreover, in each case, it can be benzo-fused. In particular, "heteroaryl(ene)" or "heteroaromatic ring" is selected from the group consisting of thienyl(ene), furyl(ene), pyrrolyl(ene), oxazolyl(ene), thiazolyl(ene), imidazolyl(ene), pyrazolyl(ene), isoxazolyl(ene), isothiazolyl(ene), oxadiazolyl(ene), triazolyl(ene), thiadiazolyl(ene) *etc.,* and benzo derivatives thereof; or pyridinyl(ene), pyridazinyl(ene), pyrimidinyl(ene), pyrazinyl(ene), triazinyl(ene), etc., and benzo derivatives thereof.

As used herein, the term "aralkyl" preferably means aryl or heteroaryl substituted alkyl, wherein aryl, heteroaryl and alkyl are as defined herein. Normally, the aryl group may have 6-14 carbon atoms, the heteroaryl group may have 5-14 ring atoms, and the alkyl group may have 1-6 carbon atoms. Exemplary aralkyl group includes, but is not limited to, benzyl, phenylethyl, phenylpropyl, phenylbutyl.

As used herein, the term "halo" or "halogen" are defined to include F, Cl, Br, or I.

As used herein, the term "nitrogen containing heterocycle" refers to a saturated or unsaturated monocyclic or bicyclic group having 2, 3, 4, 5, 6, 7, 8, 9, 10, 11, 12 or 13 carbon atoms and at least one nitrogen atom in the ring, which may further optionally comprise one or more (e.g., one, two, three or four) ring members selected from the group consisting of N, O, C=O, S, S=O and S(=O)₂. The nitrogen containing heterocycle is attached to the rest of the molecule through the nitrogen atom and any other ring atom in said nitrogen containing heterocycle. The nitrogen containing heterocycle is optionally benzo-fused, and is preferably attached to the rest of the molecule through the nitrogen atom in said nitrogen containing heterocycle and any carbon atom in the fused benzene ring.

The term "substituted" means that one or more (e.g., one, two, three, or four) hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds.

If a substituent is described as being "optionally substituted", the substituent may be either (1) not substituted, or (2) substituted. If a carbon of a substituent is described as being optionally substituted with one or more of a list of substituents, one or more of the hydrogens on the carbon (to the extent there are any) may separately and/or together be replaced with an independently selected optional substituent. If a nitrogen of a substituent is described as being optionally substituted with one or more from a list of substituents, one or more of the hydrogens on the nitrogen (to the extent there are any) may each be replaced with an independently selected optional substituent.

If substituents are described as being "independently selected" from a group, each substituent is selected independent of the other(s). Each substituent therefore may be identical to or different from the other substituent(s).

As used herein, the term "one or more" means one or more than one (e.g., 2, 3, 4, 5 or 10) as reasonable.

As used herein, unless specified, the point of attachment of a substituent can be from any suitable position of the substituent.

When a bond to a substituent is shown to cross a bond connecting two atoms in a ring, then such substituent may be bonded to any of the ring-forming atoms in that ring that are substitutable.

The present invention also includes all pharmaceutically acceptable isotopically labeled compounds, which are identical to those of the present invention except that one or more atoms are replaced by an atom having the same atomic number, but an atomic mass or mass number different from the atomic mass or mass number which predominates in nature. Examples of isotopes suitable for inclusion in the compound of the present invention include, but are not limited to, isotopes of hydrogen, such as ²H, ³H; carbon, such as ¹¹C, ¹³C, and ¹⁴C; chlorine, such as ³⁶Cl; fluorine, such as ¹⁸F; iodine, such as ¹²³I and ¹²⁵I; nitrogen, such as ¹³N and ¹⁵N; oxygen, such as ¹⁵O, ¹⁷O, and ¹⁸O; phosphorus, such as ³²P; and sulfur, such as ³⁵S. Certain isotopically labeled compounds of the present invention, for example those incorporating a radioactive isotope, are useful in drug and/or substrate tissue distribution studies (e.g., assays). The radioactive isotopes tritium, i.e., ³H, and carbon-14, i.e., ¹⁴C, are particularly useful for this purpose in view of their ease of incorporation and ready means of detection. Substitution with positron-emitting isotopes, such as ¹¹C, ¹⁸F, ¹⁵O and ¹³N, can be useful in positron emission tomography (PET) studies for examining substrate receptor occupancy. Isotopically labeled compounds of the present invention can generally be prepared by processes analogous to those described in the accompanying Schemes and/or in the Examples and Preparations, by using an appropriate isotopically labeled reagent in place of the non-labeled reagent previously employed. Pharmaceutically acceptable solvates in accordance with the invention include those wherein the solvent of crystallization may be isotopically substituted, e.g., D₂O, acetone-*d₆*, or DMSO-*d₆*.

The term "stereoisomer" refers to isomers with at least one asymmetric center. A compound having one or more (e.g., one, two, three or four) asymmetric centers can give rise to a racemic mixture, single enantiomer, diastereomer mixture and individual diastereomer. Certain individual molecules may exist as geometric isomers (cis/trans). Similarly, the compound of the present invention may exist as a mixture of two or more structurally different forms in rapid equilibrium (generally referred to as tautomer). Typical examples of a tautomer include a keto-enol tautomer, phenol-keto tautomer, nitroso-oxime tautomer, imine-enamine tautomer and the like. It is to be understood that all such isomers and mixtures thereof in any proportion (such as 60%, 65%, 70%, 75%, 80%, 85%, 90%, 95%, 96%, 97%, 98%, and 99%) are encompassed within the scope of the present invention.

The chemical bonds of the compound of the present invention may be depicted herein using a solid line ( - ), a solid wedge ( 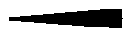 ), or a dotted wedge ( 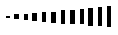 ). The use of a solid line to depict bonds to asymmetric carbon atoms is meant to indicate that all possible stereoisomers (e.g., specific enantiomers, racemic mixtures, *etc.)* at that carbon atom are included. The use of either a solid or dotted wedge to depict bonds to asymmetric carbon atoms is meant to indicate that the stereoisomer shown is present. When present in racemic compounds, solid and dotted wedges are used to define relative stereochemistry, rather than absolute stereochemistry. Unless stated otherwise, it is intended that the compound of the present invention can exist as stereoisomers, which include cis and trans isomers, optical isomers such as R and S enantiomers, diastereomers, geometric isomers, rotational isomers, conformational isomers, atropisomers, and mixtures thereof. The compound of the present invention may exhibit more than one type of isomerism, and consist of mixtures thereof (such as racemates and diastereomeric pairs).

The present invention includes all possible crystalline forms or polymorphs of the compound of the present invention, either as a single polymorph, or as a mixture of more than one polymorphs, in any ratio.

It also should be understood that, certain compounds of the present invention can be used for the treatment in a free form, or where appropriate, in a form of a pharmaceutically acceptable derivative. In the present invention, the pharmaceutically acceptable derivative includes, but is not limited to a pharmaceutically acceptable salt, ester, solvate, N-oxide, metabolite or prodrug, which can directly or indirectly provide the compound of the present invention or a metabolite or residue thereof after being administered to a patient in need thereof. Therefore, "the compound of the present invention" mentioned herein also means to encompass various derivative forms of the compound as mentioned above.

A pharmaceutically acceptable salt of the compound of the present invention includes an acid addition salt and a base addition salt thereof.

A suitable acid addition salt is formed from an acid which forms a pharmaceutically acceptable salt. Specific examples include acetate, adipate, aspartate, benzoate, besylate, bicarbonate/carbonate, bisulfate/sulfate, borate, camphorsulfonate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulfate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts.

A suitable base addition salt is formed from a base which forms a pharmaceutically acceptable salt. Specific examples include aluminum, arginine, benzathine, calcium, choline, diethylamine, diolamine, glycine, lysine, magnesium, meglumine, olamine, potassium, sodium, tromethamine and zinc salts.

For a review on suitable salts, see "Handbook of Pharmaceutical Salts: Properties, Selection, and Use" by Stahl and Wermuth (Wiley-VCH, 2002). The method for preparing a pharmaceutically acceptable salt of the compound of the present invention is known to a person skilled in the art.

As used herein, the term "ester" refers to those derived from the compounds of the various formulae in the present application, which include physiologically-hydrolyzable esters (which may be hydrolyzed under physiological conditions to release the compounds of the present invention in the form of free acids or alcohols). The compound of the present invention itself may be an ester as well.

The compound of the present invention can exist as a solvate (preferably a hydrate), wherein the compound of the present invention contains a polar solvent, in particular water, methanol or ethanol for example, as a structural element of the crystal lattice of the compound. The amount of the polar solvent, in particular water, may exist in a stoichiometric or non-stoichiometric ratio.

As can be appreciated by a person skilled in the art, not all nitrogen containing heterocycles can form N-oxides since the nitrogen requires an available lone-pair electron for oxidation to the oxide; a person skilled in the art will recognize those nitrogen containing heterocycles which can form N-oxides. A person skilled in the art will also recognize that tertiary amines can form N-oxides. Synthetic methods for the preparation of N-oxides of heterocycles and tertiary amines are well known to a person skilled in the art, and they include the oxidation of heterocycles and tertiary amines with peroxy acids such as peracetic acid and m-chloroperbenzoic acid (MCPBA), hydrogen peroxide, alkyl hydroperoxides such as *tert-butyl* hydroperoxide, sodium perborate, and dioxiranes such as dimethyldioxirane. These methods for the preparation of N-oxides have been extensively described and reviewed in literatures, see e.g., T. L. Gilchrist, Comprehensive Organic Synthesis, vol. 7, pp 748-750; A. R. Katritzky and A. J. Boulton, Eds., Academic Press; and G. W. H. Cheeseman and E. S. G. Werstiuk, Advances in Heterocyclic Chemistry, vol. 22, pp 390-392, A. R. Katritzky and A. J. Boulton, Eds., Academic Press.

The present invention further encompasses the compound of the present invention having a protecting group. During any of the processes for preparation of the compound of the present invention, it may be necessary and/or desirable to protect sensitive or reactive groups on any of the molecules concerned, thereby resulting in the chemically protected form of the compound of the present invention. This may be achieved by means of conventional protecting groups, e.g., those described in T.W. Greene & P.G.M. Wuts, Protective Groups in Organic Synthesis, John Wiley & Sons, 1991, which is incorporated herein by reference. The protecting groups may be removed at a convenient subsequent stage using methods known from the art.

The term "about" refers to a range within ±10%, preferably within ±5%, and more preferably within ±2% of the specified value.

As used herein, the term "effective amount" refers to the amount of a compound being administered which will relieve to some extent one or more of the symptoms of the disorder being treated.

Unless otherwise indicated, the term "treating" or "treatment", as used herein, means reversing, alleviating, inhibiting the progress of, or preventing the disorder or condition to which such term applies, or one or more symptoms of such disorder or condition.

As used herein, the "treatment of cough" or "treating cough" means any therapy that reduces the number and/or the severity of cough. Preferably, it means a reduction in the number of coughs, i.e., a direct antitussive effect that reduces the body's urge to cough. In preferred embodiments, compared with the control, the method of the present invention reduces the number of coughs by 40%-95%, preferably 50%-90% in a same period of time.

As used herein, the term "subject" includes a human or non-human animal. An exemplary human subject includes a human subject having a disease (such as one described herein) (referred to as a patient), or a normal subject. The term "non-human animal" as used herein includes all vertebrates, such as non-mammals (e.g., birds, amphibians, reptiles) and mammals, such as non-human primates, livestock and/or domesticated animals (such as sheep, dog, cat, cow, pig and the like).

### MODE OF CARRYING OUT THE INVENTION

In some embodiments, the present invention provides a compound of Formula (I) or a pharmaceutically acceptable salt, ester, stereoisomer, polymorph, solvate, N-oxide, or isotopically labeled compound thereof for use in a method for treating, suppressing or alleviating cough or cough impulse : 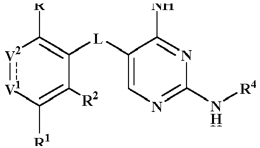 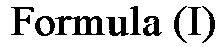 wherein:
L is selected from the group consisting of C(=O), CRR', NR, O, S, S=O and S(=O)₂;
V¹ is selected from the group consisting of N, 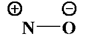 and NR;
V² is selected from the group consisting of CR⁶ and C(=O);
--- represents either a single bond or a double bond, provided that when --- is a single bond, V¹ is NR and V² is C(=O);
R and R' are each independently selected from the group consisting of H, halogen, C₁₋₆ alkyl, C₂₋₆ alkenyl, C₂₋₆ alkynyl, saturated or partially unsaturated C₃₋₁₀ cyclic hydrocarbyl, saturated or partially unsaturated 3- to 10-membered heterocyclyl, C₆₋₁₀ aryl, 5- to 14-membered heteroaryl and C₆₋₁₂ aralkyl, and at most 2 ring members in the cyclic hydrocarbyl and heterocyclyl are C(=O);
R¹, R², R³ and R⁶ are each independently selected from the group consisting of H, halogen, -CN, -NO₂, -NH₂, -OH, -SH, -Se-R, -Si(R)₃, C₁₋₆ alkyl, C₁₋₆ alkenyl, C₂₋₆ alkynyl, saturated or partially unsaturated C₃₋₁₀ cyclic hydrocarbyl, saturated or partially unsaturated 3- to 10-membered heterocyclyl, C₆₋₁₀ aryl, 5- to 14-membered heteroaryl, C₆₋₁₂ aralkyl, C₁₋₆ haloalkyl, -C(=O)R^{a}, -OC(=O)R^{a}, -C(=O)OR^{a}, -OR^{a}, -SR^{a}, -S(=O)R^{a}, -S(=O)₂R^{a}, -S(=O)₂NR^{a}R^{b}, -S(=O)(=NR)R^{a}, -NR^{a}R^{b}, -C(=O)NR^{a}R^{b}, -C(=S)NR^{a}R^{b}, -C(=NR)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆ alkylene-NR^{a}R^{b}, -C₁₋₆ alkylene-OR^{a}, -C₁₋₆ alkylene-C(=O)R, -C₁₋₆ alkenylene-OR^{a}, -O-C₁₋₆ alkylene-NR^{a}R^{b} and -P(=O)R^{a}R^{b};
R⁴ and R⁵ are each independently selected from the group consisting of H, -C(=O)OR^{a}, -NR^{a}R^{b}, -NR^{a}-C(=O)R^{b}, -NR^{a}-C(=O)OR^{b}, -C₁₋₆ alkylene-NR^{a}R^{b}, -C₁₋₆ alkylene-OR^{a}, -C₁₋₆ alkylene-O-C₁₋₆ alkylene-OR^{a}, C₁₋₆ alkyl, C₁₋₆ haloalkyl, C₂₋₆ alkenyl, C₂₋₆ alkynyl, saturated or partially unsaturated C₃₋₁₀ cyclic hydrocarbyl, saturated or partially unsaturated 3- to 10-membered heterocyclyl, C₆₋₁₀ aryl, 5- to 14-membered heteroaryl and C₆₋₁₂ aralkyl;
alternatively, R¹ and R⁴ together form -NHCH₂CH₂-O-CH₂CH₂-;
the above alkyl, alkylene, alkenyl, alkynyl, cyclic hydrocarbyl, heterocyclyl, aryl, heteroaryl and aralkyl, at each occurrence, are each optionally substituted with one or more substituents independently selected from the group consisting of halogen, hydroxyl, oxo, amino, cyano, nitro, -Si(R)₃, C₁₋₆ alkyl, saturated or partially unsaturated C₃₋₆ cyclic hydrocarbyl, saturated or partially unsaturated 3- to 10-membered heterocyclyl, C₆₋₁₀ aryl, 5- to 14-membered heteroaryl, C₆₋₁₂ aralkyl, -C(=O)R^{a}, -OC(=O)R^{a}, -C(=O)OR^{a}, -OR^{a}, -SR^{a}, -S(=O)R^{a}, -S(=O)₂R^{a}, -S(=O)₂NR^{a}R^{b}, -NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆ alkylene-NR^{a}R^{b}, -C₁₋₆ alkylene-OR^{a}, -C₁₋₆ alkenylene-OR^{a} and -O-C₁₋₆ alkylene-NR^{a}R^{b}, the alkyl, cyclic hydrocarbyl, heterocyclyl, aryl, heteroaryl and aralkyl are further optionally substituted with one or more substituents independently selected from the group consisting of halogen, hydroxyl, oxo, amino, cyano, nitro, -NR^{a}R^{b}, C₁₋₆ alkyl, -O-C₁₋₆ alkyl, saturated or partially unsaturated C₃₋₆ cyclic hydrocarbyl, saturated or partially unsaturated 3- to 10-membered heterocyclyl, C₆₋₁₀ aryl, 5- to 14-membered heteroaryl and C₆₋₁₂ aralkyl; and
R^{a} and R^{b}, at each occurrence, are each independently selected from the group consisting of H, -OH, C₁₋₆ alkyl, C₂₋₆ alkenyl, C₂₋₆ alkynyl, saturated or partially unsaturated C₃₋₁₀ cyclic hydrocarbyl, saturated or partially unsaturated 3- to 10-membered heterocyclyl, C₆₋₁₀ aryl, 5- to 14-membered heteroaryl and C₆₋₁₂ aralkyl; alternatively, R^{a} and R^{b} together with the atom to which they are attached form a 3- to 12-membered heterocycle or heteroaromatic ring, the above groups are further optionally substituted with one or more substituents independently selected from the group consisting of halogen, hydroxyl, oxo, amino, cyano, nitro, C₁₋₆ alkyl, -O-C₁₋₆ alkyl, saturated or partially unsaturated C₃₋₆ cyclic hydrocarbyl, saturated or partially unsaturated 3- to 10-membered heterocyclyl, C₆₋₁₀ aryl, 5- to 14-membered heteroaryl and C₆₋₁₂ aralkyl.

In preferred embodiments, L is selected from the group consisting of CH₂, O, S and NH.

In preferred embodiments, V¹ is selected from the group consisting of N, 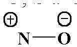 and NCH₃.

In preferred embodiments, V² is selected from the group consisting of CH, C-NHCH₃, C-OCH₃, C-F and C(=O).

In preferred embodiments, R^{a} and R^{b}, at each occurrence, are each independently selected from the group consisting of H, -OH, methyl, ethyl, n-propyl, isopropyl, cyclopropyl, phenyl, benzyl, methoxy and ethoxy; alternatively, R^{a} and R^{b} together with the atom to which they are attached form a 5- to 8-membered heterocycle or heteroaromatic ring.

In preferred embodiments, R¹, R², R³ and R⁶ are each independently selected from the group consisting of H, F, Cl, Br, I, -CN, -NH₂, -OH, -SH, -Se-CH₃, -Si(CH₃)₃, -CH₂NH₂, -CH₂NHCH₃, -CH₂N(CH₃)₂, methyl, ethyl, n-propyl, isopropyl, n-butyl, isobutyl, *tert-butyl,* cyclopropyl, cyclobutyl, cyclopentyl, cyclohexyl, vinyl, propenyl, allyl, ethynyl, propynyl, trifluoromethyl, acetyl, -C(=O)OH, -C(=O)NH₂, -C(=S)NH₂, -C(=NH)NH₂, -NHCH₃, -NHCH₂CH₃, -NHCH₂CF₃, -N(CH₃)₂, -N(CH₃)(C₂H₅), -N(C₂H₅)₂, -NHCH₂CH₂OH, -NH-C(=O)CH₃, -NH-C(=O)CH=CH₂, methoxy, ethoxy, propoxy, phenyl, -NH-C(=O)-NH₂, -NH-C(=O)OCH₃, -SCH₃, -SCH₂CH₃, -SC(CH₃)₃, -SBn, -S(=O)CH₃, -S(=O)Bn, -S(=O)₂CH₃, -S(=O)₂Bn, -S(=O)₂NH₂, -S(=O)₂NHCH₃, -S(=O)₂N(CH₃)₂, -S(=O)(=NH)CH₃, -P(=O)(CH₃)₂, -P(=O)(C₂H₅)₂,

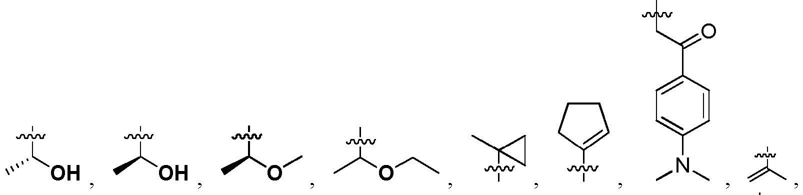

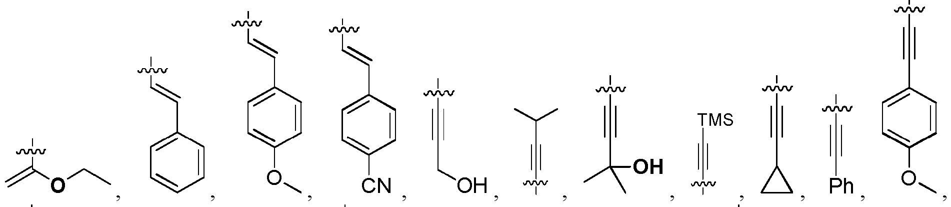

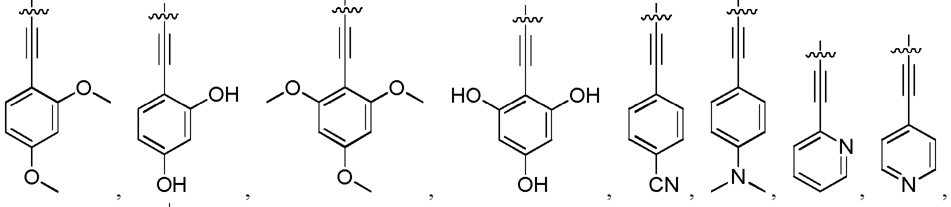

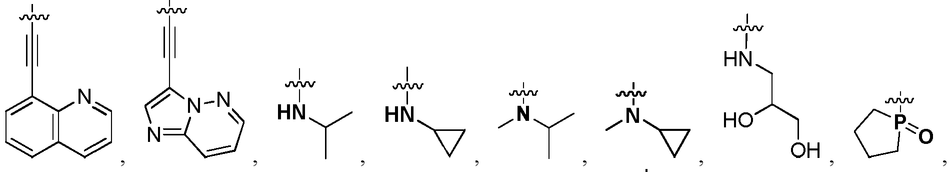

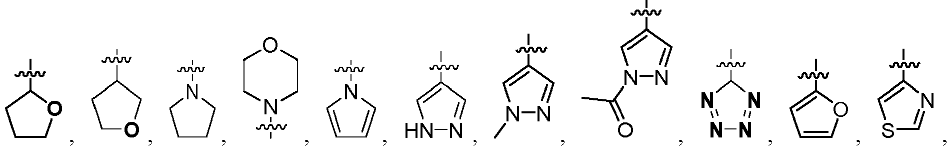

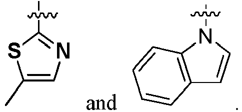

In preferred embodiments, R⁴ and R⁵ are each independently selected from the group consisting of H, -C(=O)OC(CH₃)₃, -NH₂, -NHCH₃, -NHPh, -NHC(=O)CH₃, -NHBoc, methyl, ethyl, -CH₂CF₃, n-propyl, isopropyl, n-butyl, isobutyl, *tert-butyl,* cyclopropyl, cyclobutyl, cyclopentyl, cyclohexyl, phenyl, benzyl, 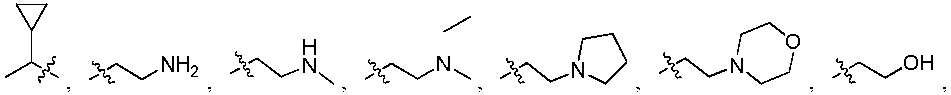 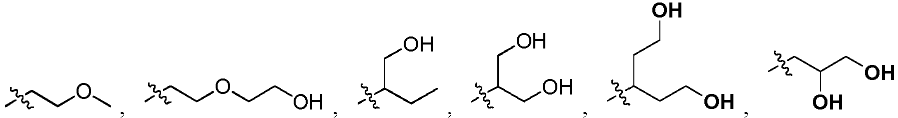 and

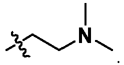

In preferred embodiments, the compound of Formula (I) has the structure of any of the following formulae: 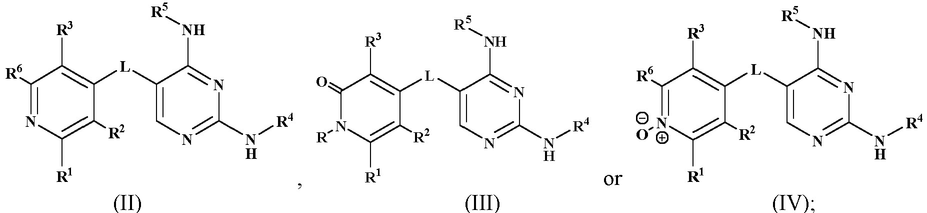
preferably has the structure of any of the following formulae: 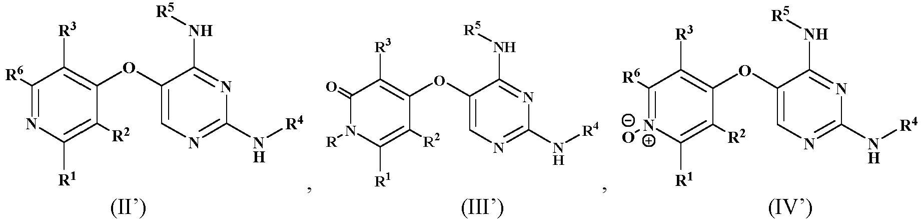 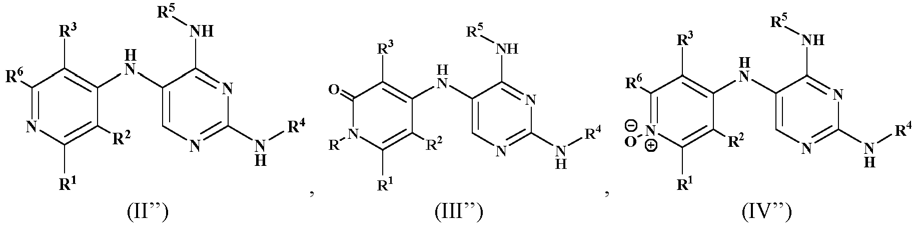 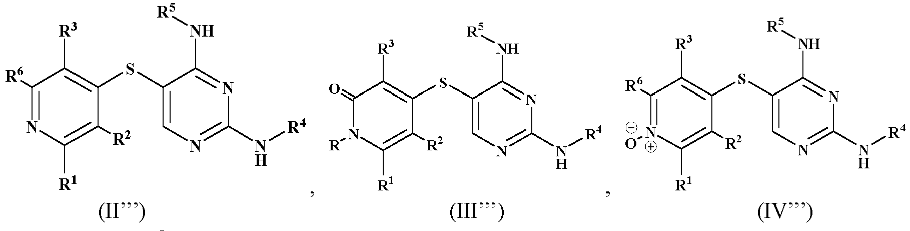 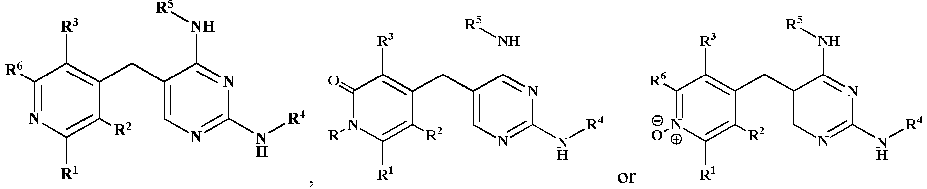 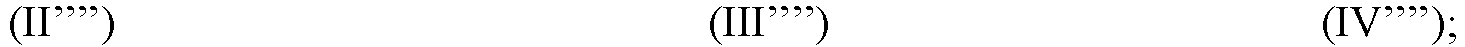
more preferably, the compound of Formula (I) has the structure of any of the following formulae: 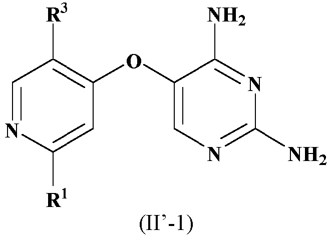
wherein:
   R¹ is selected from the group consisting of F, Cl, Br, I and C₂₋₆ alkynyl, preferably Br or ethynyl; and
   R³ is C₁₋₆ alkyl, preferably isopropyl.

The technical solution obtained by any combination of the various embodiments is encompassed by the invention.

In preferred embodiments, the compound of Formula (I) has the following structure:

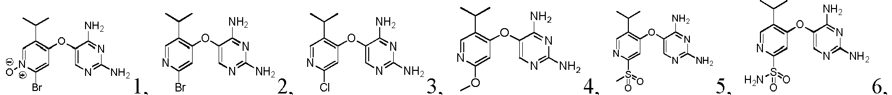

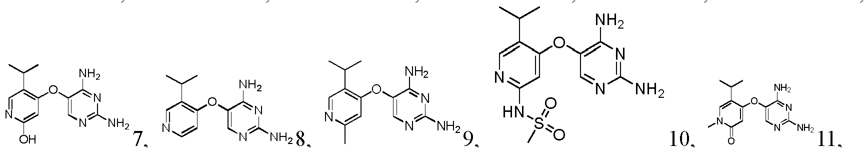

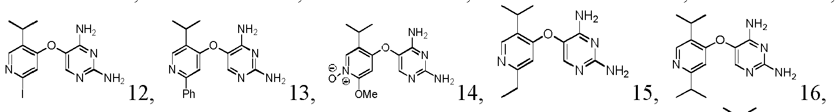

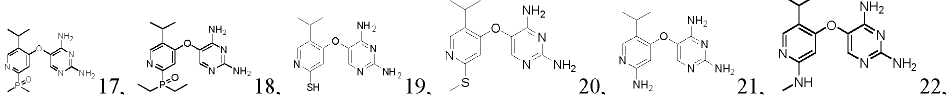

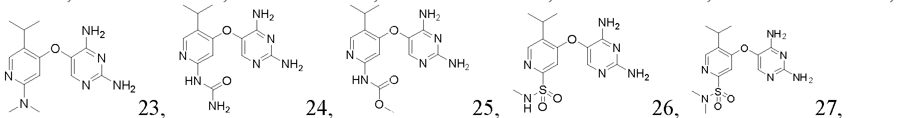

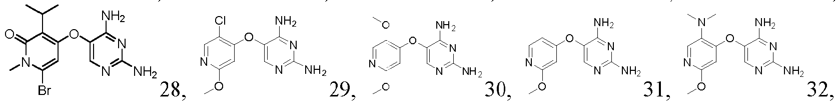

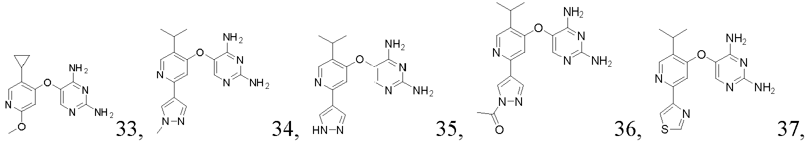

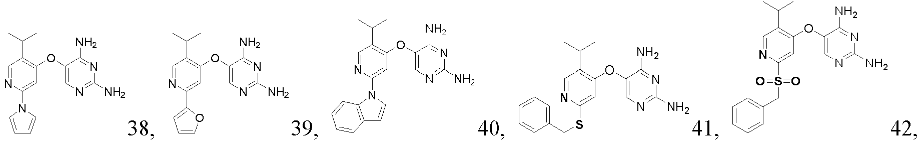

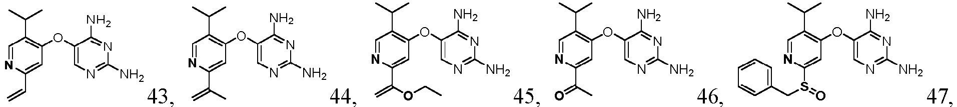

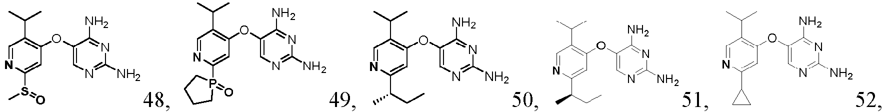

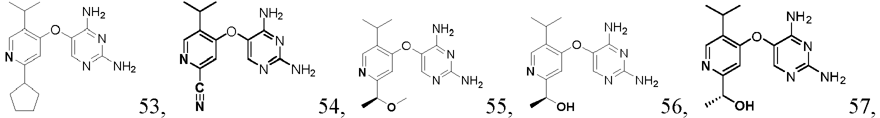

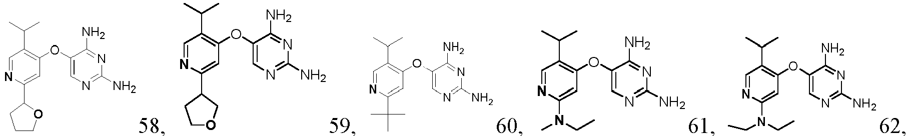

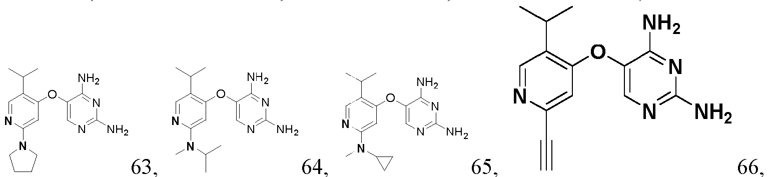

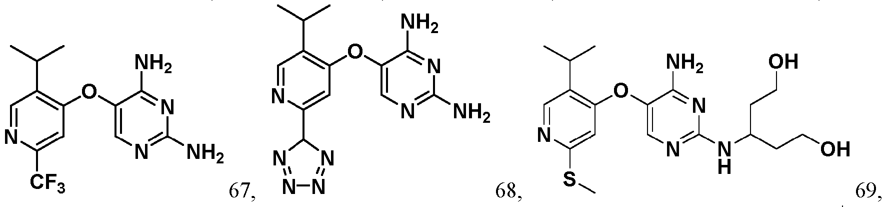

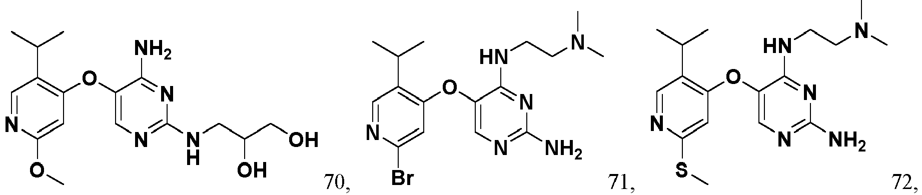

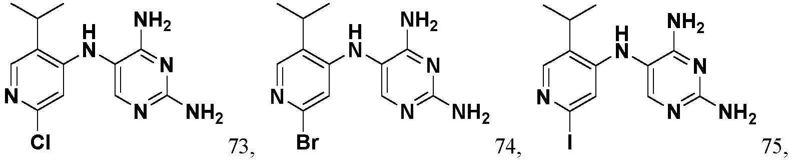

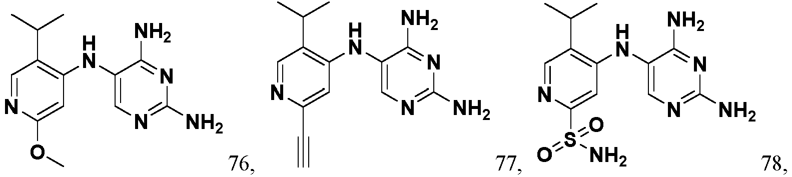

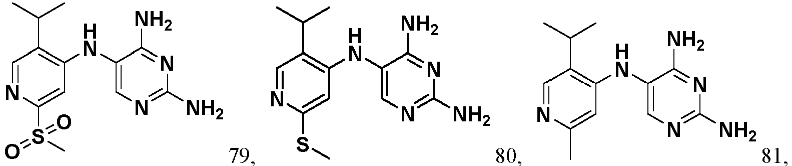

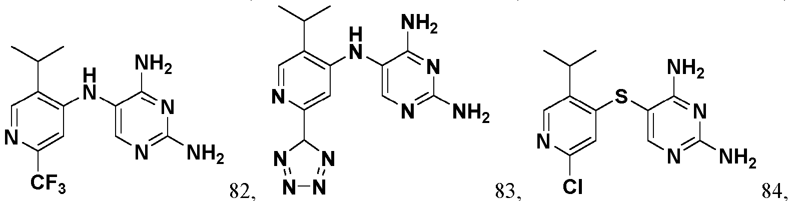

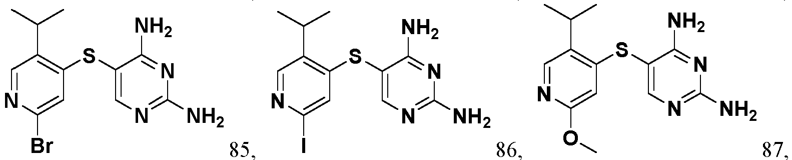

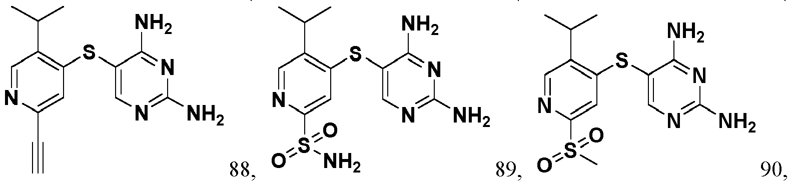

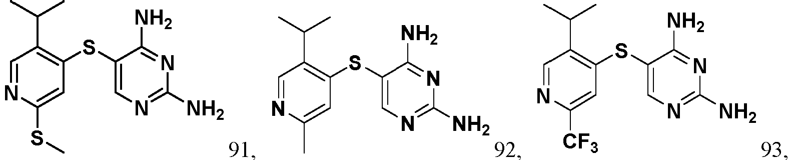

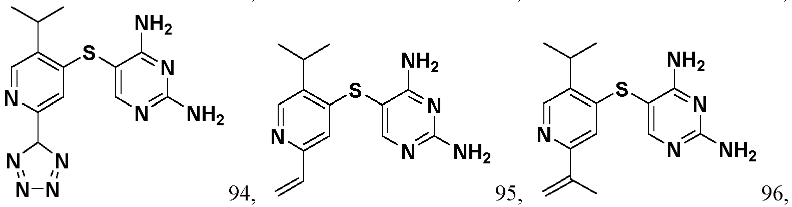

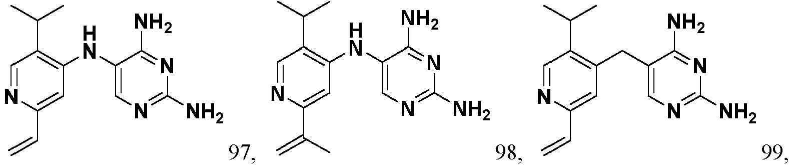

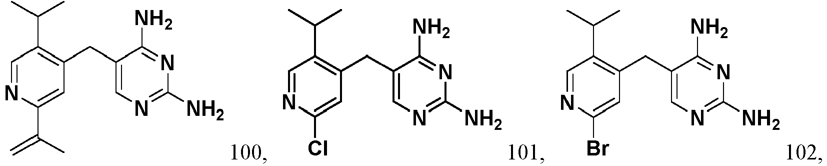

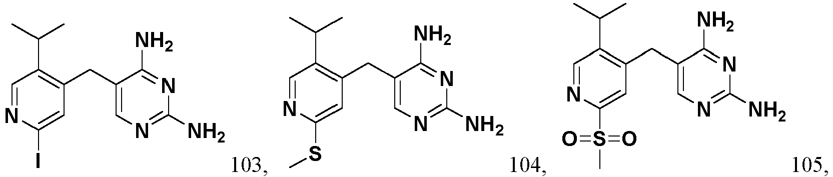

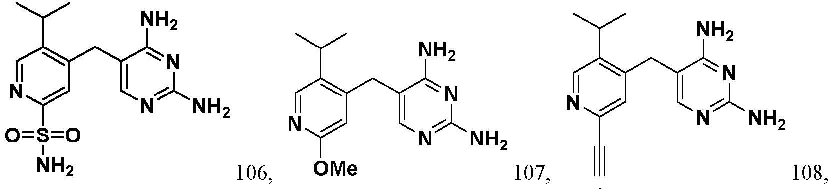

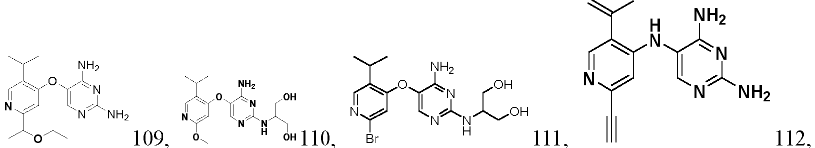

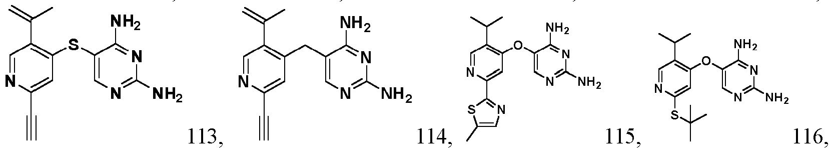

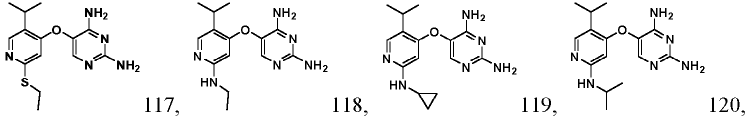

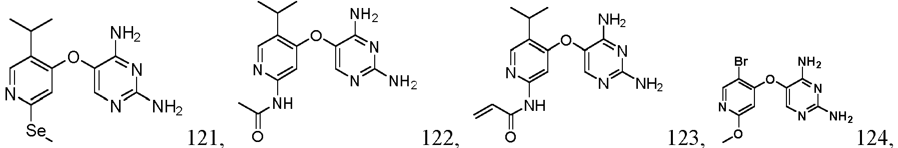

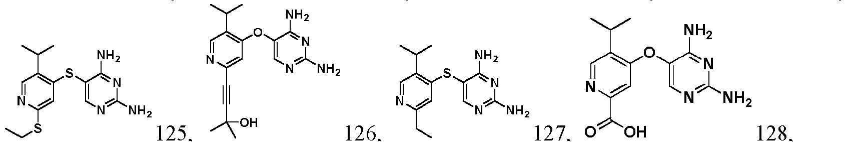

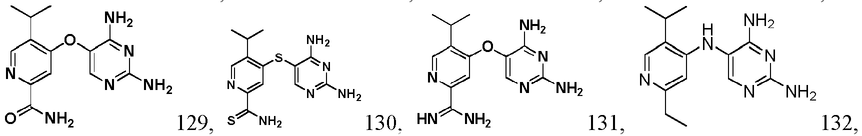

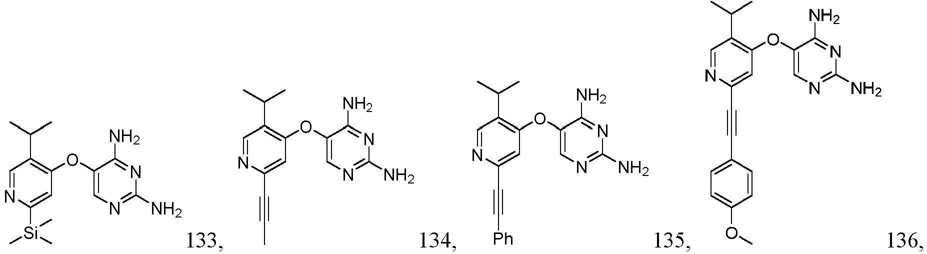

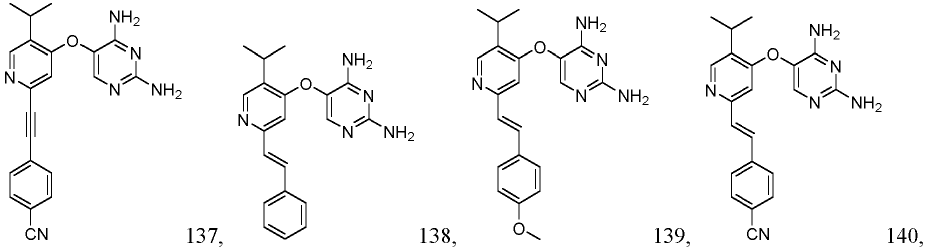

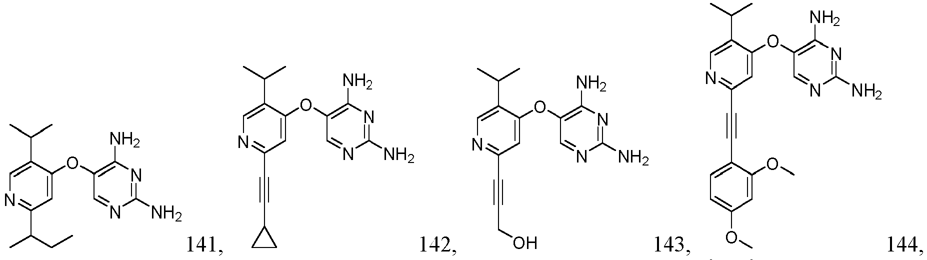

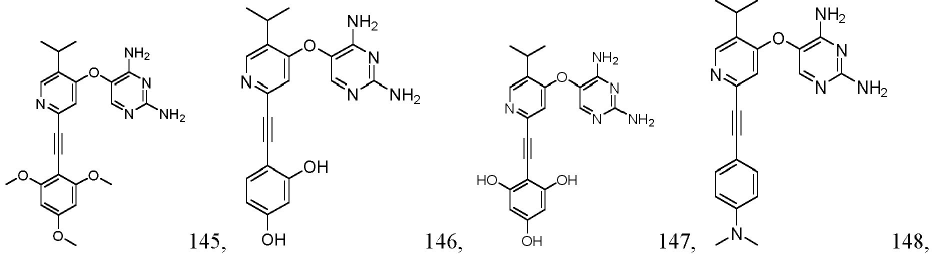

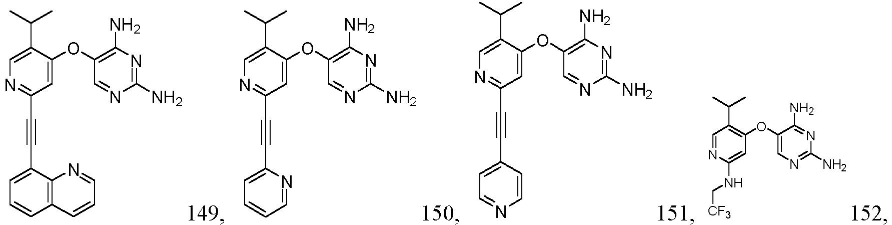

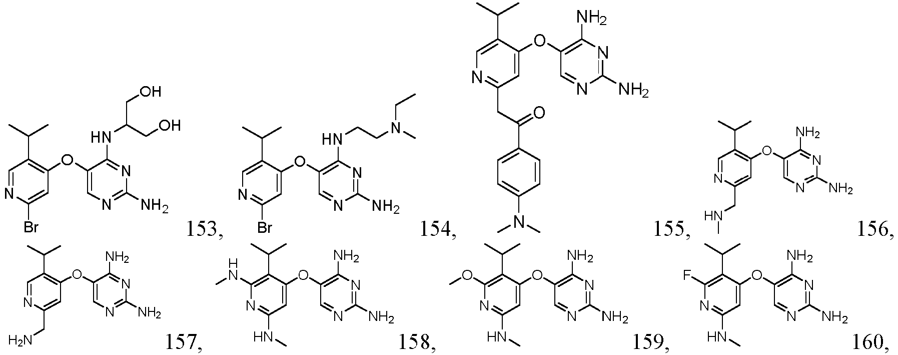

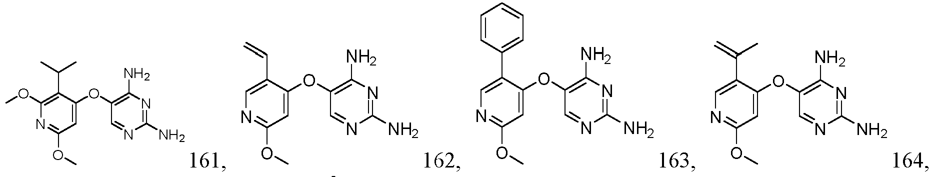

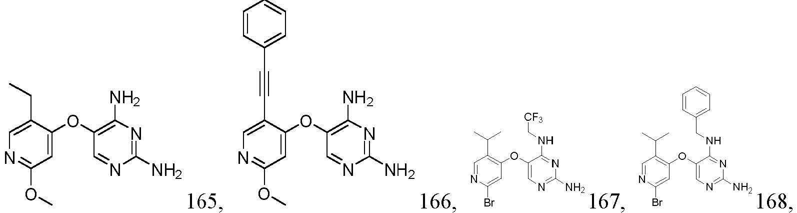

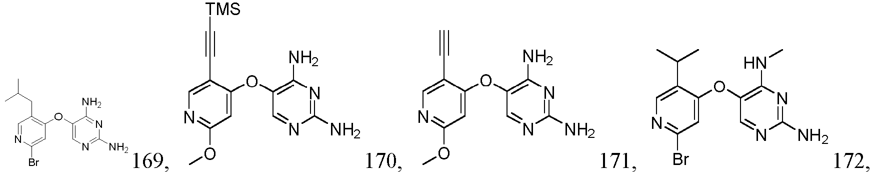

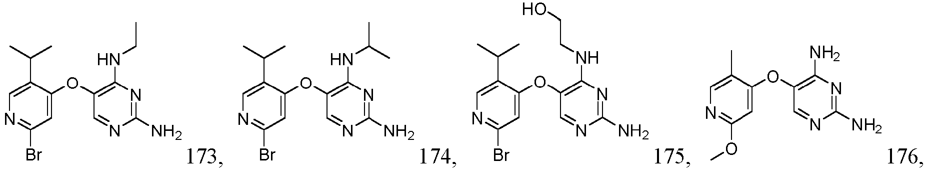

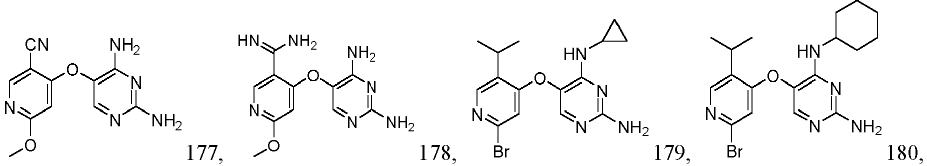

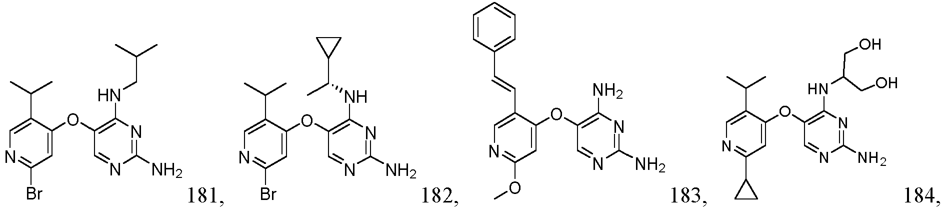

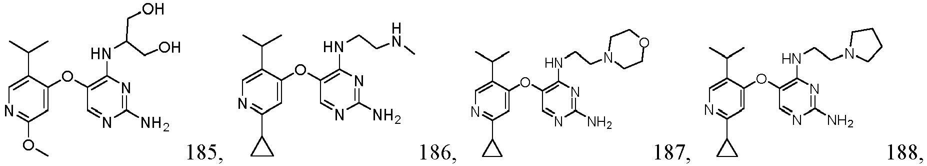

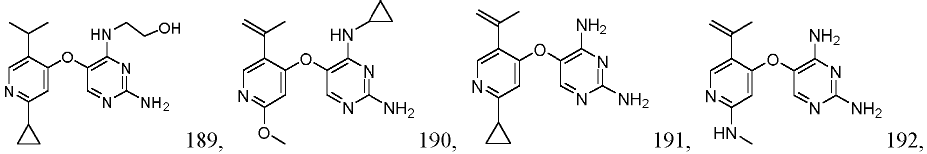

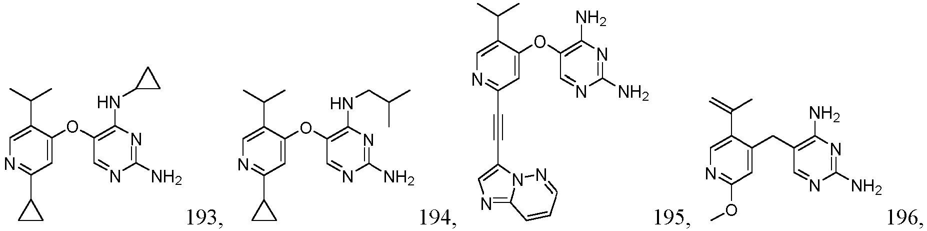

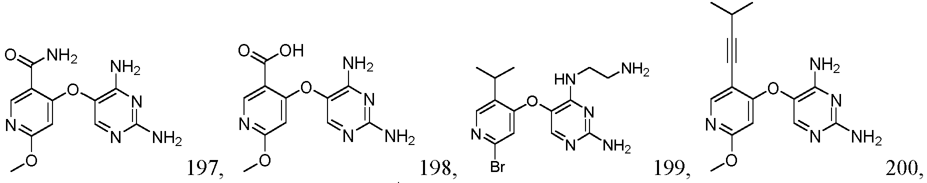

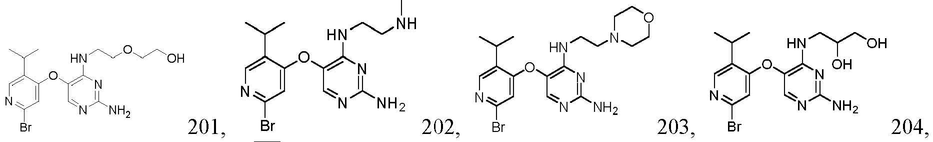

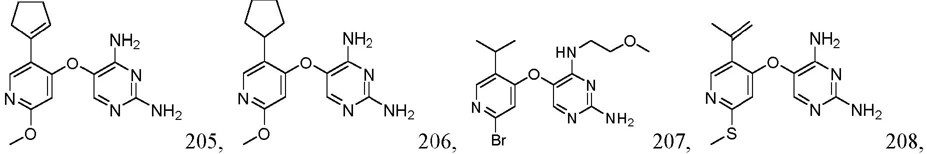

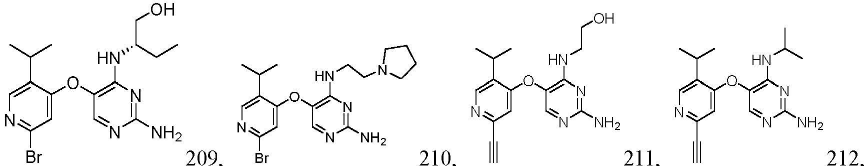

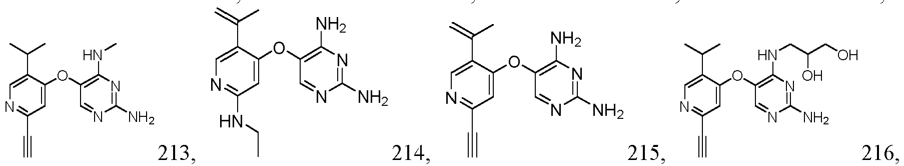

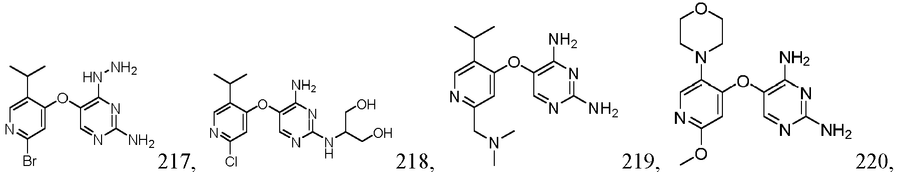

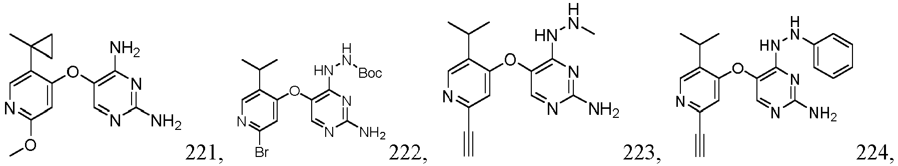

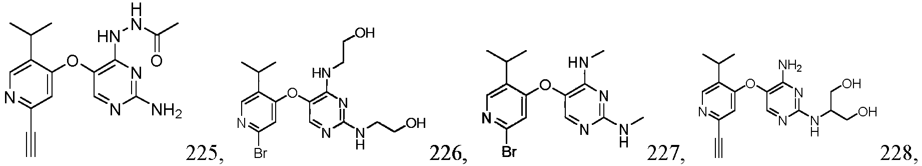

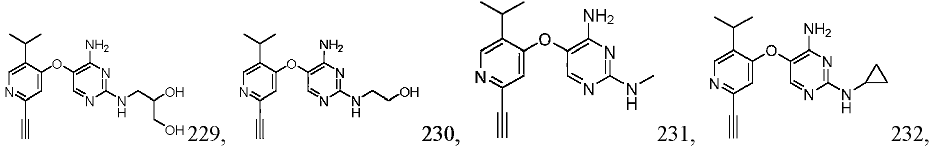

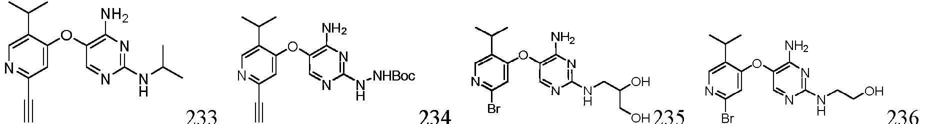

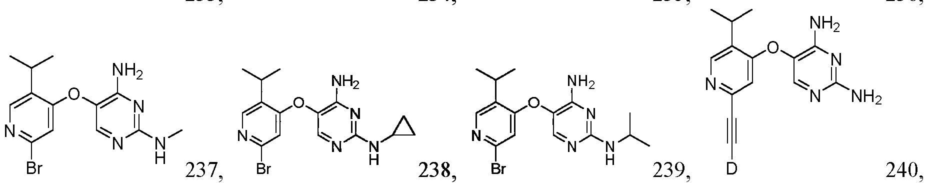

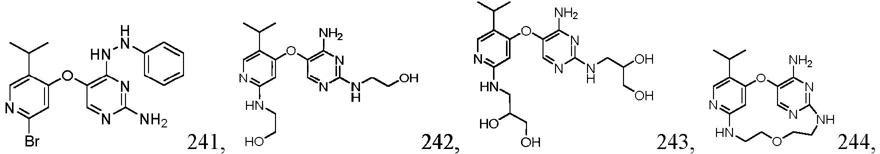

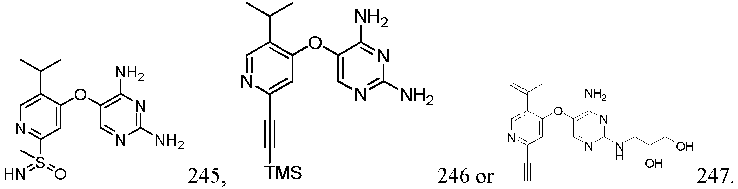

In some embodiments, the compound of Formula (I) or a pharmaceutically acceptable salt, ester, stereoisomer, polymorph, solvate, N-oxide, or isotopically labeled compound thereof is administered in an amount of about 0.005 mg/day to about 5000 mg/day, e.g., in an amount of about 0.005, 0.05, 0.5, 5, 10, 20, 30, 40, 50, 100, 150, 200, 250, 300, 350, 400, 450, 500, 550, 600, 650, 700, 750, 800, 850, 900, 950, 1000, 1500, 2000, 2500, 3000, 3500, 4000, 4500 or 5000 mg/day.

In some embodiments, the compound of Formula (I) or a pharmaceutically acceptable salt, ester, stereoisomer, polymorph, N-oxide, or isotopically labeled compound thereof is administered in an amount of about 1 ng/kg to about 200 mg/kg, about 1 µg/kg to about 100 mg/kg or about 1 mg/kg to about 50 mg/kg per day, e.g., is administered in an amount of about 1 µg/kg, about 10 µg/kg, about 25 µg/kg, about 50 µg/kg, about 75 µg/kg, about 100 µg/kg, about 125 µg/kg, about 150 µg/kg, about 175 µg/kg, about 200 µg/kg, about 225 µg/kg, about 250 µg/kg, about 275 µg/kg, about 300 µg/kg, about 325 µg/kg, about 350 µg/kg, about 375 µg/kg, about 400 µg/kg, about 425 µg/kg, about 450 µg/kg, about 475 µg/kg, about 500 µg/kg, about 525 µg/kg, about 550 µg/kg, about 575 µg/kg, about 600 µg/kg, about 625 µg/kg, about 650 µg/kg, about 675 µg/kg, about 700 µg/kg, about 725 µg/kg, about 750 µg/kg, about 775 µg/kg, about 800 µg/kg, about 825 µg/kg, about 850 µg/kg, about 875 µg/kg, about 900 µg/kg, about 925 µg/kg, about 950 µg/kg, about 975 µg/kg, about 1 mg/kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 60 mg/kg, about 70 mg/kg, about 80 mg/kg, about 90 mg/kg, about 100 mg/kg, about 125 mg/kg, about 150 mg/kg, about 175 mg/kg, about 200 mg/kg body weight per unit dose.

In some embodiments, the daily dose of the compound of Formula (I) or a pharmaceutically acceptable salt, ester, stereoisomer, polymorph, solvate, N-oxide, or isotopically labeled compound thereof is administered at one time or is administered in two, three or four doses.

In some embodiments, the compound of Formula (I) or a pharmaceutically acceptable salt, ester, stereoisomer, polymorph, N-oxide, or isotopically labeled compound thereof is administered continuously for at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days, at least 23 days, at least 24 days, at least 25 days, at least 30 days, at least 35 days, at least 40 days, at least 45 days, at least 50 days, at least half a year, at least 1 year, at least 2 years, at least 3 years, at least 4 years, at least 5 years or more years.

In some embodiments, the compound of Formula (I) or a pharmaceutically acceptable salt, ester, stereoisomer, polymorph, solvate, N-oxide, or isotopically labeled compound thereof is administered for one or more (e.g., 1, 2, 3, 4, 5, 6, 7, 8, 9 or 10) courses of treatment, wherein each course of treatment lasts for at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days, at least 23 days, at least 24 days, at least 25 days, at least 30 days, at least 35 days, at least 40 days, at least 45 days or at least 50 days; and the interval between every two courses of treatment is 0, 1, 2, 3, 4, 5, 6, 7, 8, 9, 10 days, two weeks, three weeks, or four weeks.

In some embodiments, the compound of Formula (I) or a pharmaceutically acceptable salt, ester, stereoisomer, polymorph, solvate, N-oxide, or isotopically labeled compound thereof is administered through injection (e.g., intravenous, intraarterial, subcutaneous, intraperitoneal, intramuscular injection, including dripping), or transdermal administration, or is administered via oral, buccal, nasal, transmucosal, or topical route, as an ophthalmic formulation, or via inhalation.

In some embodiments, the compound of Formula (I) or a pharmaceutically acceptable salt, ester, stereoisomer, polymorph, solvate, N-oxide, or isotopically labeled compound thereof is administered in a dosage form selected from the group consisting of tablet, capsule, lozenge, hard candy, powder, spray, emulsion, cream, salve, suppository, gel, paste, lotion, injection, nanoformulation, patch, aqueous suspension, injectable solution, elixir, and syrup.

In some embodiments, "cough" is selected from the group consisting of acute cough, sub-acute cough, chronic cough, treatment-resistant cough, treatment-resistant chronic cough, idiopathic chronic cough, post-viral cough, iatrogenic cough, cough associated with post-nasal drip, cough associated with cold, upper respiratory infection, asthma, lung cancer and/or chronic obstructive pulmonary disease (COPD), cough associated with interstitial disease, cough associated with pulmonary fibrosis, cough associated with gastroesophageal reflux disease (GERD), cough associated with smoking or a form of bronchitis, and neuronal hypersensitivity underlying acute, sub-acute or chronic cough; preferably, the cough is selected from the group consisting of chronic cough, treatment-resistant cough, treatment-resistant chronic cough, and cough associated with pulmonary fibrosis.

The method of the present invention can also be applied to various diseases accompanied by cough, for example, various respiratory diseases such as cold (cold syndrome), acute bronchitis, chronic bronchitis, bronchiectasis, pneumonia, pulmonary tuberculosis, silicosis and silicotuberculosis, lung cancer, upper respiratory inflammation (pharyngitis, laryngitis and catarrhal rhinitis), asthmatic bronchitis, bronchial asthma, infantile asthma, (chronic) pulmonary emphysema, pneumoconiosis, pulmonary fibrosis, pulmonary suppuration, pleurisy, tonsillitis, cough hives, pertussis and the like; cough during bronchographic or bronchoscopic examinations or the like.

### Example

In order to make the objects and technical solutions of the invention clearer, the invention will be further described below with reference to specific examples. It should be understood that the following examples are only intended for illustrating the invention and are not to be understood as limiting the scope of the invention. Further, specific experimental methods not mentioned in the following examples are carried out in accordance with conventional experimental methods.

Unless otherwise stated, the reagents employed in the following examples were purchased from companies such as Sigma, etc.

Compounds 2 and 66 of the present application were prepared according to the method described in PCT/CN2018/112829.

### Example 1

Effect on the Guinea Pig Cough Model

In this test, dextromethorphan (purchased from Shanghai Send Pharmaceutical Technology Co., Ltd.) was used as a positive control compound. A clinical control compound having the following structure was employed, and it was prepared according to the method described in WO 2008/040652 A1:

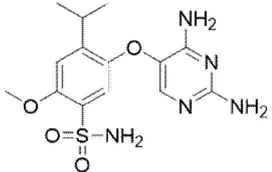

Test animals were 300-350 g Dunkin-Hartley guinea pigs (purchased from Shanghai Jiagan Biotechnology Co., Ltd). After being purchased and adaptively fed for one week, the animals were randomly divided into groups based on body weight, each test group comprising 10-15 test animals. The vehicle (a 0.5% CMCNa solution), the test compounds, the positive control compound (dextromethorphan) or the clinical control compound were orally administrated (compounds at test dosages were respectively added to a 0.5% CMCNa solution, uniformly dispersed through thorough ultrasonic treatment; the solution was vortex mixed again before administration to each animal, and the test dosage ranged from 1 mg/kg to 100 mg/kg). Administration was performed 30 minutes to 1 hour before inducing cough.

Method of inducing cough: test animals were put in a body pneumotachograph (Buxco) and allowed to stand for several minutes. After the animals were stable, 250 uM ATP was nebulized for 2 minutes or 0.6 mM histamine was nebulized for 2 minutes. After 3 minutes, 0.5 M citric acid was nebulized for 5 minutes. Starting from the nebulization of citric acid, the number of coughs of the animals in 10 minutes were recorded by employing a small animal pulmonary function detector (Buxco).

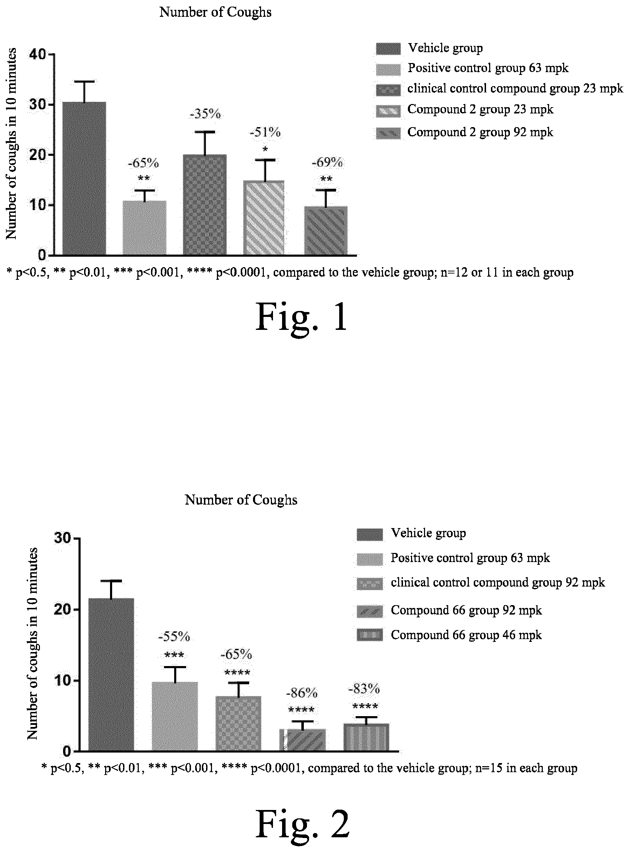

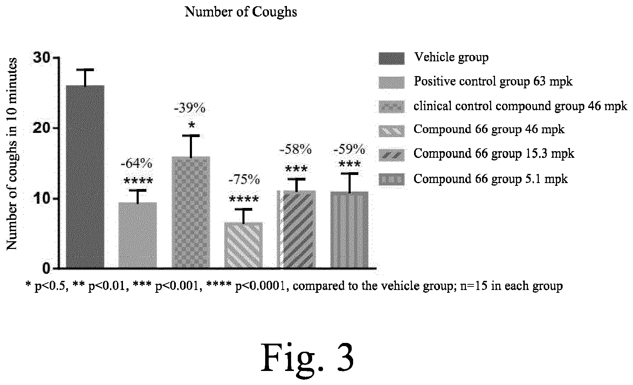

The data in Figure 1 to Figure 3 showed that compounds 22 and 66 of the present application had a significant antitussive effect, which was better than that of the clinical control compound.

### Example 2. Inhibition on the P2X3-mediated current in 1321N1 Cell line Stably Transfected with P2X3

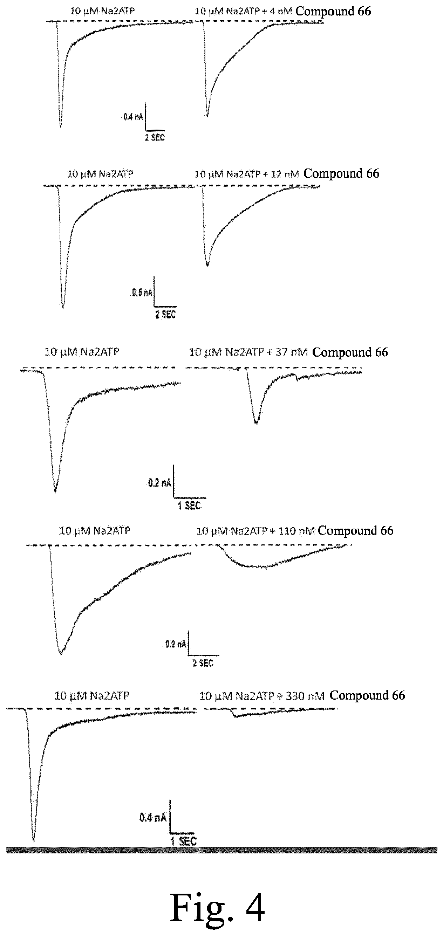

Membrane current was recorded by employing HEKA EPC-10 patch clamp amplifier and PATCHMASTER acquisition system. 1321N1 P2X3 stable transfected cells were transferred to an about 1 ml bath embedded in an inverted microscope platform, and an extracellular fluid (2 mM CaCl₂, 1 mM MgCl₂, 5 mM KCl, 155 mM NaCl, 12 mM glucose and 10 mM HEPES (pH=7.4)) was perfused by using a gravitational perfusion system. The P2X3-mediated current of a single cell was recorded in a whole cell recording mode. After formation of a gigaseal and rapture of the membrane, clamping potential was set at -60 mV. 10 µM Na2ATP was perfused for 5 seconds, and the P2X3-mediated current induced at this point was taken as a control current. The cells were then treated with a solution of compound 66 at a specific concentration (prepared with the extracellular fluid) for 5 minutes. The solution of compound 66 at this concentration and 10 µM Na2ATP were co-applied to induce a cell current (see Figure 4 for the effect of the compound on the current), and an inhibition rate relative to the control current was calculated according to the following formula:$Inhibition Rate Relative to the Control Current = \left(1-{I}_{2}{/ I}_{1}\right) * 100 %$ wherein I₁ represents the control current, and I₂ represents the current after application of compound 66. The concentrations of the test compound 66 included 4, 12, 37, 110 and 330 nM, and at least three cells (n≥3) were tested at each concentration.

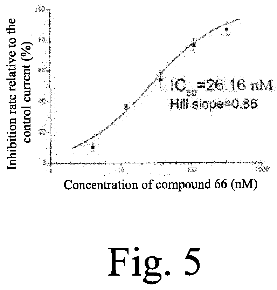

The concentration of compound 66 (as the horizontal axis) was plotted against the inhibition rate relative to the control current (as the vertical axis) (see Figure 5), and the data were fitted with the Hill equation to obtain that the concentration required for compound 66 to inhibit the P2X3-mediated current induced by 10 µM Na2ATP by 50% (IC₅₀) was 26.16 nM.

### Example 3. Inhibition on the P2X3-Mediated Current in ex vivo Cultured Rat Dorsal Root Ganglion (DRG)

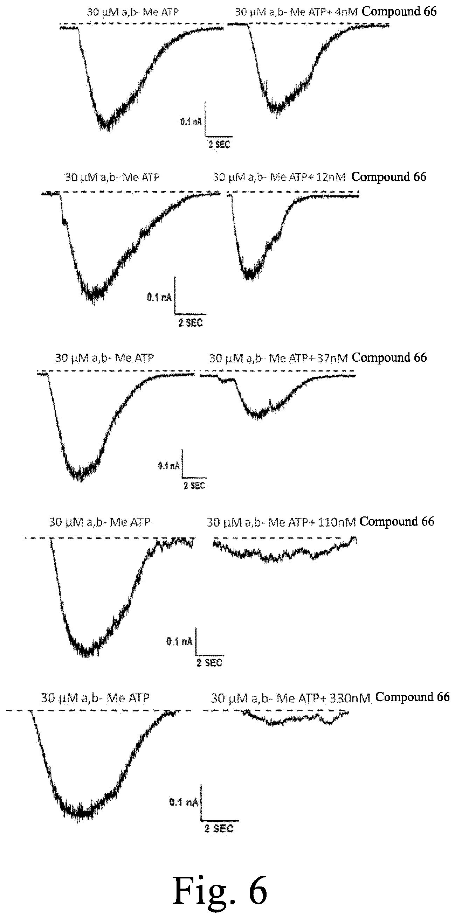

Membrane current was recorded by employing HEKA EPC-10 patch clamp amplifier and PATCHMASTER acquisition system. The primary cells of rat DRG were transferred to an about 1 ml bath embedded in an inverted microscope platform, and an extracellular fluid (2 mM CaCl₂, 1 mM MgCl₂, 5 mM KCl, 155 mM NaCl, 12 mM glucose and 10 mM HEPES (pH=7.4)) was perfused by using a gravitational perfusion system. The P2X3-mediated current of a single cell was recorded in a whole cell recording mode. After formation of a gigaseal and rapture of the membrane, clamping potential was set at -60 mV. 30 µM a,b-Me ATP (also known as "α,β-meATP") was perfused for 5 seconds, and the P2X3-mediated current induced at this point was taken as a control current. The cells were then treated with a solution of compound 66 at a specific concentration (prepared with the extracellular fluid) for 5 minutes. The solution of compound 66 at this concentration and 30 µM a,b-Me ATP were co-applied to induce a cell current (see Figure 6 for the effect of the compound on the current), and an inhibition rate relative to the control current was calculated according to the following formula:$Inhibition Rate Relative to the Control Current = \left(1-{I}_{2}{/ I}_{1}\right) * 100 %$ wherein I₁ represents the control current, and I₂ represents the current after application of compound 66. The concentrations of the test compound 66 included 4, 12, 37, 110 and 330 nM, and at least three cells (n≥3) were tested at each concentration.

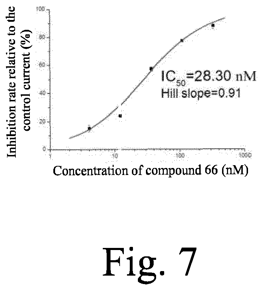

The concentration of compound 66 (as the horizontal axis) was plotted against the inhibition rate relative to the control current (as the vertical axis) (see Figure 7), and the data were fitted with the Hill equation to obtain that the concentration required for compound 66 to inhibit the P2X3-mediated current induced by 30 µM a,b-Me ATP by 50% (IC₅₀) was 28.30 nM.

## Claims

1. A compound of Formula (I) or a pharmaceutically acceptable salt, ester, stereoisomer, polymorph, solvate, N-oxide, or isotopically labeled compound thereof for use of treating, suppressing or alleviating cough or cough impulse: 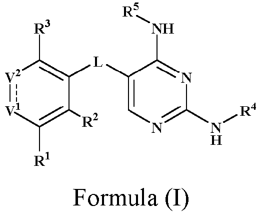 wherein:
L is selected from the group consisting of C(=O), CRR', NR, O, S, S=O and S(=O)₂;
V¹ is selected from the group consisting of N, 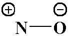 and NR;
V² is selected from the group consisting of CR⁶ and C(=O);
--- represents either a single bond or a double bond, provided that when --- is a single bond, V¹ is NR and V² is C(=O);
R and R' are each independently selected from the group consisting of H, halogen, C₁₋₆ alkyl, C₂₋₆ alkenyl, C₂₋₆ alkynyl, saturated or partially unsaturated C₃₋₁₀ cyclic hydrocarbyl, saturated or partially unsaturated 3- to 10-membered heterocyclyl, C₆₋₁₀ aryl, 5- to 14-membered heteroaryl and C₆₋₁₂ aralkyl, and at most 2 ring members in the cyclic hydrocarbyl and heterocyclyl are C(=O);
R¹, R², R³ and R⁶ are each independently selected from the group consisting of H, halogen, -CN, -NO₂, -NH₂, -OH, -SH, -Se-R, -Si(R)₃, C₁₋₆ alkyl, C₂₋₆ alkenyl, C₂₋₆ alkynyl, saturated or partially unsaturated C₃₋₁₀ cyclic hydrocarbyl, saturated or partially unsaturated 3-to 10-membered heterocyclyl, C₆₋₁₀ aryl, 5- to 14-membered heteroaryl, C₆₋₁₂ aralkyl, C₁₋₆ haloalkyl, -C(=O)R^{a}, -OC(=O)R^{a}, -C(=O)OR^{a}, -OR^{a}, -SR^{a}, -S(=O)R^{a}, -S(=O)₂R^{a}, -S(=O)₂NR^{a}R^{b}, -S(=O)(=NR)R^{a}, -NR^{a}R^{b}, -C(=O)NR^{a}R^{b}, -C(=S)NR^{a}R^{b}, -C(=NR)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆ alkylene-NR^{a}R^{b}, -C₁₋₆ alkylene-OR^{a}, -C₁₋₆ alkylene-C(=O)R, -C₁₋₆ alkenylene-OR^{a}, -O-C₁₋₆ alkylene-NR^{a}R^{b} and -P(=O)R^{a}R^{b};
R⁴ and R⁵ are each independently selected from the group consisting of H, -C(=O)OR^{a}, -NR^{a}R^{b}, -NR^{a}-C(=O)R^{b}, -NR^{a}-C(=O)OR^{b}, -C₁₋₆ alkylene-NR^{a}R^{b}, -C₁₋₆ alkylene-OR^{a}, -C₁₋₆ alkylene-O-C₁₋₆ alkylene-OR^{a}, C₁₋₆ alkyl, C₁₋₆ haloalkyl, C₂₋₆ alkenyl, C₂₋₆ alkynyl, saturated or partially unsaturated C₃₋₁₀ cyclic hydrocarbyl, saturated or partially unsaturated 3- to 10-membered heterocyclyl, C₆₋₁₀ aryl, 5- to 14-membered heteroaryl and C₆₋₁₂ aralkyl;
alternatively, R¹ and R⁴ together form -NHCH₂CH₂-O-CH₂CH₂-;
the above alkyl, alkylene, alkenyl, alkynyl, cyclic hydrocarbyl, heterocyclyl, aryl, heteroaryl and aralkyl, at each occurrence, are each optionally substituted with one or more substituents independently selected from the group consisting of halogen, hydroxyl, oxo, amino, cyano, nitro, -Si(R)₃, C₁₋₆ alkyl, saturated or partially unsaturated C₃₋₆ cyclic hydrocarbyl, saturated or partially unsaturated 3- to 10-membered heterocyclyl, C₆₋₁₀ aryl, 5-to 14-membered heteroaryl, C₆₋₁₂ aralkyl, -C(=O)R^{a}, -OC(=O)R^{a}, -C(=O)OR^{a}, -OR^{a}, -SR^{a}, -S(=O)R^{a}, -S(=O)₂R^{a}, -S(=O)₂NR^{a}R^{b}, -NR^{a}R^{b}, -C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆ alkylene-NR^{a}R^{b}, -C₁₋₆ alkylene-OR^{a}, -C₁₋₆ alkenylene-OR^{a} and -O-C₁₋₆ alkylene-NR^{a}R^{b}, the alkyl, cyclic hydrocarbyl, heterocyclyl, aryl, heteroaryl and aralkyl are further optionally substituted with one or more substituents independently selected from the group consisting of halogen, hydroxyl, oxo, amino, cyano, nitro, -NR^{a}R^{b}, C₁₋₆ alkyl, -O-C₁₋₆ alkyl, saturated or partially unsaturated C₃₋₆ cyclic hydrocarbyl, saturated or partially unsaturated 3- to 10-membered heterocyclyl, C₆₋₁₀ aryl, 5- to 14-membered heteroaryl and C₆₋₁₂ aralkyl; and
R^{a} and R^{b}, at each occurrence, are each independently selected from the group consisting of H, -OH, C₁₋₆ alkyl, C₂₋₆ alkenyl, C₂₋₆ alkynyl, saturated or partially unsaturated C₃₋₁₀ cyclic hydrocarbyl, saturated or partially unsaturated 3- to 10-membered heterocyclyl, C₆₋₁₀ aryl, 5- to 14-membered heteroaryl and C₆₋₁₂ aralkyl; alternatively, R^{a} and R^{b} together with the atom to which they are attached form a 3- to 12-membered heterocycle or heteroaromatic ring, the above groups are further optionally substituted with one or more substituents independently selected from the group consisting of halogen, hydroxyl, oxo, amino, cyano, nitro, C₁₋₆ alkyl, -O-C₁₋₆ alkyl, saturated or partially unsaturated C₃₋₆ cyclic hydrocarbyl, saturated or partially unsaturated 3- to 10-membered heterocyclyl, C₆₋₁₀ aryl, 5-to 14-membered heteroaryl and C₆₋₁₂ aralkyl.

2. The compound for use according to claim 1, wherein the compound has the structure of any of the following formulae: 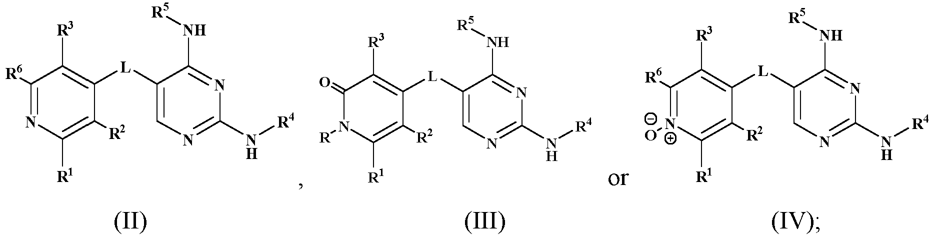
preferably has the structure of any of the following formulae: 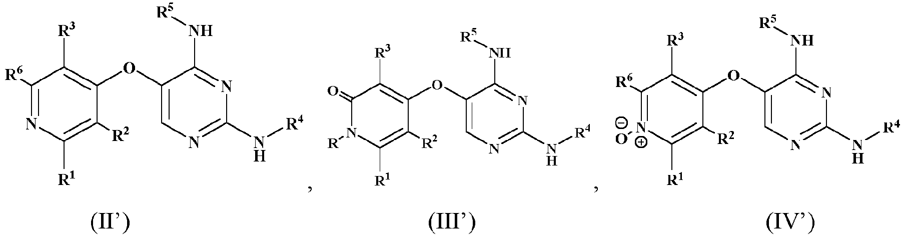 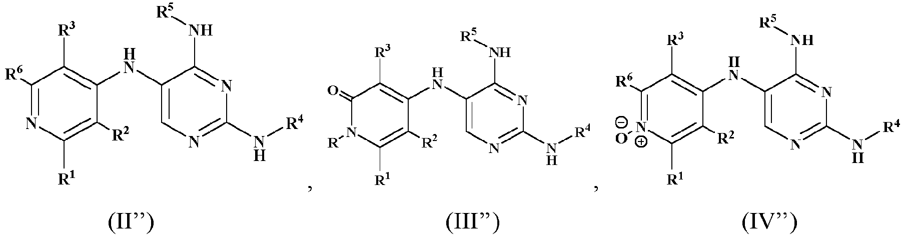 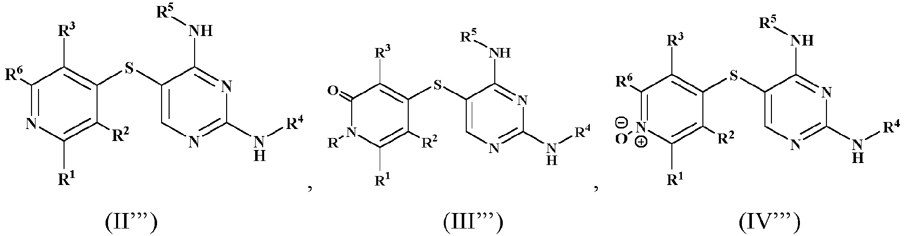 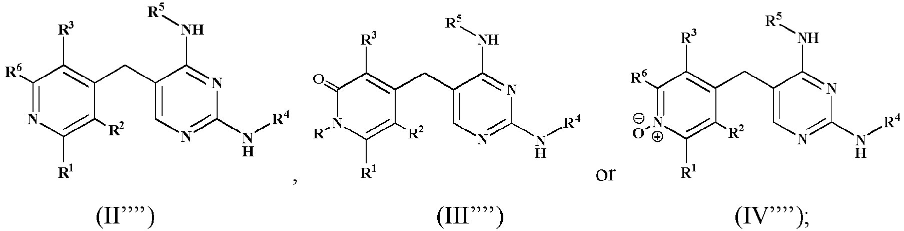
more preferably, the compound of Formula (I) has the structure of any of the following formulae: 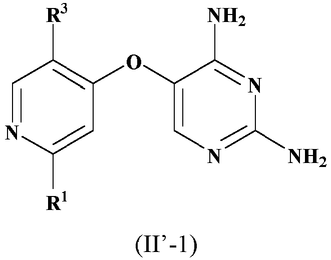
wherein:
R¹ is selected from the group consisting of F, Cl, Br, I and C₂₋₆ alkynyl, preferably Br or ethynyl; and
R³ is C₁₋₆ alkyl, preferably isopropyl.

3. The compound for use according to claim 1 or 2, wherein the compound has the following structure:

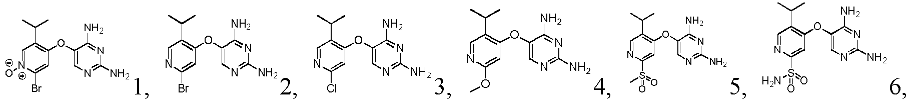

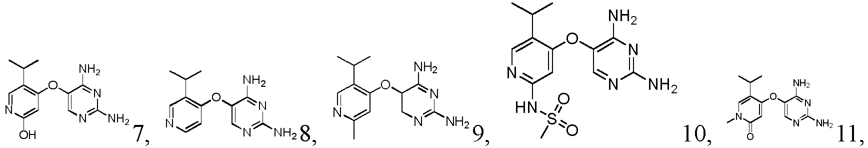

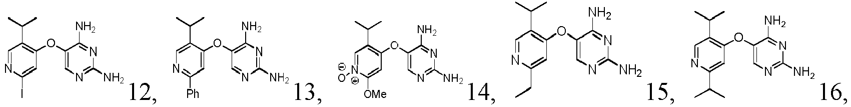

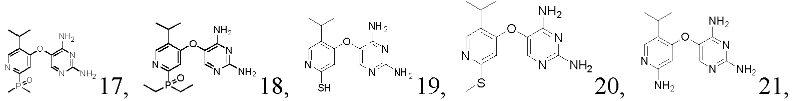

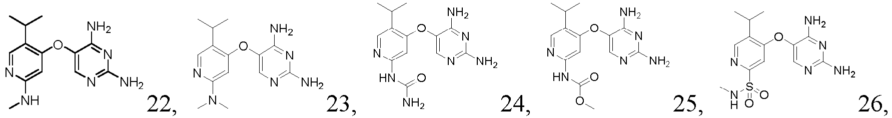

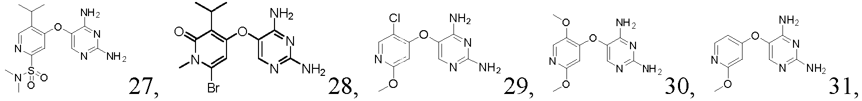

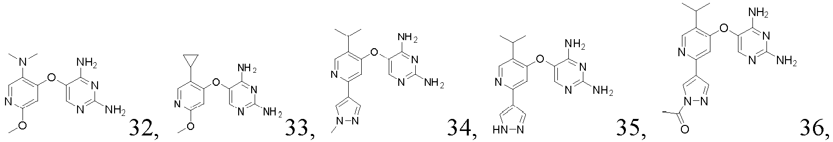

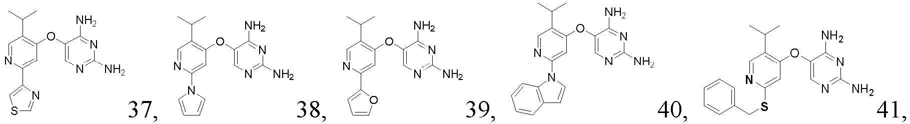

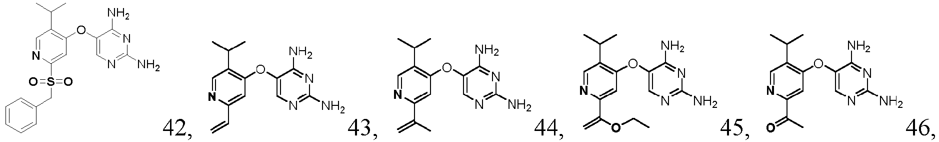

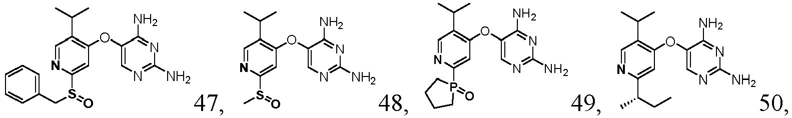

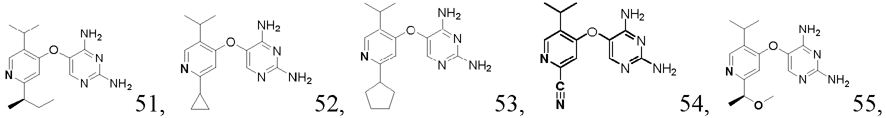

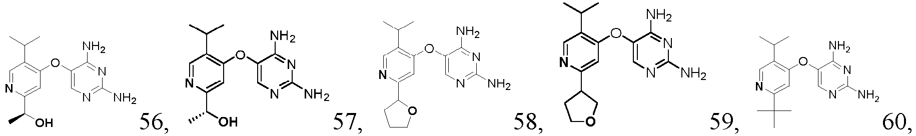

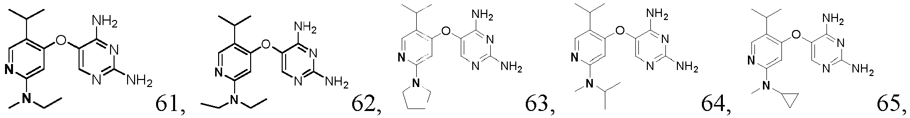

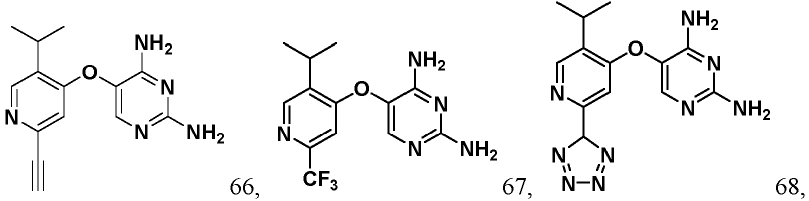

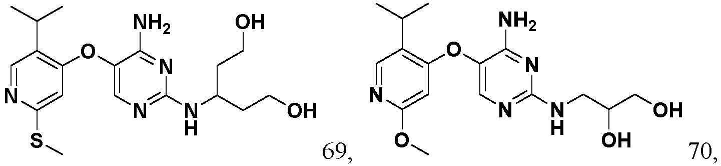

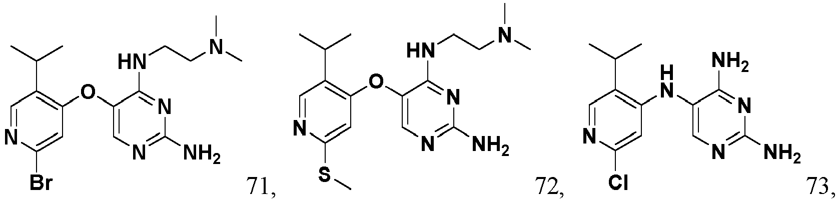

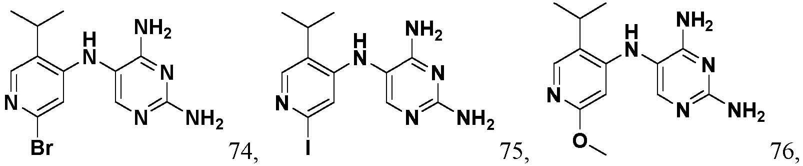

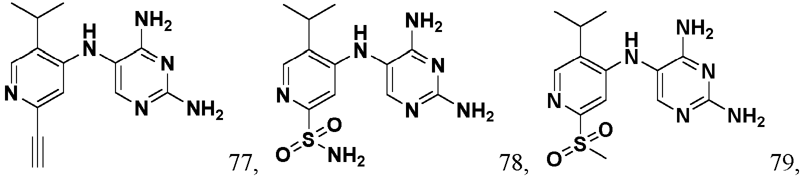

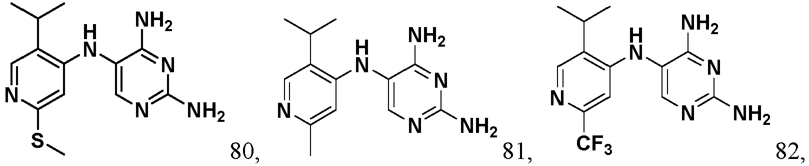

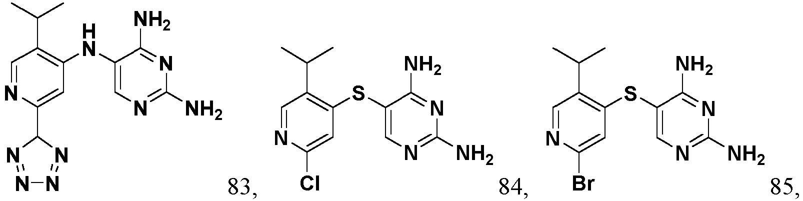

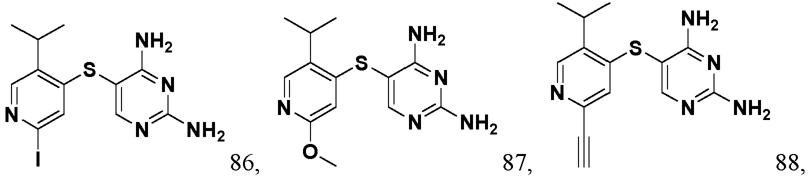

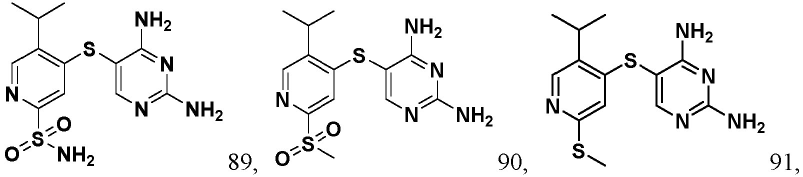

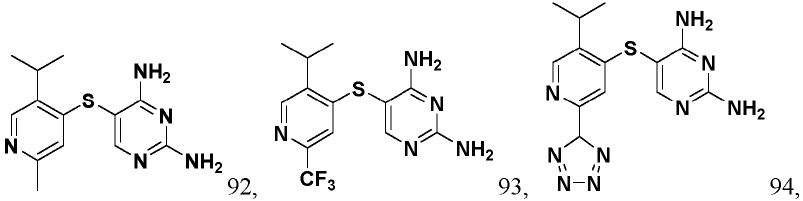

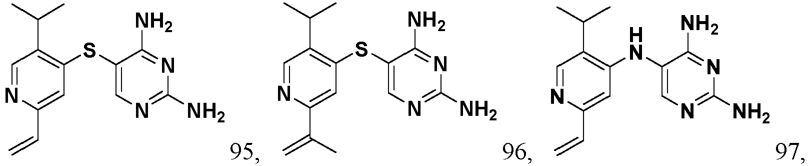

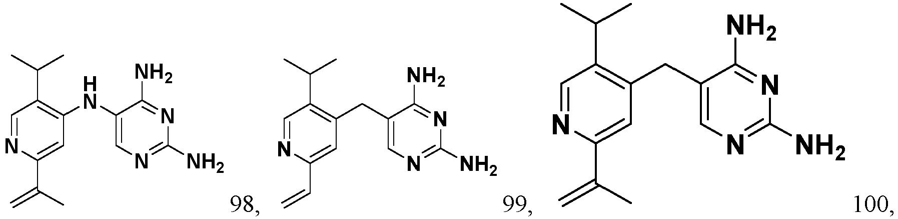

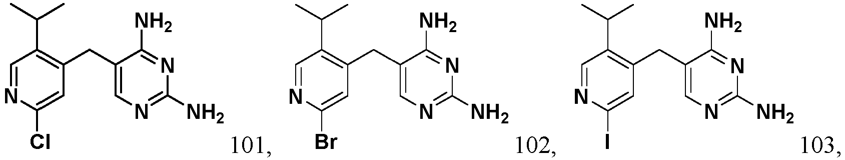

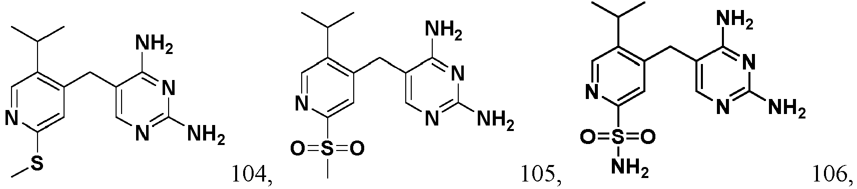

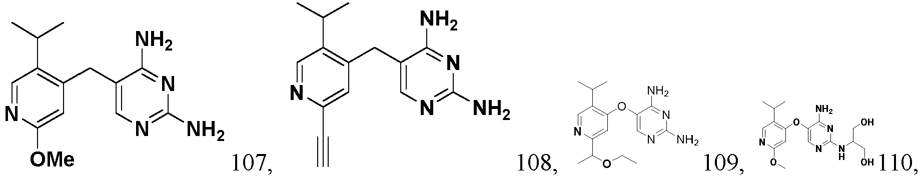

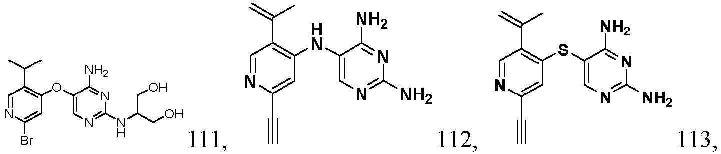

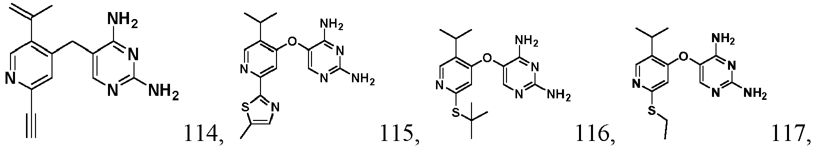

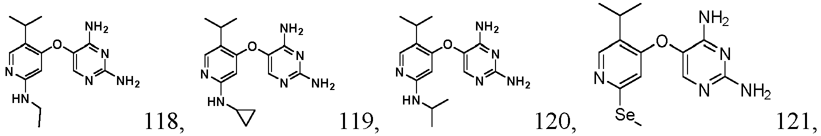

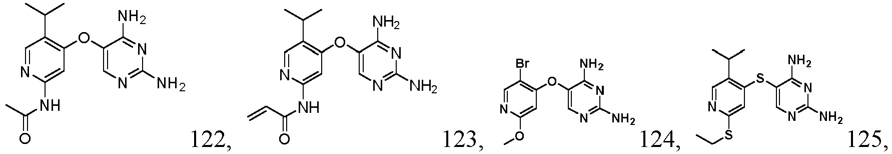

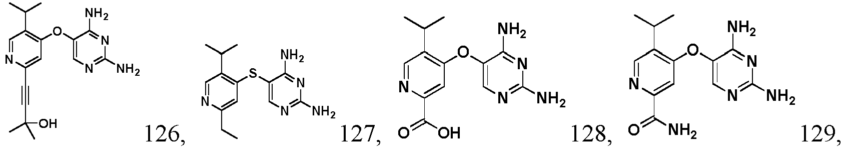

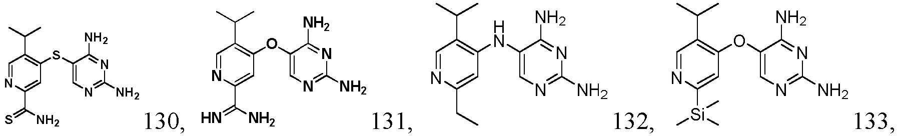

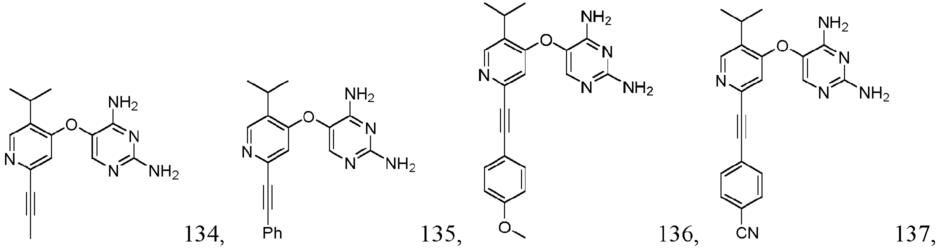

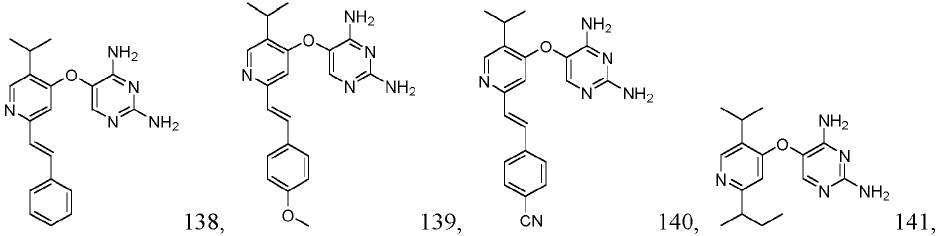

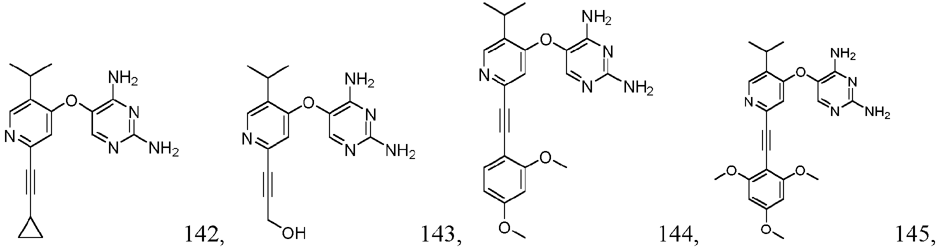

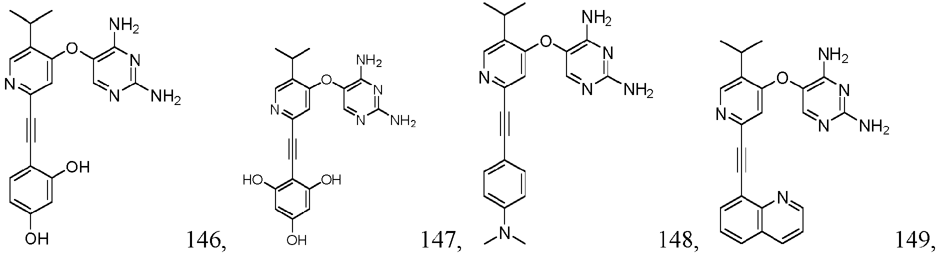

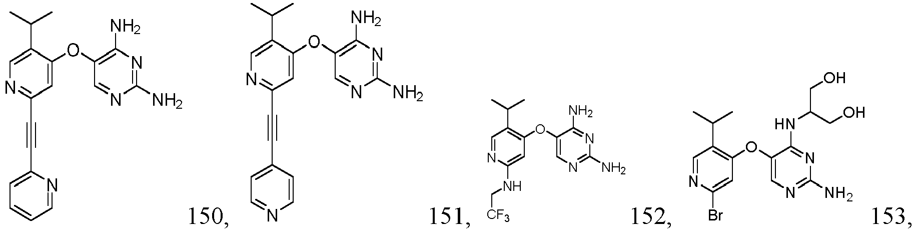

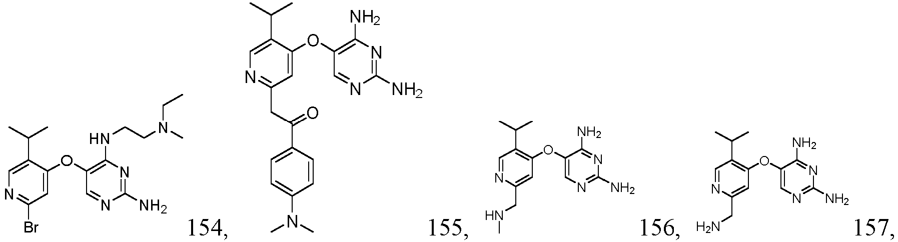

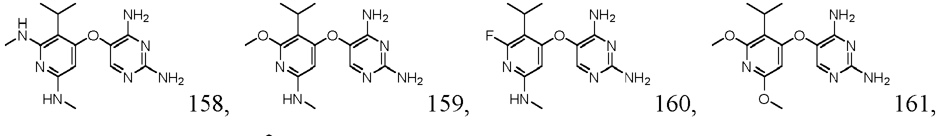

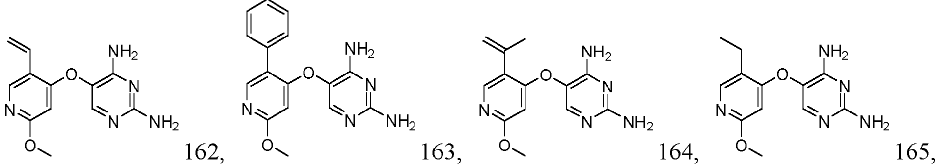

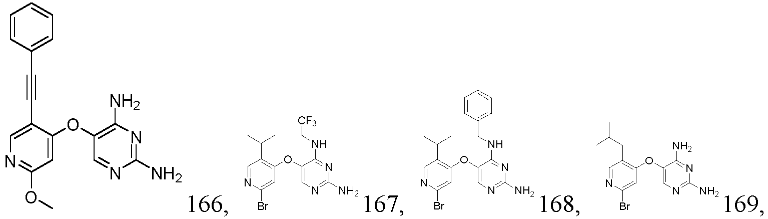

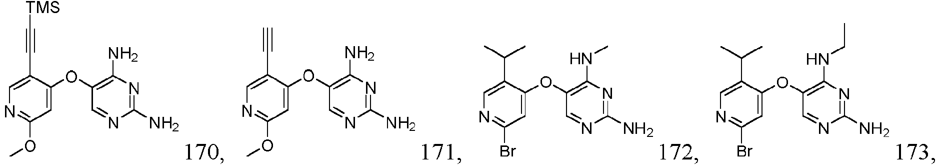

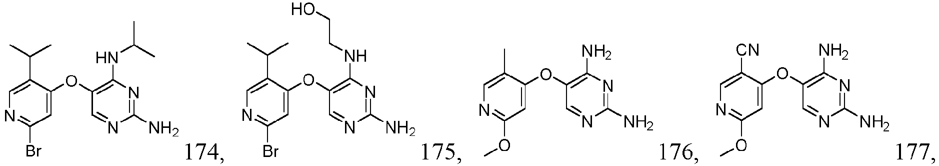

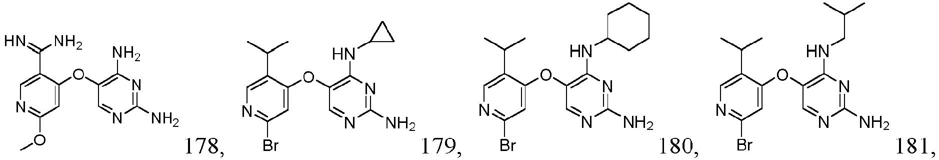

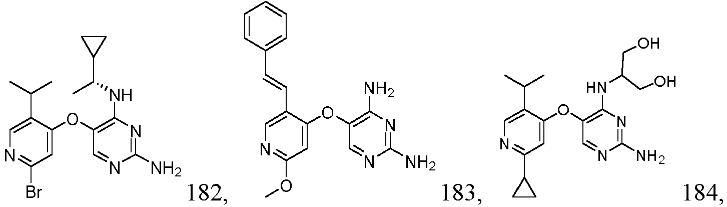

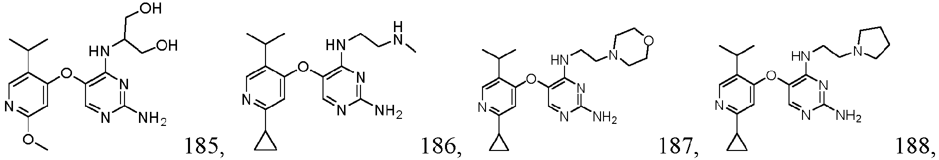

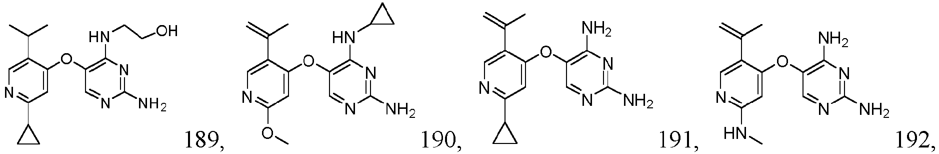

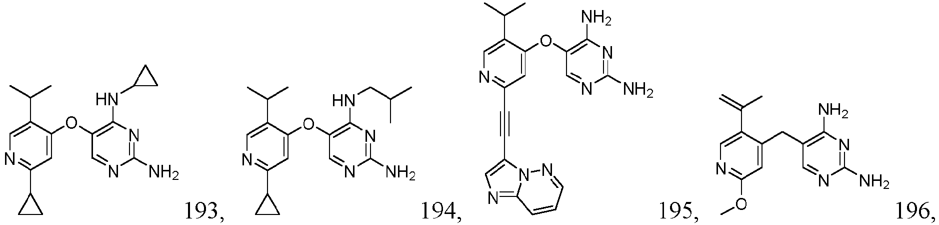

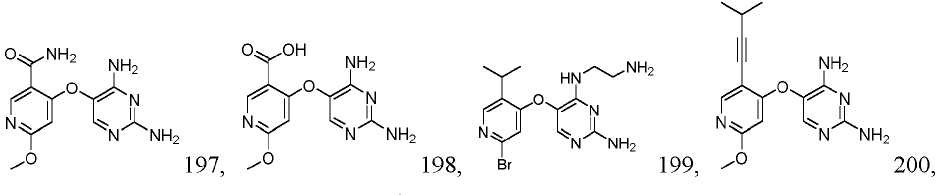

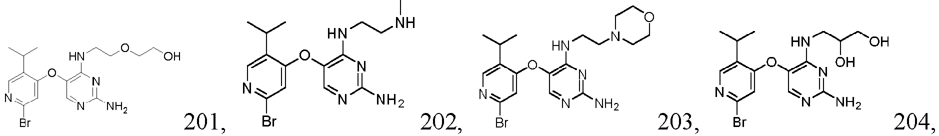

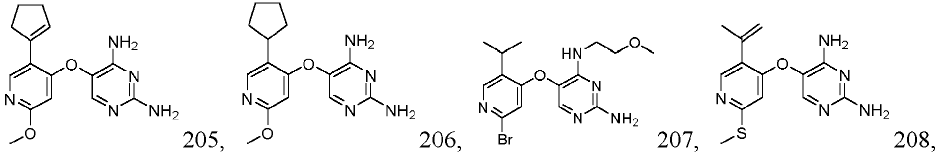

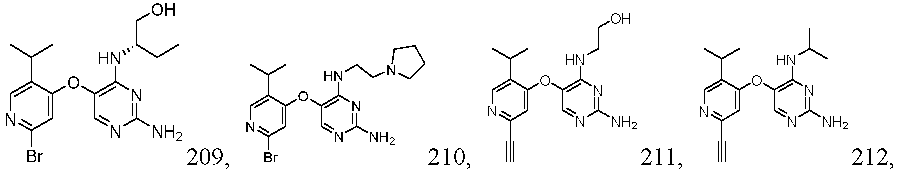

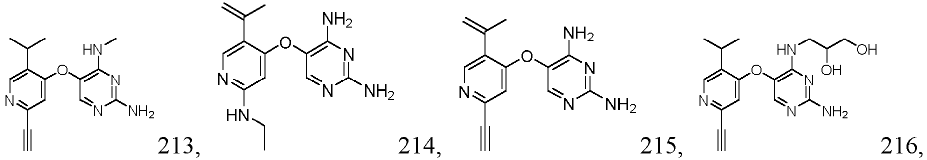

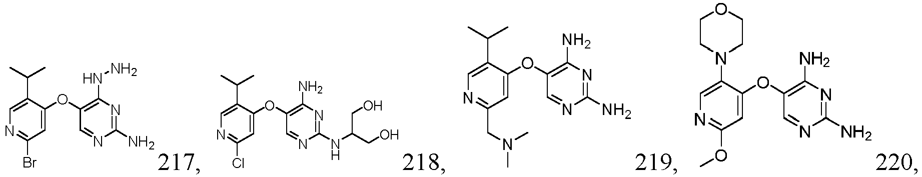

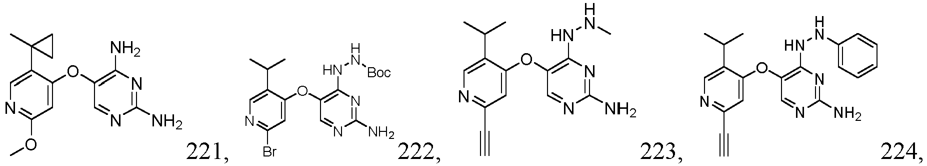

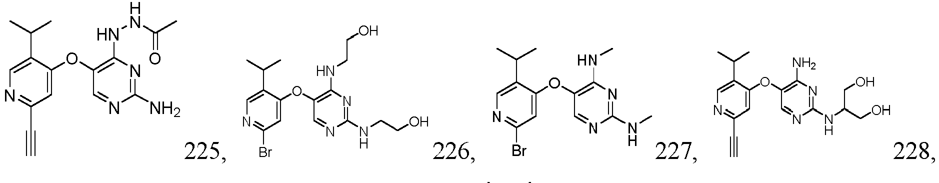

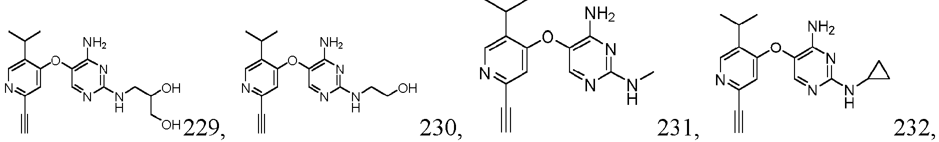

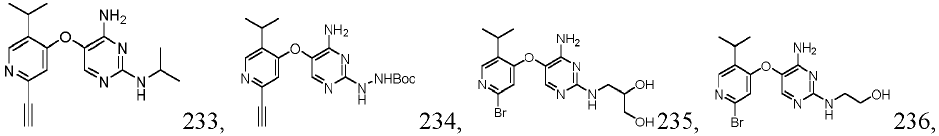

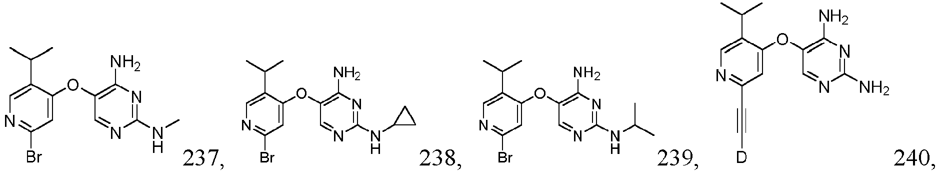

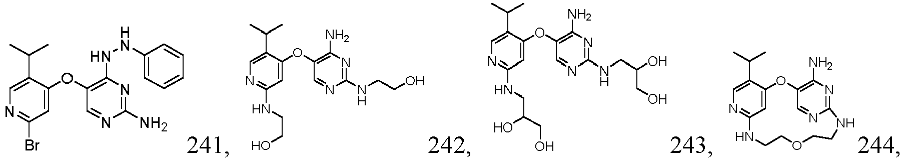

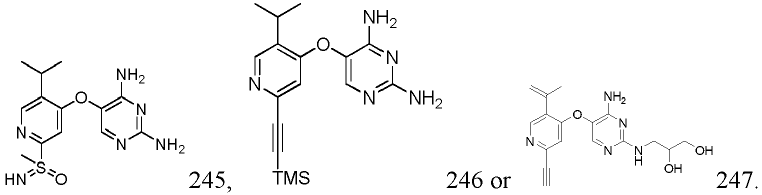

4. The compound for use according to any one of claims 1 to 3, wherein the compound of Formula (I) or a pharmaceutically acceptable salt, ester, stereoisomer, polymorph, solvate, N-oxide, or isotopically labeled compound thereof is for administration in an amount of about 0.005 mg/day to about 5000 mg/day, e.g., in an amount of about 0.005, 0.05, 0.5, 5, 10, 20, 30, 40, 50, 100, 150, 200, 250, 300, 350, 400, 450, 500, 550, 600, 650, 700, 750, 800, 850, 900, 950, 1000, 1500, 2000, 2500, 3000, 3500, 4000, 4500 or 5000 mg/day.

5. The compound for use according to any one of claims 1 to 3, wherein the compound of Formula (I) or a pharmaceutically acceptable salt, ester, stereoisomer, polymorph, solvate, N-oxide, or isotopically labeled compound thereof is for administration in an amount of about 1 ng/kg to about 200 mg/kg, about 1 µg/kg to about 100 mg/kg or about 1 mg/kg to about 50 mg/kg per day, e.g., is for administration in an amount of about 1 µg/kg, about 10 µg/kg, about 25 µg/kg, about 50 µg/kg, about 75 µg/kg, about 100 µg/kg, about 125 µg/kg, about 150 µg/kg, about 175 µg/kg, about 200 µg/kg, about 225 µg/kg, about 250 µg/kg, about 275 µg/kg, about 300 µg/kg, about 325 µg/kg, about 350 µg/kg, about 375 µg/kg, about 400 µg/kg, about 425 µg/kg, about 450 µg/kg, about 475 µg/kg, about 500 µg/kg, about 525 µg/kg, about 550 µg/kg, about 575 µg/kg, about 600 µg/kg, about 625 µg/kg, about 650 µg/kg, about 675 µg/kg, about 700 µg/kg, about 725 µg/kg, about 750 µg/kg, about 775 µg/kg, about 800 µg/kg, about 825 µg/kg, about 850 µg/kg, about 875 µg/kg, about 900 µg/kg, about 925 µg/kg, about 950 µg/kg, about 975 µg/kg, about 1 mg/kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 60 mg/kg, about 70 mg/kg, about 80 mg/kg, about 90 mg/kg, about 100 mg/kg, about 125 mg/kg, about 150 mg/kg, about 175 mg/kg, about 200 mg/kg body weight per unit dose.

6. The compound for use according to any one of claims 1 to 5, wherein the daily dose of the compound of Formula (I) or a pharmaceutically acceptable salt, ester, stereoisomer, polymorph, solvate, N-oxide, or isotopically labeled compound thereof is for administration at one time or is for administration in two, three or four doses.

7. The compound for use according to any one of claims 1 to 6, wherein the compound of Formula (I) or a pharmaceutically acceptable salt, ester, stereoisomer, polymorph, solvate, N-oxide, or isotopically labeled compound thereof is for continuous administration for at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days, at least 23 days, at least 24 days, at least 25 days, at least 30 days, at least 35 days, at least 40 days, at least 45 days, at least 50 days, at least half a year, at least 1 year, at least 2 years, at least 3 years, at least 4 years, or at least 5 years.

8. The compound for use according to any one of claims 1 to 7, wherein the compound of Formula (I) or a pharmaceutically acceptable salt, ester, stereoisomer, polymorph, solvate, N-oxide, or isotopically labeled compound thereof is for administration for one or more (e.g., 1, 2, 3, 4, 5, 6, 7, 8, 9 or 10) courses of treatment, wherein each course of treatment lasts for at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days, at least 23 days, at least 24 days, at least 25 days, at least 30 days, at least 35 days, at least 40 days, at least 45 days or at least 50 days; and the interval between every two courses of treatment is 0, 1, 2, 3, 4, 5, 6, 7, 8, 9, 10 days, two weeks, three weeks, or four weeks.

9. The compound for use according to any one of claims 1 to 8, wherein the compound of Formula (I) or a pharmaceutically acceptable salt, ester, stereoisomer, polymorph, solvate, N-oxide, or isotopically labeled compound thereof is for administration through injection (e.g., intravenous, intraarterial, subcutaneous, intraperitoneal, intramuscular injection, including dripping), or transdermal administration, or is for administration via oral, buccal, nasal, transmucosal, or topical route, as an ophthalmic formulation, or via inhalation.

10. The compound for use according to any one of claims 1 to 9, wherein the compound of Formula (I) or a pharmaceutically acceptable salt, ester, stereoisomer, polymorph, solvate, N-oxide, or isotopically labeled compound thereof is for administration in a dosage form selected from the group consisting of tablet, capsule, lozenge, hard candy, powder, spray, emulsion, cream, salve, suppository, gel, paste, lotion, injection, nanoformulation, patch, aqueous suspension, solution, elixir, and syrup.

11. The compound for use according to any one of claims 1 to 10, wherein the cough is selected from the group consisting of acute cough, sub-acute cough, chronic cough, treatment-resistant cough, treatment-resistant chronic cough, idiopathic chronic cough, post-viral cough, iatrogenic cough, cough associated with post-nasal drip, cough associated with cold, upper respiratory infection, asthma, lung cancer and/or chronic obstructive pulmonary disease (COPD), cough associated with interstitial disease, cough associated with pulmonary fibrosis, cough associated with gastroesophageal reflux disease (GERD), cough associated with smoking or a form of bronchitis, and neuronal hypersensitivity underlying acute, sub-acute or chronic cough; preferably, the cough is selected from the group consisting of chronic cough, treatment-resistant cough, treatment-resistant chronic cough, and cough associated with pulmonary fibrosis.

## Patentansprüche

1. Verbindung der Formel (I) oder pharmazeutisch verträgliche(s)/verträglicher Salz, Ester, Stereoisomer, Polymorph, Solvat, N-Oxid oder isotopenmarkierte Verbindung davon zur Verwendung bei der Behandelung, Unterdrückung oder Linderung von Husten oder Hustenimpuls: 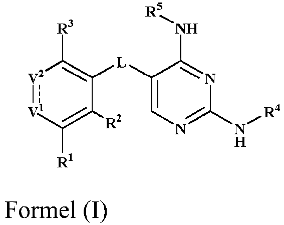 wobei:
L ausgewählt ist aus der Gruppe bestehend aus C(=O), CRR', NR, O, S, S=O und S(=O)₂;
V¹ ausgewählt ist aus der Gruppe bestehend aus N, 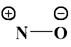 und NR;
V² ausgewählt ist aus der Gruppe bestehend aus CR⁶ und C(=O);
--- entweder eine Einfachbindung oder eine Doppelbindung darstellt, vorausgesetzt, dass, wenn --- eine Einfachbindung ist, V¹ NR ist und V² C(=O) ist;
R und R' jeweils unabhängig ausgewählt sind aus der Gruppe bestehend aus H, Halogen, C₁₋₆-Alkyl, C₂₋₆-Alkenyl, C₂₋₆-Alkinyl, gesättigtem oder teilweise ungesättigtem cyclischen C₃₋₁₀-Kohlenwasserstoff, gesättigtem oder teilweise ungesättigtem 3- bis 10-gliedrigen Heterocyclyl, C₆₋₁₀-Aryl, 5- bis 14-gliedrigem Heteroaryl und C₆₋₁₂-Aralkyl und höchstens 2 Ringelemente in dem cyclischen Kohlenwasserstoff und Heterocyclyl C(=O) sind;
R¹, R², R³ und R⁶ jeweils unabhängig ausgewählt sind aus der Gruppe bestehend aus H, Halogen, -CN, -NO₂, -NH₂, -OH, -SH, -Se-R, -Si(R)₃, C₁₋₆-Alkyl, C₂₋₆-Alkenyl, C₂₋₆-Alkinyl, gesättigtem oder teilweise ungesättigtem cyclischen C₃₋₁₀-Kohlenwasserstoff, gesättigtem oder teilweise ungesättigtem 3- bis 10-gliedrigen Heterocyclyl, C₆₋₁₀-Aryl, 5- bis 14-gliedrigem Heteroaryl, C₆₋₁₂-Aralkyl, C₁₋₆-Haloalkyl, -C(=O)R^{a}, -OC(=O)R^{a}, -C(=O)OR^{a}, -OR^{a}, -SR^{a}, - S(=O)R^{a}, -S(=O)₂R^{a}, -S(=O)₂NR^{a}R^{b}, -S(=O)(=NR)R^{a}, -NR^{a}R^{b}, -C(=O)NR^{a}R^{b}, -C(=S)NR^{a}R^{b}, - C(=NR)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆-Alkylen-NR^{a}R^{b}, -C₁₋₆-Alkylen-OR^{a}, -C₁₋₆-Alkylen-C(=O)R, -C₁₋₆-Alkenylen-OR^{a}, -O-C₁₋₆-Alkylen-NR^{a}R^{b} und -P(=O)R^{a}R^{b};
R⁴ und R⁵ jeweils unabhängig ausgewählt sind aus der Gruppe bestehend aus H, - C(=O)OR^{a}, -NR^{a}R^{b}, -NR^{a}-C(=O)R^{b}, -NR^{a}-C(=O)OR^{b}, -C₁₋₆-Alkylen-NR^{a}R^{b}, -C₁₋₆-Alkylen-OR^{a}, -C₁₋₆-Alkylen-O-C₁₋₆-alkylen-OR^{a}, C₁₋₆-Alkyl, C₁₋₆-Haloalkyl, C₂₋₆-Alkenyl, C₂₋₆-Alkinyl, gesättigtem oder teilweise ungesättigtem cyclischen C₃₋₁₀-Kohlenwasserstoff, gesättigtem oder teilweise ungesättigtem 3- bis 10-gliedrigen Heterocyclyl, C₆₋₁₀-Aryl, 5- bis 14-gliedrigem Heteroaryl und C₆₋₁₂-Aralkyl;
alternativ R¹ und R⁴ zusammen -NHCH₂CH₂-O-CH₂CH₂- bilden;
das/der obige Alkyl, Alkylen, Alkenyl, Alkinyl, cyclische Kohlenwasserstoff, Heterocyclyl, Aryl, Heteroaryl und Aralkyl bei jedem Vorkommen jeweils optional substituiert sind mit einem oder mehreren Substituenten unabhängig ausgewählt aus der Gruppe bestehend aus Halogen, Hydroxyl, Oxo, Amino, Cyano, Nitro, -Si(R)₃, C₁₋₆-Alkyl, gesättigtem oder teilweise ungesättigtem cyclischen C₃₋₆-Kohlenwasserstoff, gesättigtem oder teilweise ungesättigtem 3- bis 10-gliedrigen Heterocyclyl, C₆₋₁₀-Aryl, 5- bis 14-gliedrigem Heteroaryl, C₆₋₁₂-Aralkyl, -C(=O)R^{a}, -OC(=O)R^{a}, -C(=O)OR^{a}, -OR^{a}, -SR^{a}, -S(=O)R^{a}, -S(=O)₂R^{a}, -S(=O)₂NR^{a}R^{b}, -NR^{a}R^{b}, - C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -C₁₋₆-Alkylen-NR^{a}R^{b}, -C₁₋₆-Alkylen-OR^{a}, -C₁₋₆-Alkenylen-OR^{a} und -O-C₁₋₆-Alkylen-NR^{a}R^{b}, wobei das Alkyl, der cyclische Kohlenwasserstoff, das Heterocyclyl, das Aryl, das Heteroaryl und das Aralkyl ferner optional substituiert sind mit einem oder mehreren Substituenten unabhängig ausgewählt aus der Gruppe bestehend aus Halogen, Hydroxyl, Oxo, Amino, Cyano, Nitro, -NR^{a}R^{b}, C₁₋₆-Alkyl, -O-C₁₋₆-Alkyl, gesättigtem oder teilweise ungesättigtem cyclischen C₃₋₆-Kohlenwasserstoff, gesättigtem oder teilweise ungesättigtem 3- bis 10-gliedrigen Heterocyclyl, C₆₋₁₀-Aryl, 5- bis 14-gliedrigem Heteroaryl und C₆₋₁₂-Aralkyl; und
R^{a} und R^{b}, bei jedem Vorkommen, jeweils unabhängig ausgewählt sind aus der Gruppe bestehend aus H, -OH, C₁₋₆-Alkyl, C₂₋₆-Alkenyl, C₂₋₆-Alkinyl, gesättigtem oder teilweise ungesättigtem cyclischen C₃₋₁₀-Kohlenwasserstoff, gesättigtem oder teilweise ungesättigtem 3- bis 10-gliedrigen Heterocyclyl, C₆₋₁₀-Aryl, 5- bis 14-gliedrigem Heteroaryl und C₆₋₁₂-Aralkyl; alternativ R^{a} und R^{b} zusammen mit dem Atom, an das sie angehängt sind, einen 3- bis 12-gliedrigen Heterocyclus oder heteroaromatischen Ring bilden, die obigen Gruppen ferner optional substituiert sind mit einem oder mehreren Substituenten unabhängig ausgewählt aus der Gruppe bestehend aus Halogen, Hydroxyl, Oxo, Amino, Cyano, Nitro, C₁₋₆-Alkyl, -O-C₁₋₆-Alkyl, gesättigtem oder teilweise ungesättigtem cyclischen C₃₋₆-Kohlenwasserstoff, gesättigtem oder teilweise ungesättigtem 3- bis 10-gliedrigen Heterocyclyl, C₆₋₁₀-Aryl, 5- bis 14-gliedrigem Heteroaryl und C₆₋₁₂-Aralkyl.

2. Verbindung zur Verwendung nach Anspruch 1, wobei die Verbindung die Struktur von einer beliebigen der folgenden Formeln aufweist: 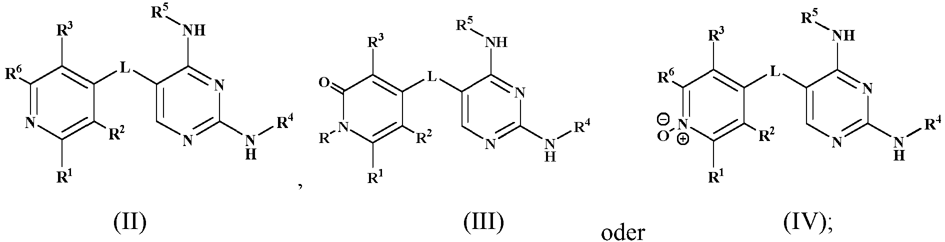
bevorzugt die Struktur von einer beliebigen der folgenden Formeln aufweist: 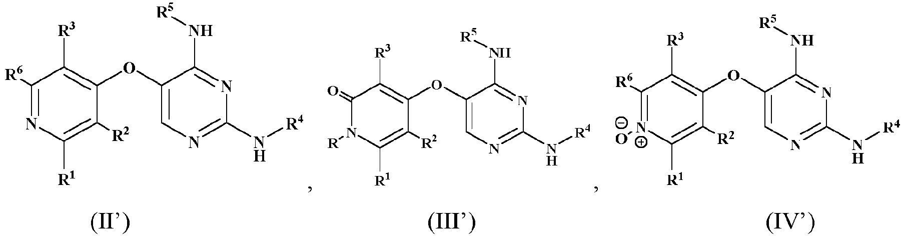 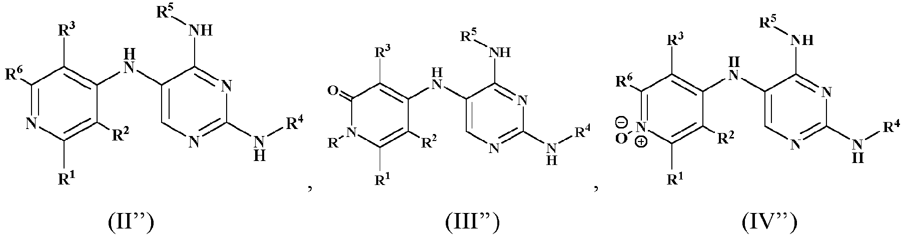 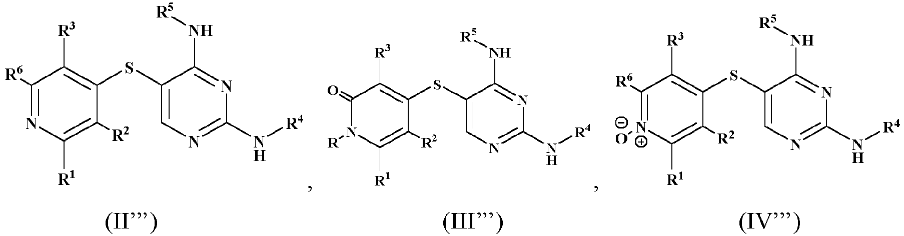 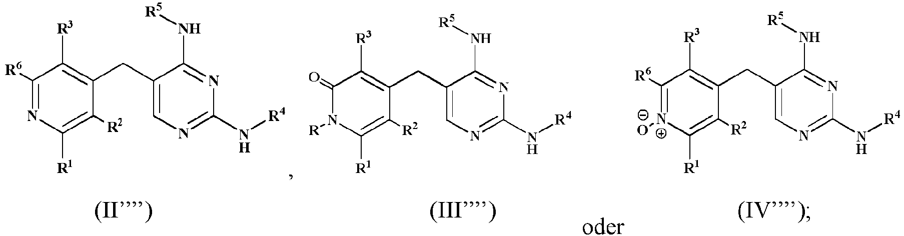
bevorzugter die Verbindung der Formel (I) die Struktur von einer beliebigen der folgenden Formeln aufweist: 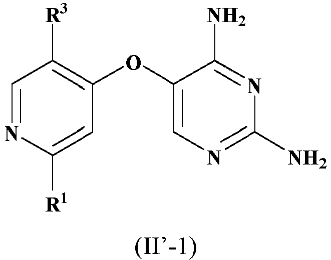
wobei:
R¹ ausgewählt ist aus der Gruppe bestehend aus F, Cl, Br, I und C₂₋₆-Alkinyl, bevorzugt Br oder Ethinyl; und
R³ C₁₋₆-Alkyl ist, bevorzugt Isopropyl.

3. Verbindung zur Verwendung nach Anspruch 1 oder 2, wobei die Verbindung die folgende Struktur aufweist:

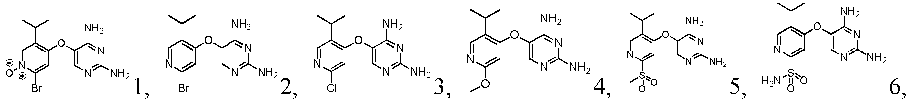

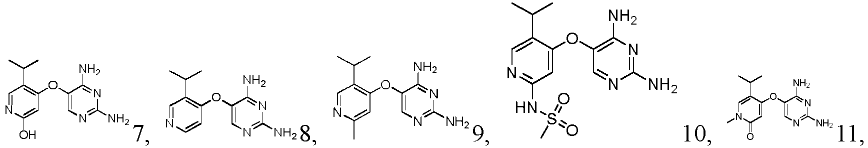

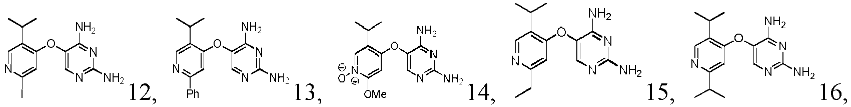

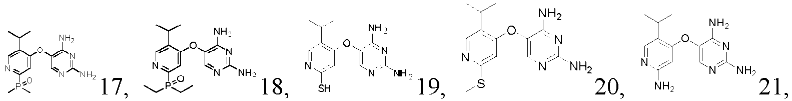

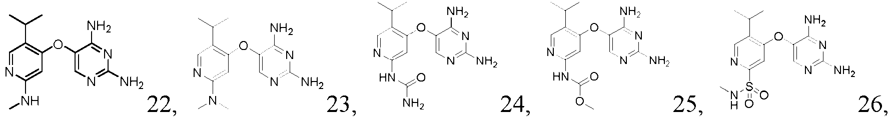

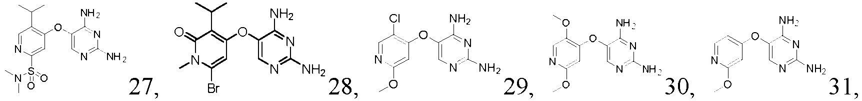

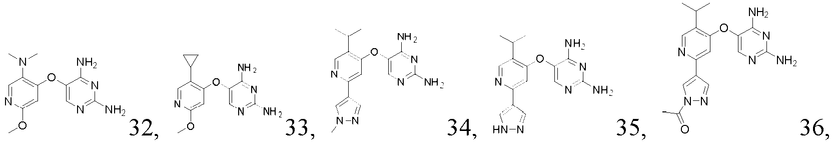

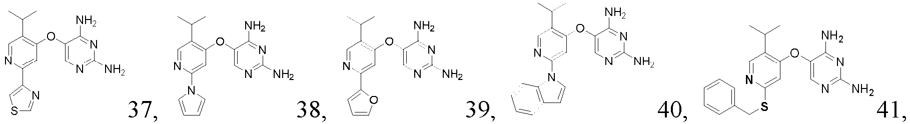

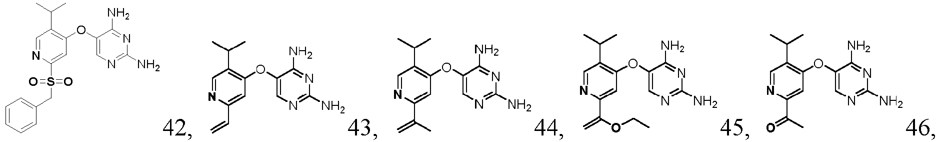

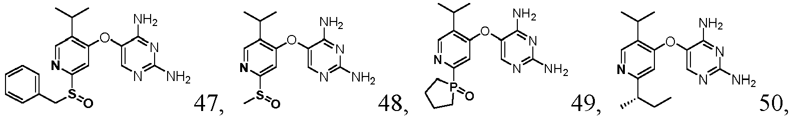

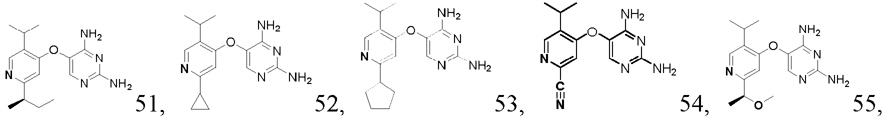

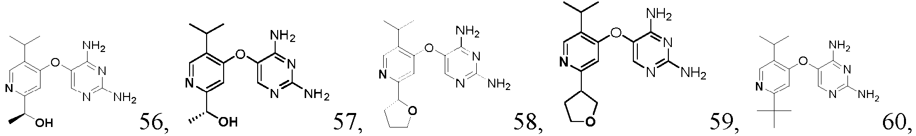

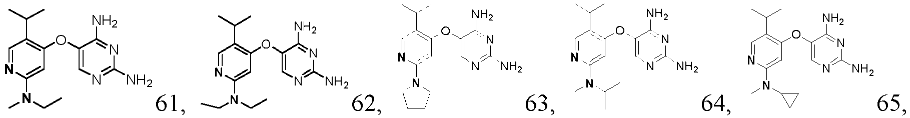

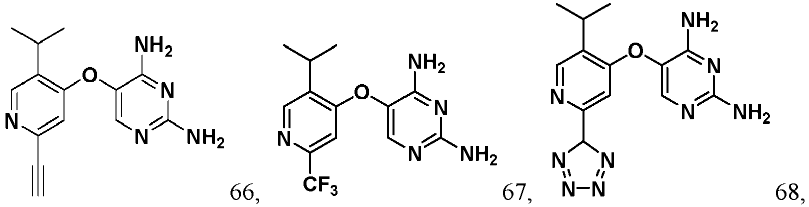

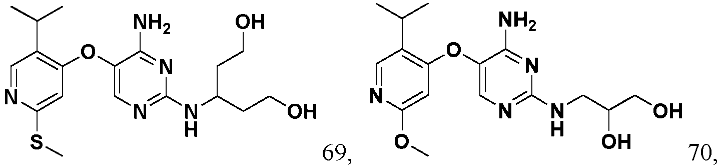

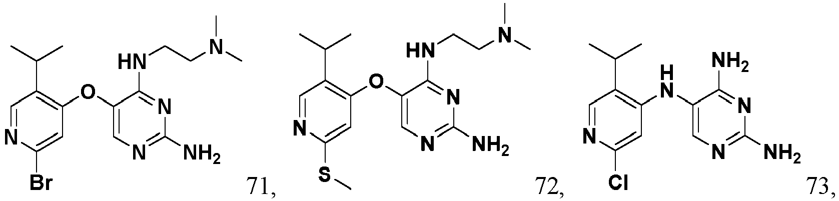

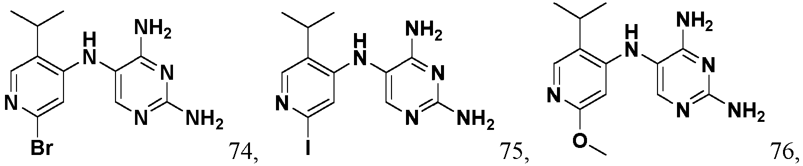

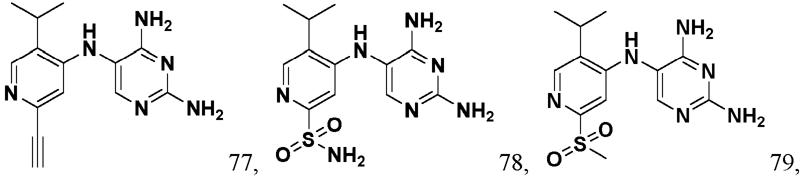

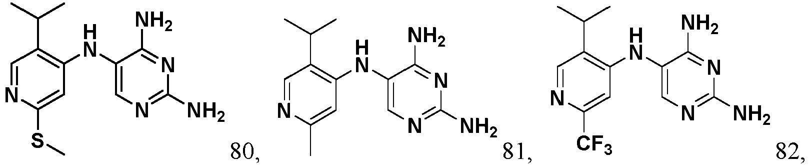

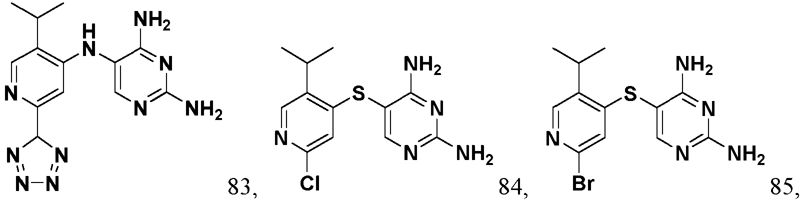

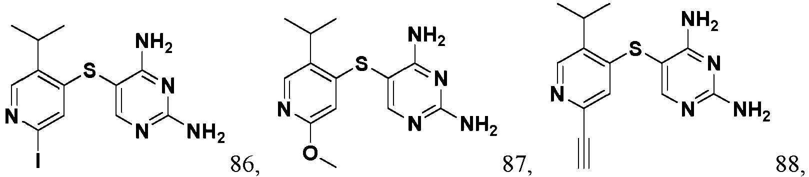

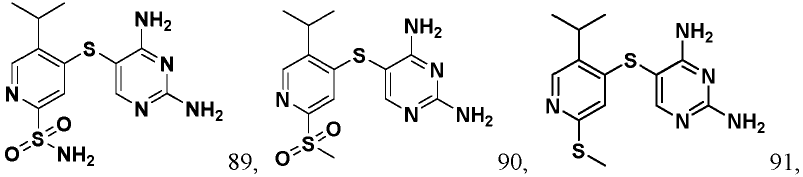

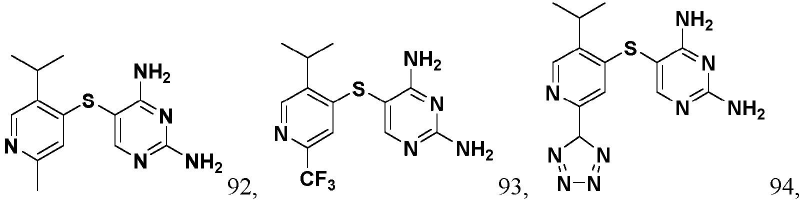

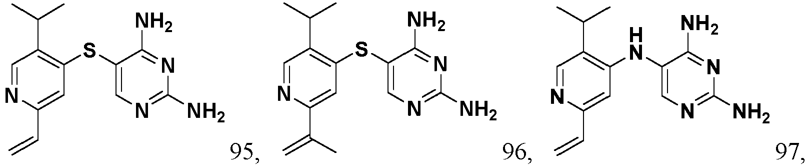

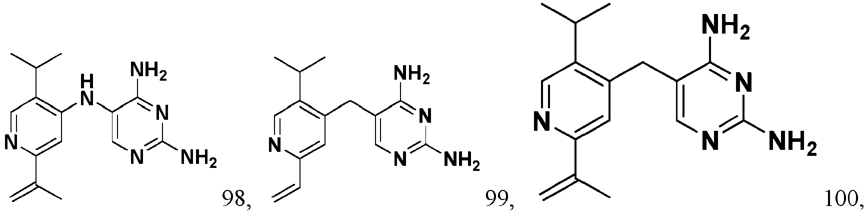

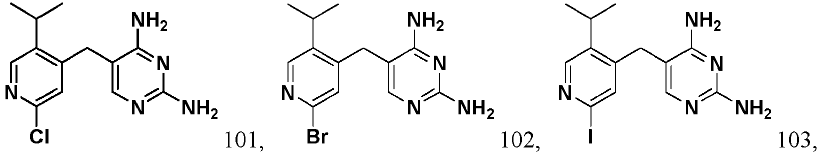

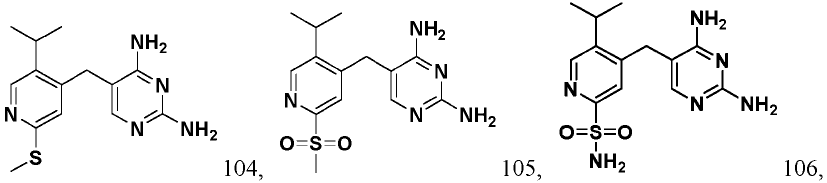

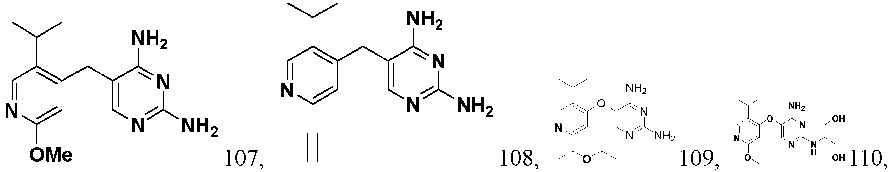

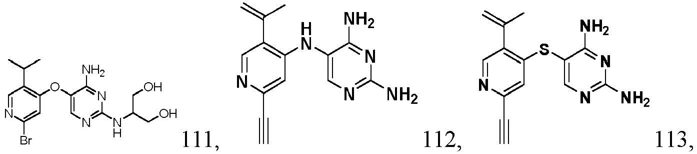

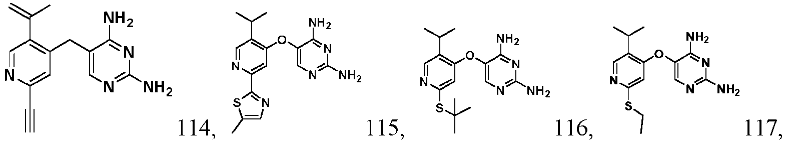

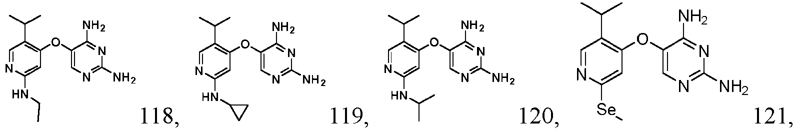

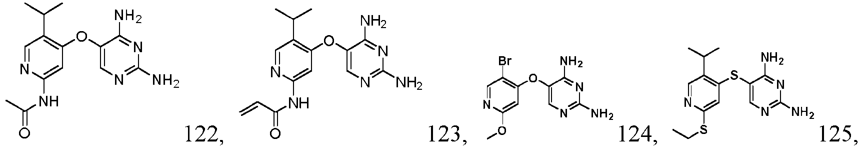

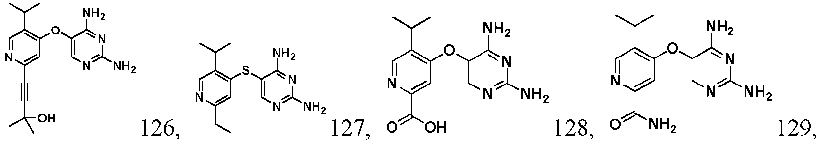

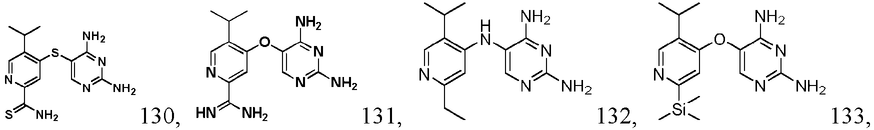

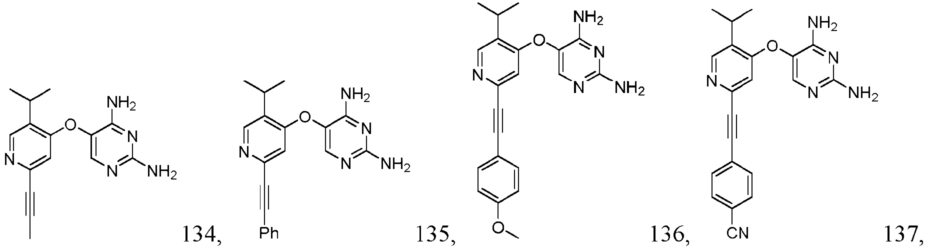

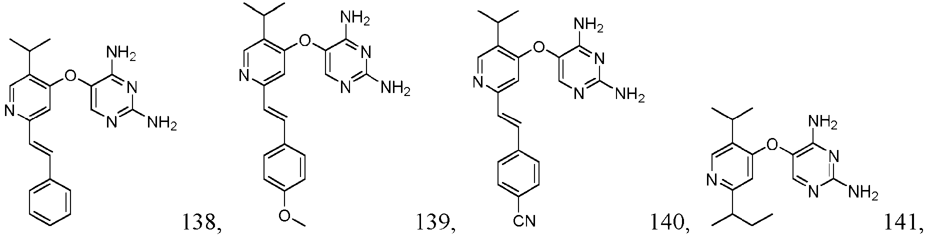

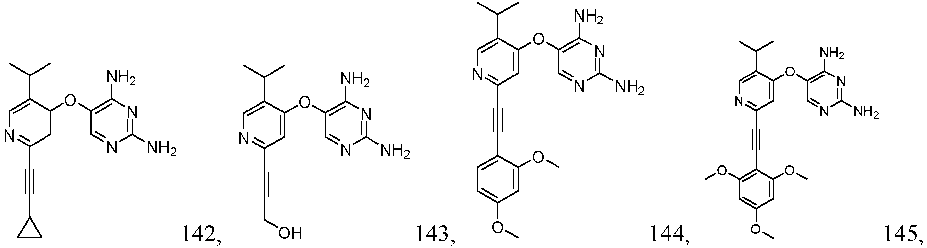

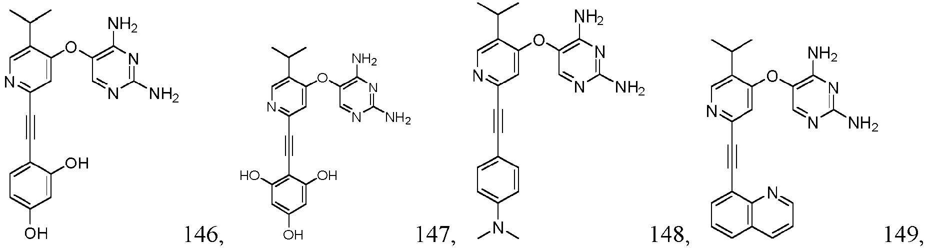

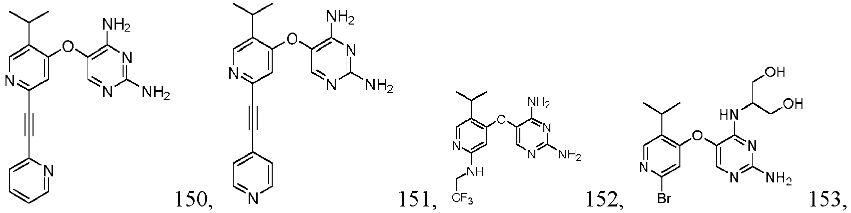

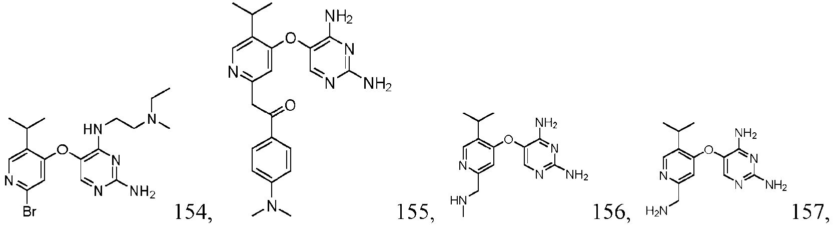

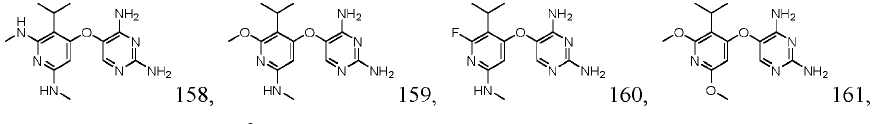

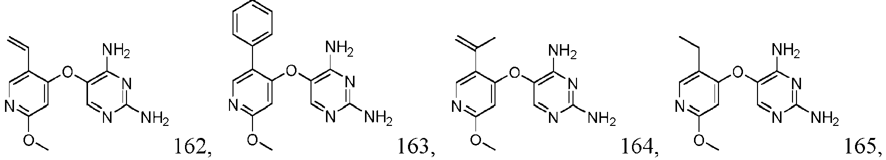

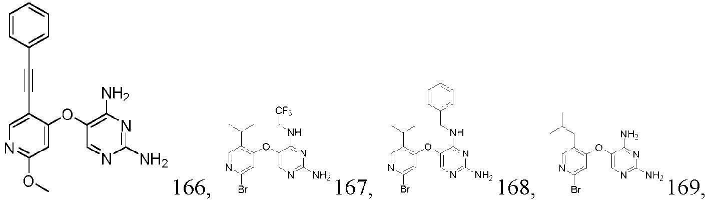

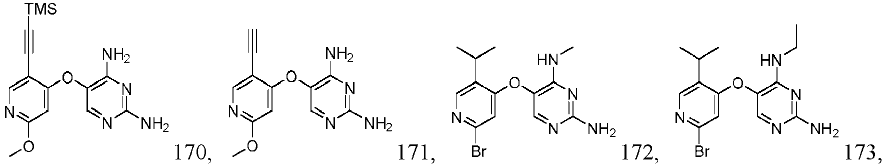

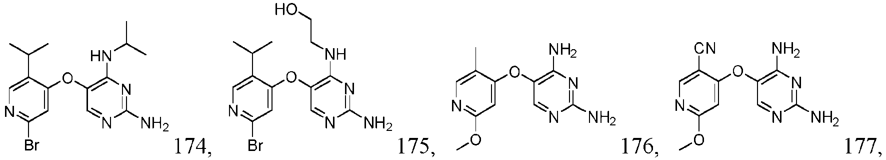

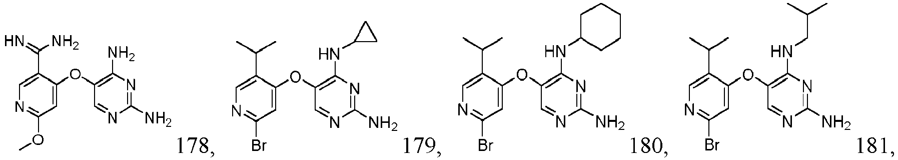

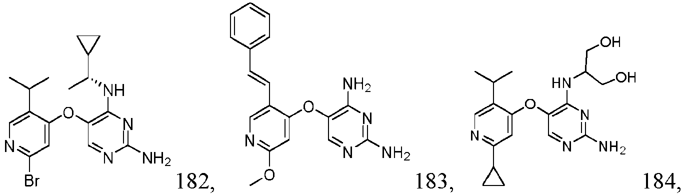

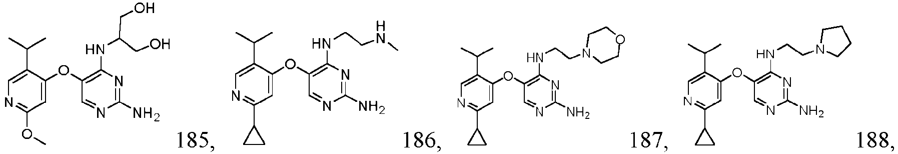

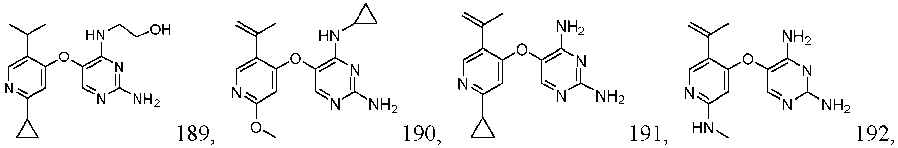

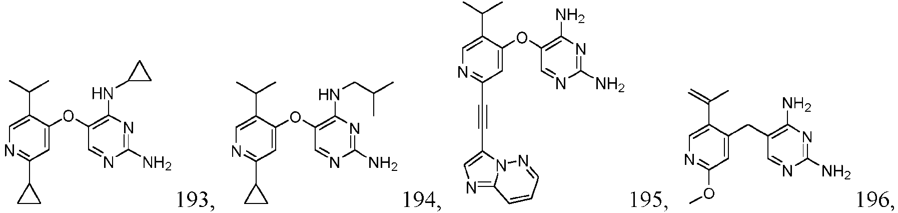

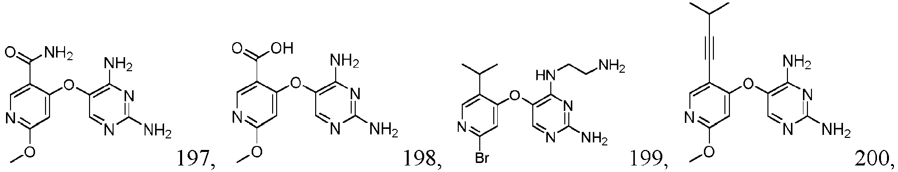

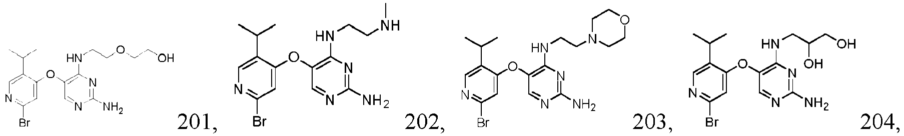

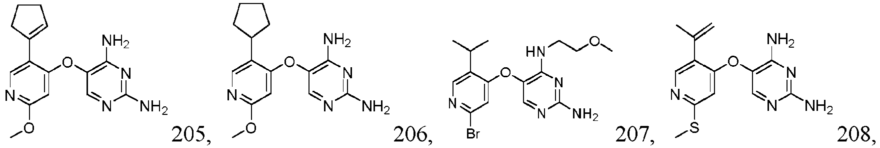

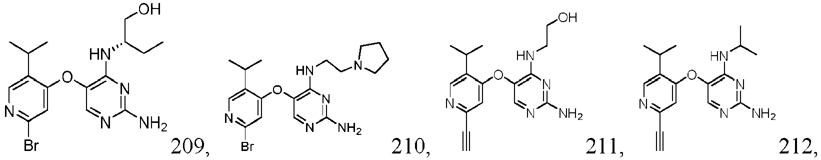

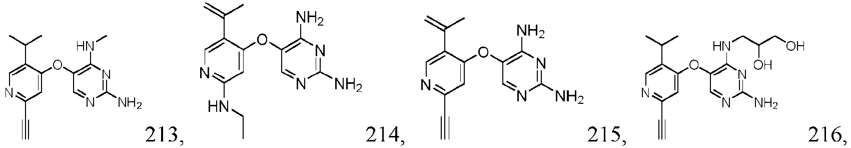

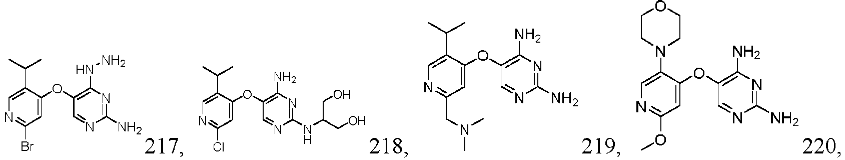

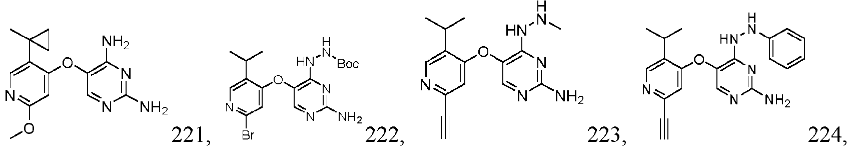

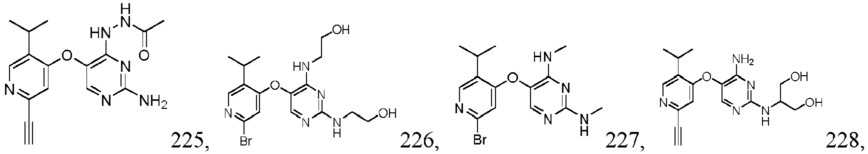

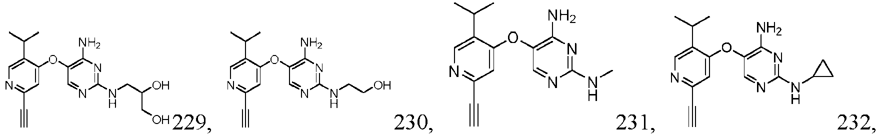

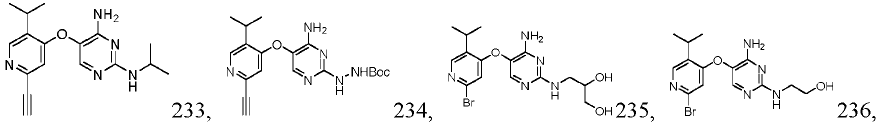

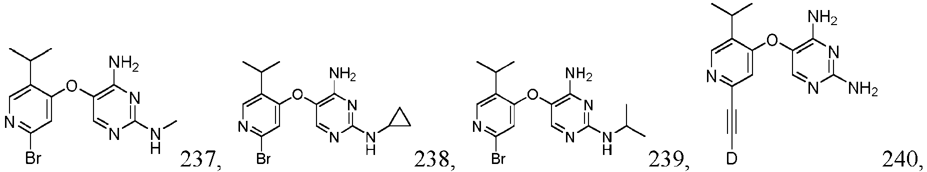

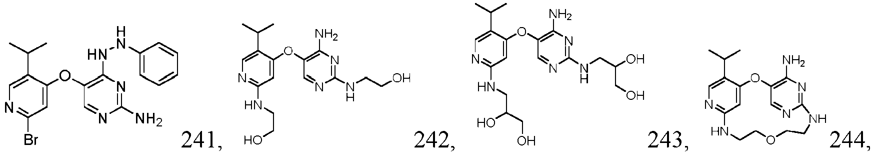

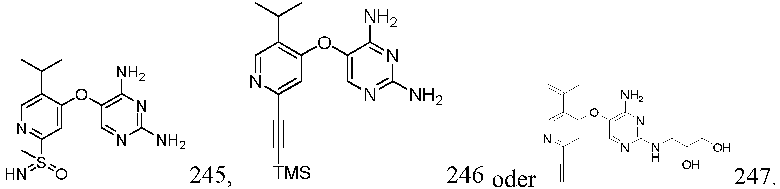

4. Verbindung zur Verwendung nach einem der Ansprüche 1 bis 3, wobei die Verbindung der Formel (I) oder ein(e) pharmazeutisch verträgliche(s)/verträglicher Salz, Ester, Stereoisomer, Polymorph, Solvat, N-Oxid oder isotopenmarkierte Verbindung davon zur Verabreichung in einer Menge von etwa 0,005 mg/Tag bis etwa 5000 mg/Tag ist, z. B. in einer Menge von etwa 0,005, 0,05, 0,5, 5, 10, 20, 30, 40, 50, 100, 150, 200, 250, 300, 350, 400, 450, 500, 550, 600, 650, 700, 750, 800, 850, 900, 950, 1000, 1500, 2000, 2500, 3000, 3500, 4000, 4500 oder 5000 mg/Tag.

5. Verbindung zur Verwendung nach einem der Ansprüche 1 bis 3, wobei die Verbindung der Formel (I) oder ein(e) pharmazeutisch verträgliche(s)/verträglicher Salt, Ester, Stereoisomer, Polymorph, Solvat, N-Oxid oder isotopenmarkierte Verbindung davon zur Verabreichung in einer Menge von etwa 1 ng/kg bis etwa 200 mg/kg, etwa 1 µg/kg bis etwa 100 mg/kg oder etwa 1 mg/kg bis etwa 50 mg/kg pro Tag ist, z. B. zur Verabreichung in einer Menge von etwa 1 µg/kg, etwa 10 µg/kg, etwa 25 µg/kg, etwa 50 µg/kg, etwa 75 µg/kg, etwa 100 µg/kg, etwa 125 µg/kg, etwa 150 µg/kg, etwa 175 µg/kg, etwa 200 µg/kg, etwa 225 µg/kg, etwa 250 µg/kg, etwa 275 µg/kg, etwa 300 µg/kg, etwa 325 µg/kg, etwa 350 µg/kg, etwa 375 µg/kg, etwa 400 µg/kg, etwa 425 µg/kg, etwa 450 µg/kg, etwa 475 µg/kg, etwa 500 µg/kg, etwa 525 µg/kg, etwa 550 µg/kg, etwa 575 µg/kg, etwa 600 µg/kg, etwa 625 µg/kg, etwa 650 µg/kg, etwa 675 µg/kg, etwa 700 µg/kg, etwa 725 µg/kg, etwa 750 µg/kg, etwa 775 µg/kg, etwa 800 µg/kg, etwa 825 µg/kg, etwa 850 µg/kg, etwa 875 µg/kg, etwa 900 µg/kg, etwa 925 µg/kg, etwa 950 µg/kg, etwa 975 µg/kg, etwa 1 mg/kg, etwa 5 mg/kg, etwa 10 mg/kg, etwa 15 mg/kg, etwa 20 mg/kg, etwa 25 mg/kg, etwa 30 mg/kg, etwa 35 mg/kg, etwa 40 mg/kg, etwa 45 mg/kg, etwa 50 mg/kg, etwa 60 mg/kg, etwa 70 mg/kg, etwa 80 mg/kg, etwa 90 mg/kg, etwa 100 mg/kg, etwa 125 mg/kg, etwa 150 mg/kg, etwa 175 mg/kg, etwa 200 mg/kg Körpergewicht pro Einheitsdosis.

6. Verbindung zur Verwendung nach einem der Ansprüche 1 bis 5, wobei die tägliche Dosis der Verbindung der Formel (I) oder eines/einer pharmazeutisch verträglichen Salzes, Esters, Stereoisomers, Polymorphs, Solvats, N-Oxids oder isotopenmarkierten Verbindung davon zur einmaligen Verabreichung ist oder zur Verabreichung in zwei, drei oder vier Dosen ist.

7. Verbindung zur Verwendung nach einem der Ansprüche 1 bis 6, wobei die Verbindung der Formel (I) oder ein(e) pharmazeutisch verträgliche(s)/verträglicher Salz, Ester, Stereoisomer, Polymorph, Solvat, N-Oxid oder isotopenmarkierte Verbindung davon zur kontinuierlichen Verabreichung für zumindest 3 Tage, zumindest 4 Tage, zumindest 5 Tage, zumindest 6 Tage, zumindest 7 Tage, zumindest 8 Tage, zumindest 9 Tage, zumindest 10 Tage, zumindest 11 Tage, zumindest 12 Tage, zumindest 13 Tage, zumindest 14 Tage, zumindest 15 Tage, zumindest 16 Tage, zumindest 17 Tage, zumindest 18 Tage, zumindest 19 Tage, zumindest 20 Tage, zumindest 21 Tage, zumindest 22 Tage, zumindest 23 Tage, zumindest 24 Tage, zumindest 25 Tage, zumindest 30 Tage, zumindest 35 Tage, zumindest 40 Tage, zumindest 45 Tage, zumindest 50 Tage, zumindest ein halbes Jahr, zumindest 1 Jahr, zumindest 2 Jahre, zumindest 3 Jahre, zumindest 4 Jahre oder zumindest 5 Jahre ist.

8. Verbindung zur Verwendung nach einem der Ansprüche 1 bis 7, wobei die Verbindung der Formel (I) oder ein(e) pharmazeutisch verträgliche(s)/verträglicher Salz, Ester, Stereoisomer, Polymorph, Solvat, N-Oxid oder isotopenmarkierte Verbindung davon zur Verabreichung für einen oder mehrere (z. B. 1, 2, 3, 4, 5, 6, 7, 8, 9 oder 10) Behandlungszyklen ist, wobei jeder Behandlungszyklus zumindest 3 Tage, zumindest 4 Tage, zumindest 5 Tage, zumindest 6 Tage, zumindest 7 Tage, zumindest 8 Tage, zumindest 9 Tage, zumindest 10 Tage, zumindest 11 Tage, zumindest 12 Tage, zumindest 13 Tage, zumindest 14 Tage, zumindest 15 Tage, zumindest 16 Tage, zumindest 17 Tage, zumindest 18 Tage, zumindest 19 Tage, zumindest 20 Tage, zumindest 21 Tage, zumindest 22 Tage, zumindest 23 Tage, zumindest 24 Tage, zumindest 25 Tage, zumindest 30 Tage, zumindest 35 Tage, zumindest 40 Tage, zumindest 45 Tage oder zumindest 50 Tage dauert; und das Intervall zwischen jeweils zwei Behandlungszyklen 0, 1, 2, 3, 4, 5, 6, 7, 8, 9, 10 Tage, zwei Wochen, drei Wochen oder vier Wochen ist.

9. Verbindung zur Verwendung nach einem der Ansprüche 1 bis 8, wobei die Verbindung der Formel (I) oder ein(e) pharmazeutisch verträgliche(s)/verträglicher Salz, Ester, Stereoisomer, Polymorph, Solvat, N-Oxid oder isotopenmarkierte Verbindung davon zur Verabreichung durch Injektion (z. B. intravenöse, intraarterielle, subkutane, intraperitoneale, intramuskuläre Injektion, beinhaltend Tropfen) oder transdermal zur Verabreichung ist oder zur Verabreichung über oralen, bukkalen, nasalen, transmukosalen oder topischen Weg, als ophthalmische Formulierung oder über Inhalation ist.

10. Verbindung zur Verwendung nach einem der Ansprüche 1 bis 9, wobei die Verbindung der Formel (I) oder ein(e) pharmazeutisch verträgliche(s)/verträglicher Salz, Ester, Stereoisomer, Polymorph, Solvat, N-Oxid oder isotopenmarkierte Verbindung davon zur Verabreichung in einer Dosierungsform ausgewählt aus der Gruppe bestehend aus Tablette, Kapsel, Pastille, Bonbon, Pulver, Spray, Emulsion, Creme, Salbe, Zäpfchen, Gel, Paste, Lotion, Injektion, Nanoformulierung, Pflaster, wässriger Suspension, Lösung, Elixier und Sirup ist.

11. Verbindung zur Verwendung nach einem der Ansprüche 1 bis 10, wobei der Husten ausgewählt ist aus der Gruppe bestehend aus akutem Husten, subakutem Husten, chronischem Husten, behandlungsresistentem Husten, behandlungsresistentem chronischem Husten, idiopathischem chronischem Husten, postviralem Husten, iatrogenem Husten, Husten assoziiert mit postnasalem Tropf, Husten assoziiert mit Erkältung, Infektion der oberen Atemwege, Asthma, Lungenkrebs und/oder chronisch obstruktiver Lungenerkrankung (COPD), Husten assoziiert mit interstitieller Erkrankung, Husten assoziiert mit Lungenfibrose, Husten assoziiert mit gastroösophagealer Refluxerkrankung (GERD), Husten assoziiert mit Rauchen oder einer Form von Bronchitis und neuronaler Überempfindlichkeit, die akutem, subakutem oder chronischem Husten zugrunde liegt; bevorzugt der Husten ausgewählt ist aus der Gruppe bestehend aus chronischem Husten, behandlungsresistentem Husten, behandlungsresistentem chronischem Husten und Husten assoziiert mit Lungenfibrose.

## Revendications

1. Composé de Formule (I) ou sel, ester, stéréoisomère, polymorphe, solvate, N-oxyde ou composé isotopiquement marqué acceptable pharmaceutiquement de celui-ci destiné à être utilisé pour traiter, supprimer ou soulager la toux ou l'envie de tousser : 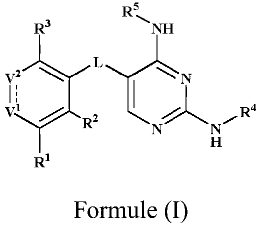 dans lequel :
L est choisi dans le groupe constitué par C(=O), CRR', NR, O, S, S=O et S(=O)₂ ;
V¹ est choisi dans le groupe constitué par N, 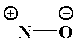 et NR ;
V² est choisi dans le groupe constitué par CR⁶ et C(=O) ;
--- représente soit une simple liaison soit une double liaison, à condition que lorsque --- est une simple liaison, V¹ est NR et V² est C(=O) ;
R et R' sont chacun indépendamment choisis dans le groupe constitué par H, un halogène, un alkyle en C₁₋₆, un alcényle en C₂₋₆, un alcynyle en C₂₋₆, un hydrocarbyle cyclique en C₃₋₁₀ saturé ou partiellement insaturé, un hétérocyclyle à 3 à 10 chaînons saturé ou partiellement insaturé, un aryle en C₆₋₁₀, un hétéroaryle à 5 à 14 chaînons et un aralkyle en C₆₋₁₂, et au plus 2 chaînons de cycle dans l'hydrocarbyle cyclique et l'hétérocyclyle sont C(=O) ;
R¹, R², R³ et R⁶ sont chacun indépendamment choisis dans le groupe constitué par H, un halogène, -CN, -NO₂, -NH₂, -OH, -SH, -Se-R, -Si(R)₃, un alkyle en C₁₋₆, un alcényle en C₂₋₆, un alcynyle en C₂₋₆, un hydrocarbyle cyclique en C₃₋₁₀ saturé ou partiellement insaturé, un hétérocyclyle à 3 à 10 chaînons saturé ou partiellement insaturé, un aryle en C₆₋₁₀, un hétéroaryle à 5 à 14 chaînons, un aralkyle en C₆₋₁₂, un halogénoalkyle en C₁₋₆, -C(=O)R^{a}, -OC(=O)R^{a}, - C(=O)OR^{a}, -OR^{a}, -SR^{a}, -S(=O)R^{a}, -S(=O)₂R^{a}, -S(=O)₂NR^{a}R^{b}, -S(=O)(=NR)R^{a}, -NR^{a}R^{b}, - C(=O)NR^{a}R^{b}, -C(=S)NR^{a}R^{b}, -C(=NR)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -alkylène en C₁₋₆-NR^{a}R^{b}, -alkylène en C₁₋₆-OR^{a}, -alkylène en C₁₋₆-C(=O)R, - alcénylène en C₁₋₆-OR^{a}, -O-alkylène en C₁₋₆-NR^{a}R^{b} et -P(=O)R^{a}R^{b} ;
R⁴ et R⁵ sont chacun indépendamment choisis dans le groupe constitué par H, -C(=O)OR^{a}, -NR^{a}R^{b}, -NR^{a}-C(=O)R^{b}, -NR^{a}-C(=O)OR^{b}, -alkylène en C₁₋₆-NR^{a}R^{b}, -alkylène en C₁₋₆-OR^{a}, - alkylène en C₁₋₆-O-alkylène en C₁₋₆-OR^{a}, un alkyle en C₁₋₆, un halogénoalkyle en C₁₋₆, un alcényle en C₂₋₆, un alcynyle en C₂₋₆, un hydrocarbyle cyclique en C₃₋₁₀ saturé ou partiellement insaturé, un hétérocyclyle à 3 à 10 chaînons saturé ou partiellement insaturé, un aryle en C₆₋₁₀, un hétéroaryle à 5 à 14 chaînons et un aralkyle en C₆₋₁₂ ;
alternativement, R¹ et R⁴ forment ensemble -NHCH₂CH₂-O-CH₂CH₂- ;
l'alkyle, l'alkylène, l'alcényle, l'alcynyle, l'hydrocarbyle cyclique, l'hétérocyclyle, l'aryle, l'hétéroaryle et l'aralkyle ci-dessus, à chaque occurrence, sont chacun éventuellement substitués par un ou plusieurs substituants choisis indépendamment dans le groupe constitué par un halogène, un hydroxyle, un oxo, un amino, un cyano, un nitro, -Si(R)₃, un alkyle en C₁₋₆, un hydrocarbyle cyclique en C₃₋₆ saturé ou partiellement insaturé, un hétérocyclyle à 3 à 10 chaînons saturé ou partiellement insaturé, un aryle en C₆₋₁₀, un hétéroaryle à 5 à 14 chaînons, un aralkyle en C₆₋₁₂, - C(=O)R^{a}, -OC(=O)R^{a}, -C(=O)OR^{a}, -OR^{a}, -SR^{a}, -S(=O)R^{a}, -S(=O)₂R^{a}, -S(=O)₂NR^{a}R^{b}, -NR^{a}R^{b}, - C(=O)NR^{a}R^{b}, -NR^{a}-C(=O)R^{b}, -NR^{a}-C(=O)OR^{b}, -NR^{a}-S(=O)₂-R^{b}, -NR^{a}-C(=O)-NR^{a}R^{b}, -alkylène en C₁₋₆-NR^{a}R^{b}, -alkylène en C₁₋₆-OR^{a}, -alcénylène en C₁₋₆-OR^{a} et -O-alkylène en C₁₋₆-NR^{a}R^{b}, l'alkyle, l'hydrocarbyle cyclique, l'hétérocyclyle, l'aryle, l'hétéroaryle et l'aralkyle sont en outre éventuellement substitués par un ou plusieurs substituants choisis indépendamment dans le groupe constitué par un halogène, un hydroxyle, un oxo, un amino, un cyano, un nitro, -NR^{a}R^{b}, un alkyle en C₁₋₆, -O-alkyle en C₁₋₆, un hydrocarbyle cyclique en C₃₋₆ saturé ou partiellement insaturé, ou un hétérocyclyle à 3 à 10 chaînons saturé ou partiellement insaturé, un aryle en C₆₋₁₀, un hétéroaryle à 5 à 14 chaînons et un aralkyle en C₆₋₁₂ ; et
R^{a} et R^{b}, à chaque occurrence, sont chacun indépendamment choisis dans le groupe constitué par H, -OH, un alkyle en C₁₋₆, un alcényle en C₂₋₆, un alcynyle en C₂₋₆, un hydrocarbyle cyclique en C₃₋₁₀ saturé ou partiellement insaturé, un hétérocyclyle à 3 à 10 chaînons saturé ou partiellement insaturé, un aryle en C₆₋₁₀, un hétéroaryle à 5 à 14 chaînons et un aralkyle en C₆₋₁₂ ; alternativement, R^{a} et R^{b} conjointement avec l'atome auquel ils sont liés forment un hétérocycle à 3 à 12 chaînons ou un cycle hétéroaromatique, les groupes ci-dessus sont en outre éventuellement substitués par un ou plusieurs substituants choisis indépendamment dans le groupe constitué par un halogène, un hydroxyle, un oxo, un amino, un cyano, un nitro, un alkyle en C₁₋₆, -O-alkyle en C₁₋₆, un hydrocarbyle cyclique en C₃₋₆ saturé ou partiellement insaturé, un hétérocyclyle à 3 à 10 chaînons saturé ou partiellement insaturé, un aryle en C₆₋₁₀, un hétéroaryle à 5 à 14 chaînons et un aralkyle en C₆₋₁₂.

2. Composé destiné à être utilisé selon la revendication 1, dans lequel le composé présente la structure de l'une quelconque des formules suivantes : 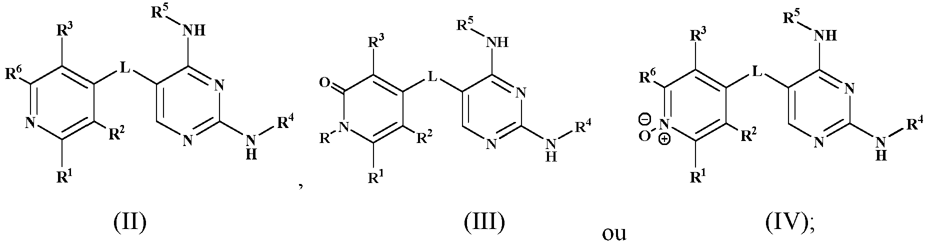
de préférence présente la structure de l'une quelconque des formules suivantes : 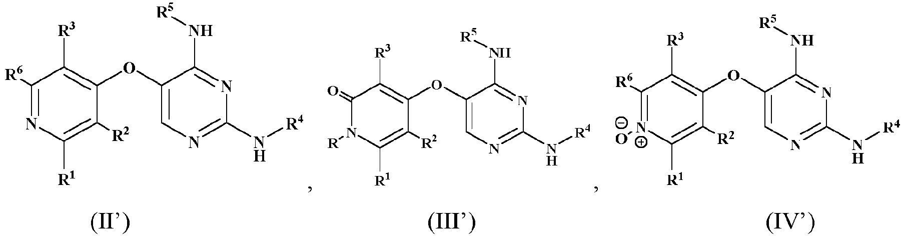 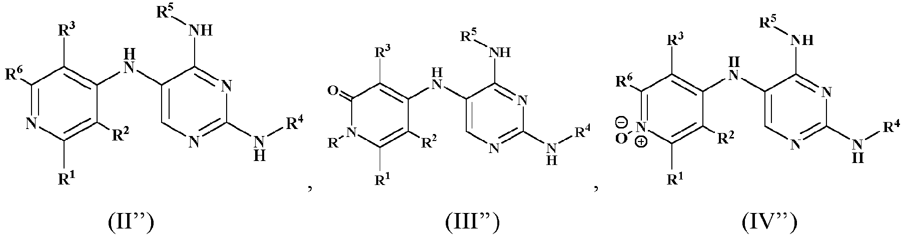 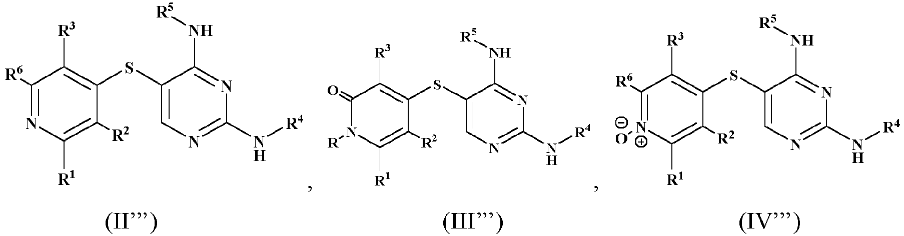 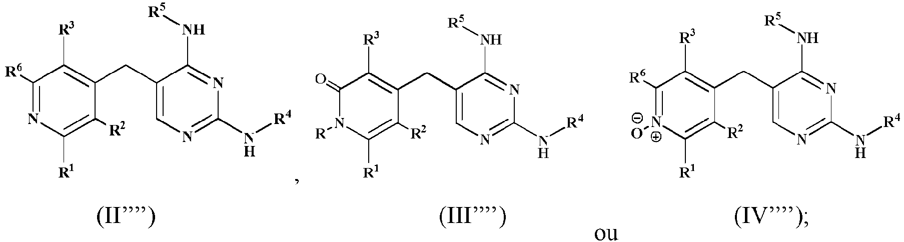
idéalement, le composé de Formule (I) présente la structure de l'une quelconque des formules suivantes : 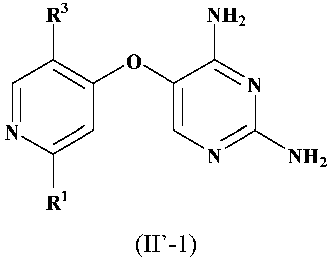
dans lequel :
R¹ est choisi dans le groupe constitué par F, Cl, Br, I et un alcynyle en C₂₋₆, de préférence Br ou un éthynyle ; et
R³ est un alkyle en C₁₋₆, de préférence un isopropyle.

3. Composé destiné à être utilisé selon l'une des revendications 1 ou 2, dans lequel le composé présente la structure suivante :

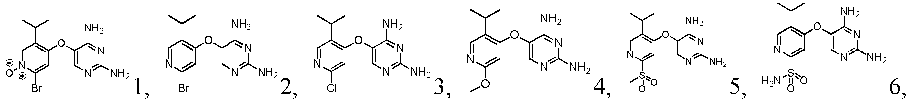

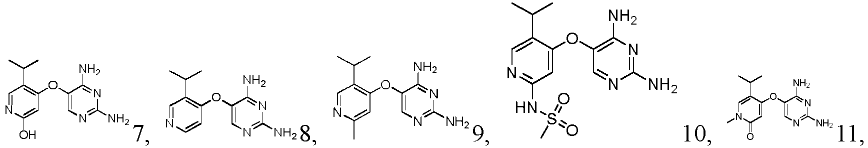

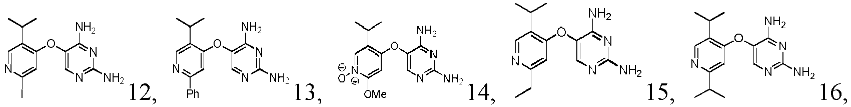

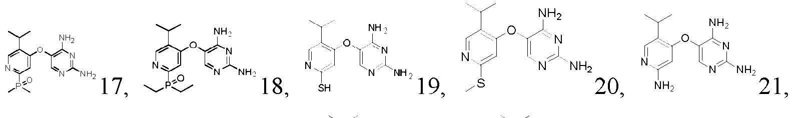

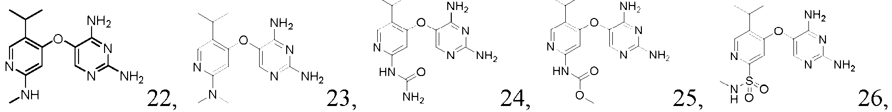

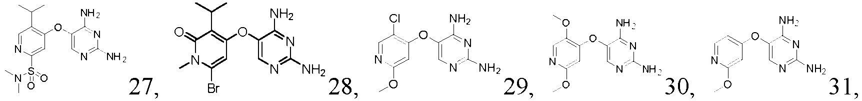

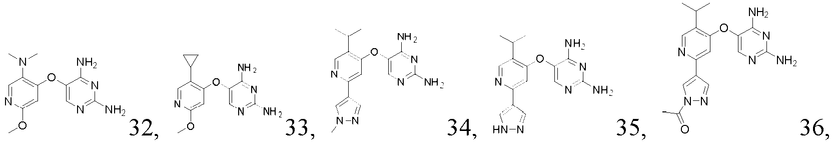

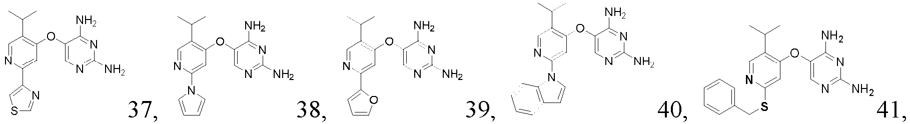

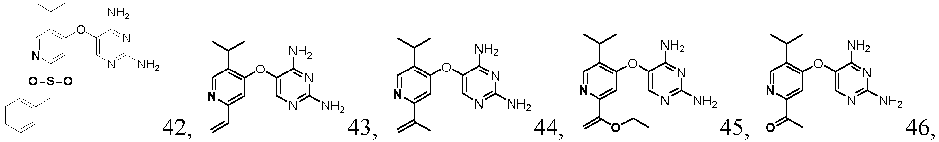

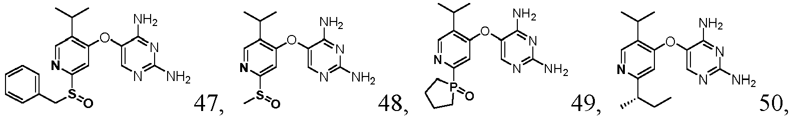

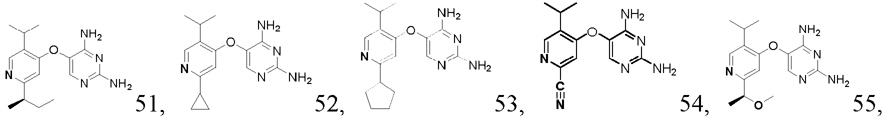

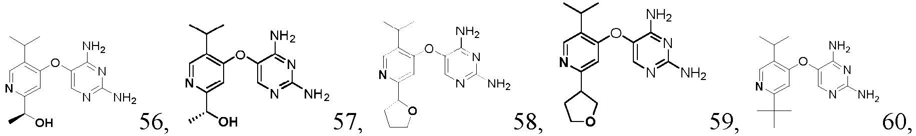

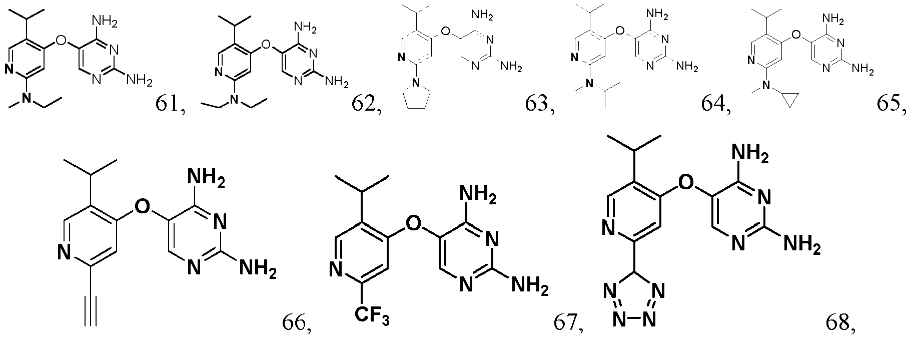

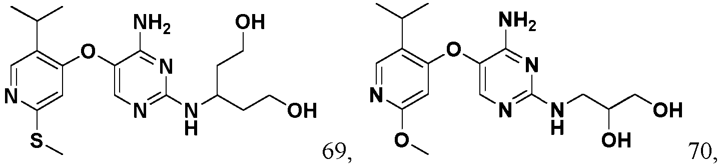

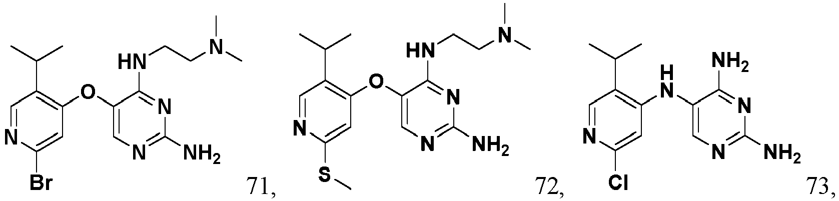

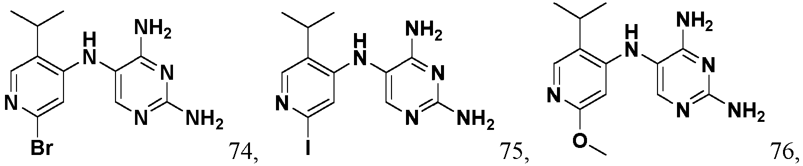

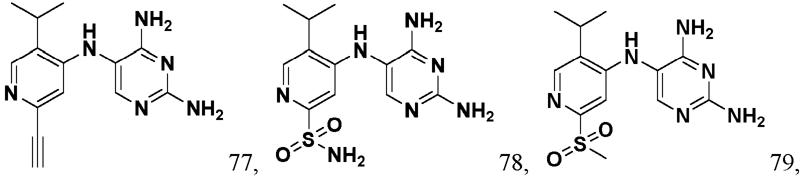

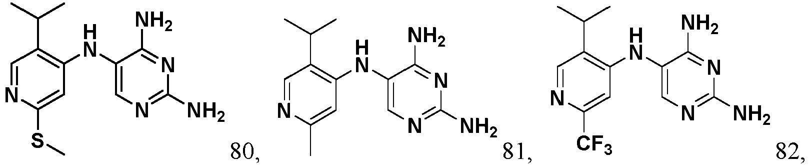

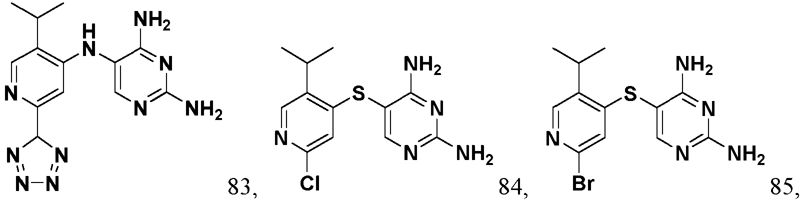

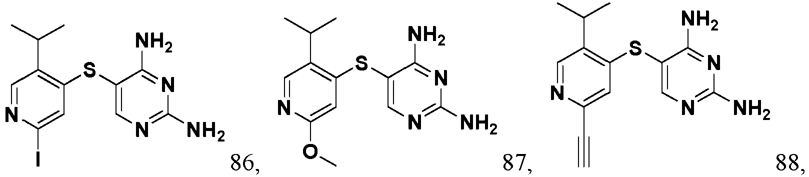

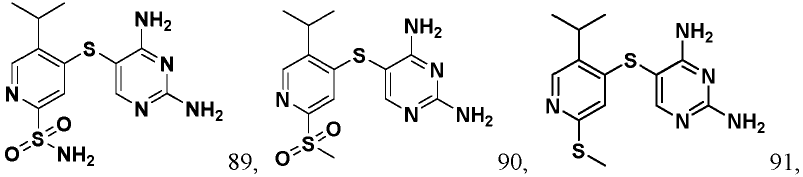

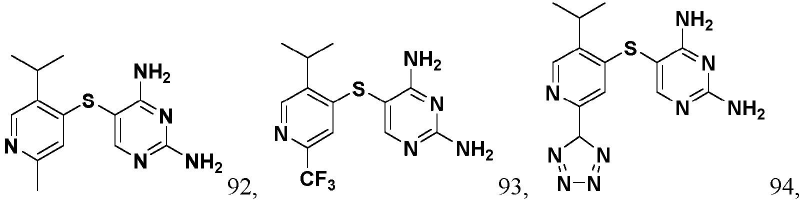

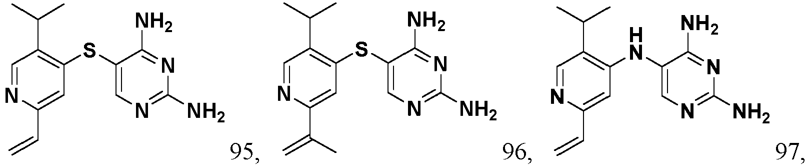

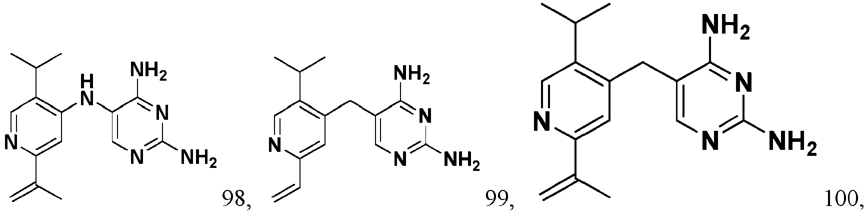

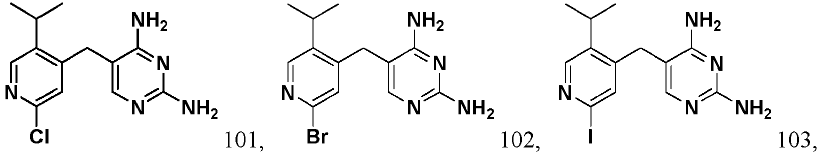

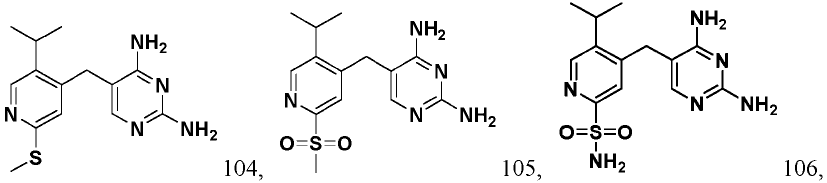

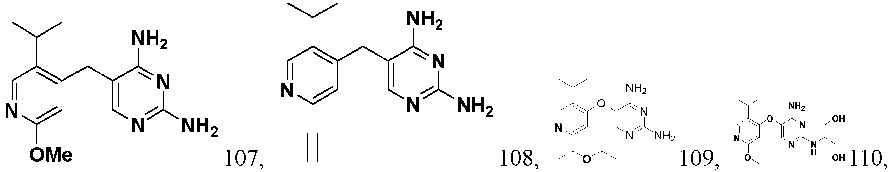

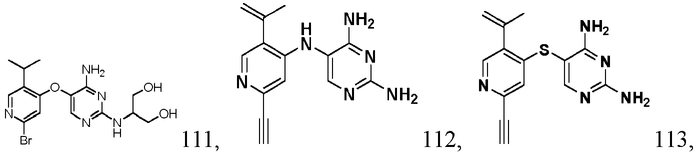

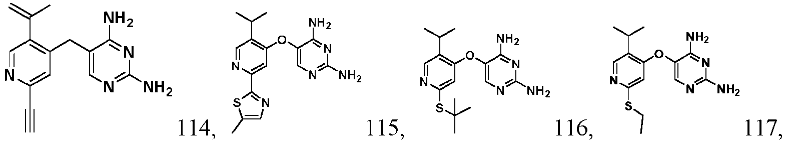

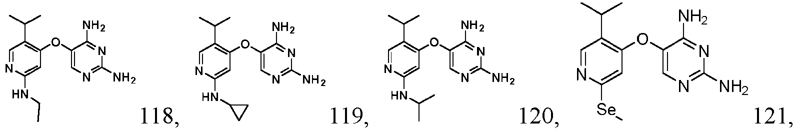

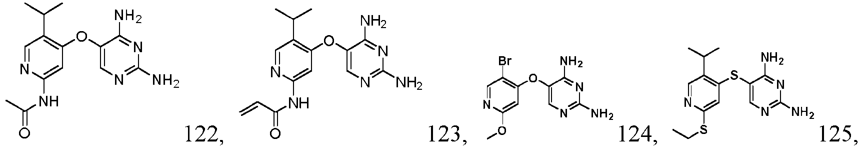

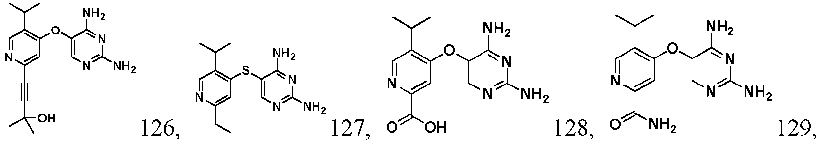

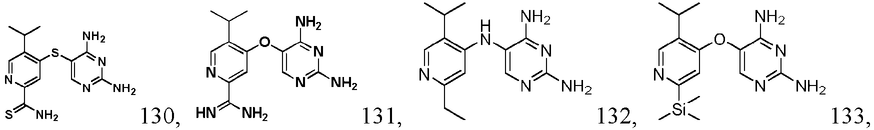

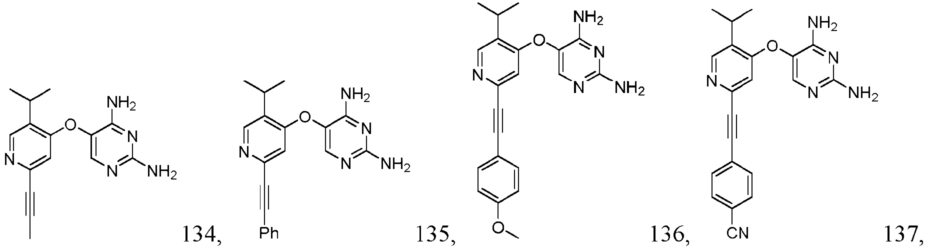

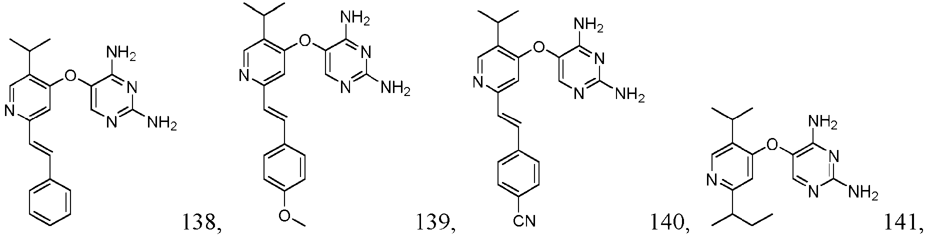

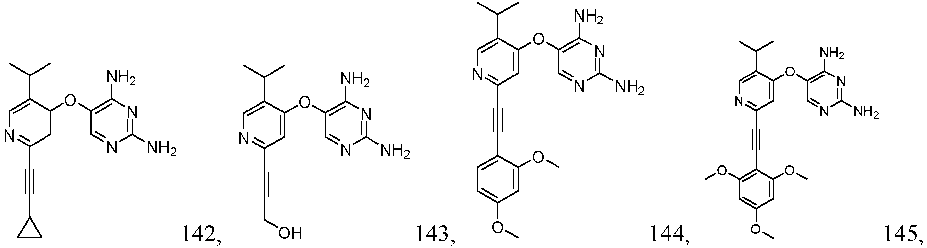

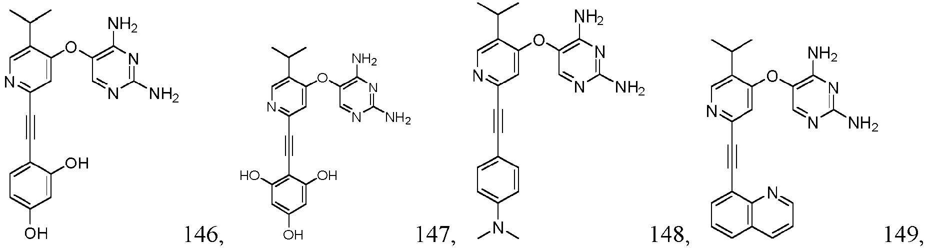

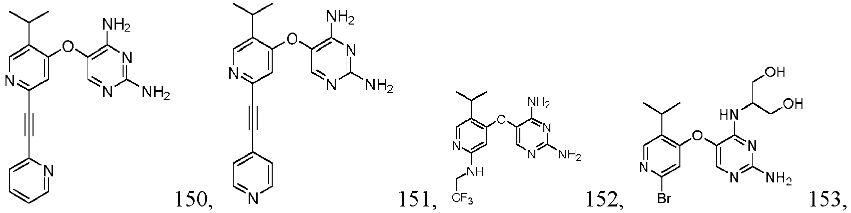

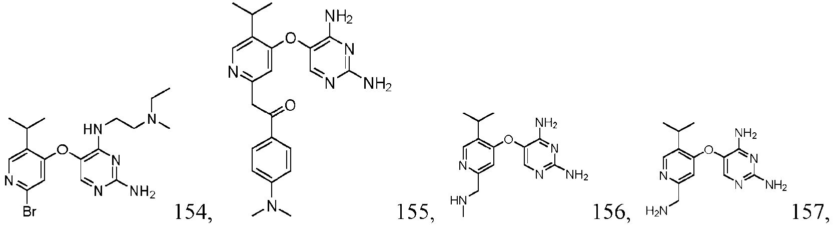

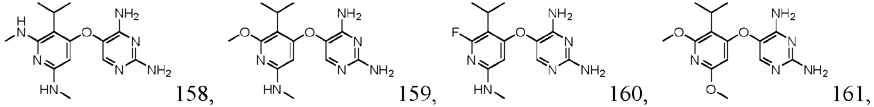

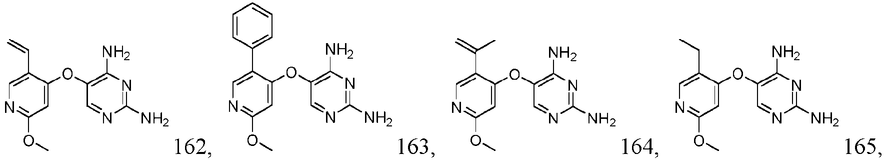

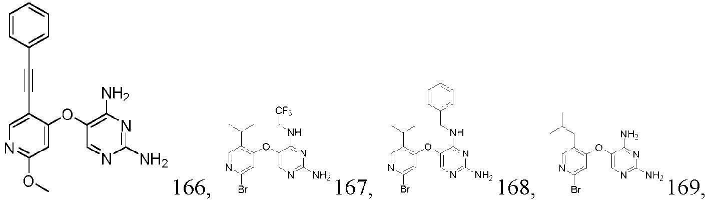

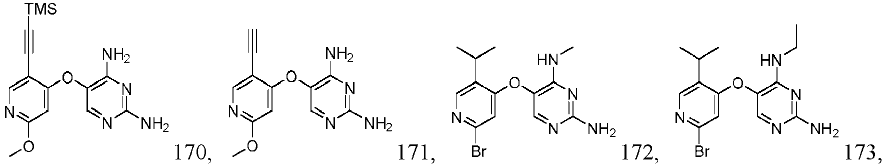

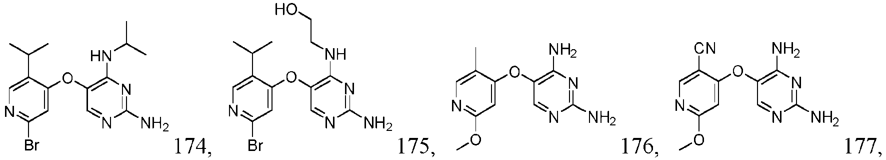

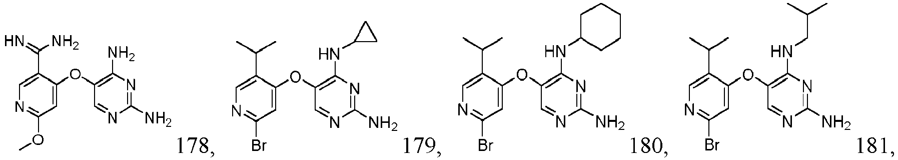

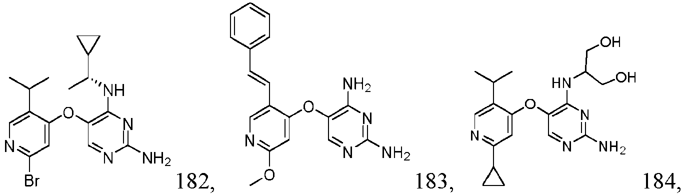

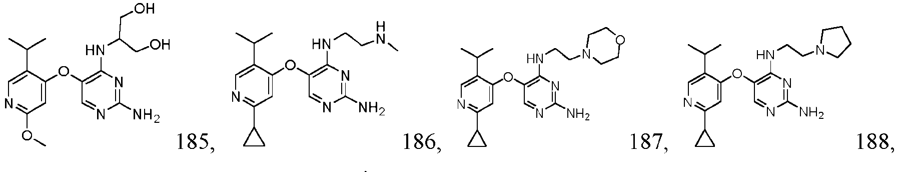

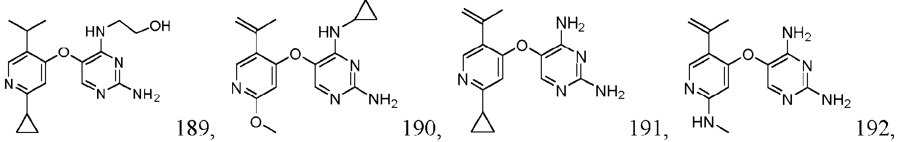

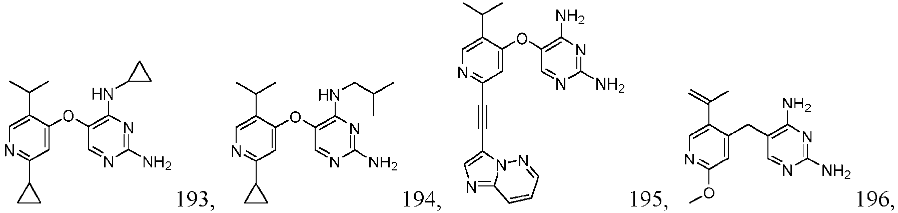

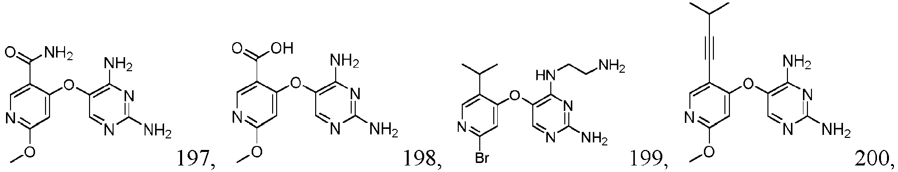

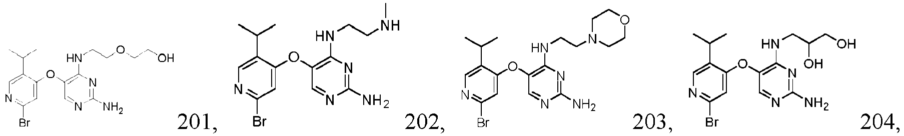

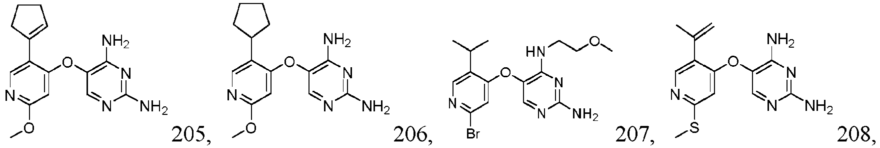

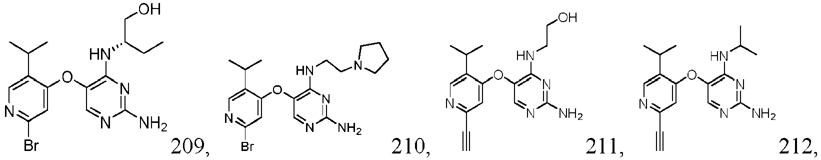

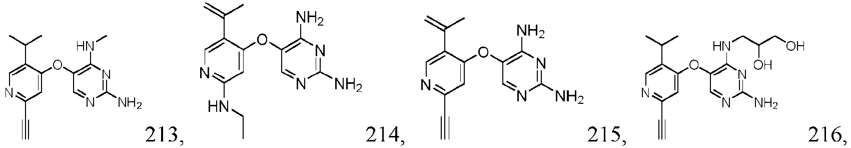

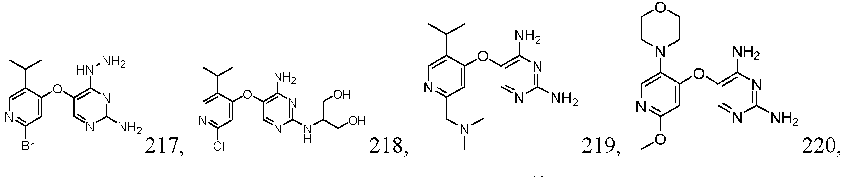

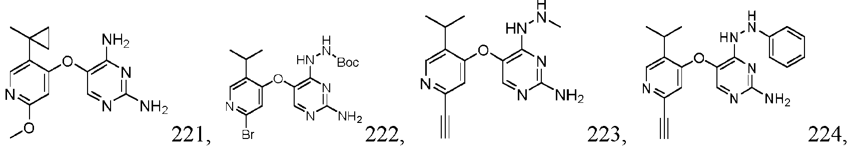

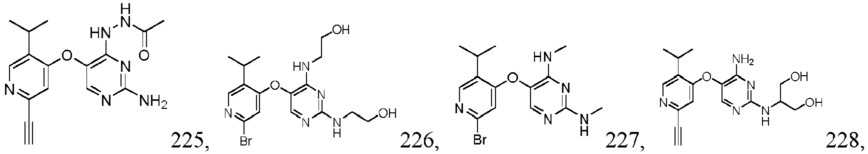

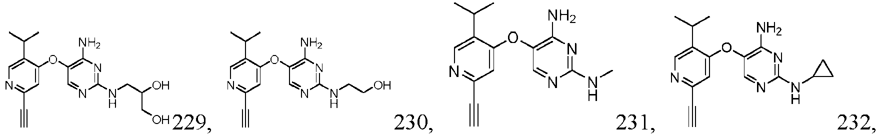

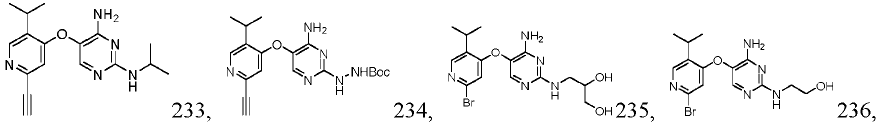

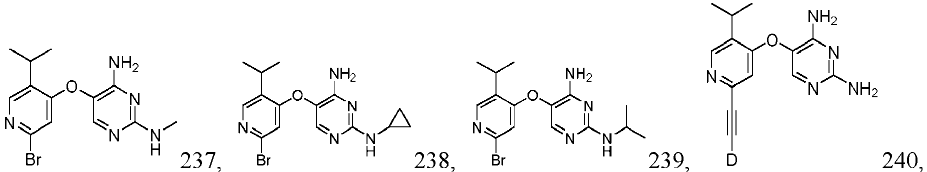

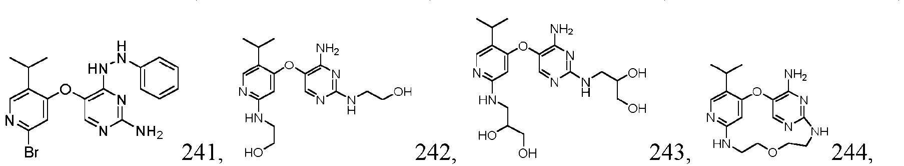

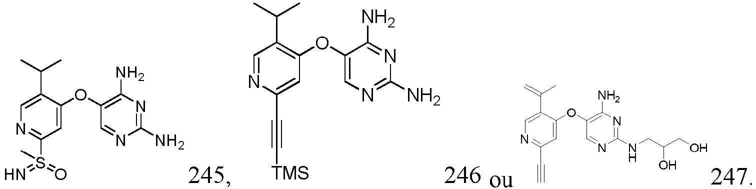

4. Composé destiné à être utilisé selon l'une quelconque des revendications 1 à 3, dans lequel le composé de Formule (I) ou un sel, ester, stéréoisomère, polymorphe, solvate, N-oxyde ou composé isotopiquement marqué acceptable pharmaceutiquement de celui-ci est destiné à être administré en une quantité d'environ 0,005 mg/jour à environ 5000 mg/jour, par exemple, en une quantité d'environ 0,005, 0,05, 0,5, 5, 10, 20, 30, 40, 50, 100, 150, 200, 250, 300, 350, 400, 450, 500, 550, 600, 650, 700, 750, 800, 850, 900, 950, 1000, 1500, 2000, 2500, 3000, 3500, 4000, 4500 ou 5000 mg/jour.

5. Composé destiné à être utilisé selon l'une quelconque des revendications 1 à 3, dans lequel le composé de Formule (I) ou un sel, ester, stéréoisomère, polymorphe, solvate, N-oxyde ou composé isotopiquement marqué acceptable pharmaceutiquement de celui-ci est destiné à être administré en une quantité d'environ 1 ng/kg à environ 200 mg/kg, d'environ 1 µg/kg à environ 100 mg/kg ou d'environ 1 mg/kg à environ 50 mg/kg par jour, par exemple, est destiné à être administré en une quantité d'environ 1 µg/kg, d'environ 10 µg/kg, d'environ 25 µg/kg, d'environ 50 µg/kg, d'environ 75 µg/kg, d'environ 100 µg/kg, d'environ 125 µg/kg, d'environ 150 µg/kg, d'environ 175 µg/kg, d'environ 200 µg/kg, d'environ 225 µg/kg, d'environ 250 µg/kg, d'environ 275 µg/kg, d'environ 300 µg/kg, d'environ 325 µg/kg, d'environ 350 µg/kg, d'environ 375 µg/kg, d'environ 400 µg/kg, d'environ 425 µg/kg, d'environ 450 µg/kg, d'environ 475 µg/kg, d'environ 500 µg/kg, d'environ 525 µg/kg, d'environ 550 µg/kg, d'environ 575 µg/kg, d'environ 600 µg/kg, d'environ 625 µg/kg, d'environ 650 µg/kg, d'environ 675 µg/kg, d'environ 700 µg/kg, d'environ 725 µg/kg, d'environ 750 µg/kg, d'environ 775 µg/kg, d'environ 800 µg/kg, d'environ 825 µg/kg, d'environ 850 µg/kg, d'environ 875 µg/kg, d'environ 900 µg/kg, d'environ 925 µg/kg, d'environ 950 µg/kg, d'environ 975 µg/kg, d'environ 1 mg/kg, d'environ 5 mg/kg, d'environ 10 mg/kg, d'environ 15 mg/kg, d'environ 20 mg/kg, d'environ 25 mg/kg, d'environ 30 mg/kg, d'environ 35 mg/kg, d'environ 40 mg/kg, d'environ 45 mg/kg, d'environ 50 mg/kg, d'environ 60 mg/kg, d'environ 70 mg/kg, d'environ 80 mg/kg, d'environ 90 mg/kg, d'environ 100 mg/kg, d'environ 125 mg/kg, d'environ 150 mg/kg, d'environ 175 mg/kg, d'environ 200 mg/kg de poids corporel par dose unitaire.

6. Composé destiné à être utilisé selon l'une quelconque des revendications 1 à 5, dans lequel la dose quotidienne du composé de Formule (I) ou d'un sel, ester, stéréoisomère, polymorphe, solvate, N-oxyde ou composé isotopiquement marqué acceptable pharmaceutiquement de celui-ci est destinée à être administrée en une seule fois ou est destinée à être administrée en deux, trois ou quatre doses.

7. Composé destiné à être utilisé selon l'une quelconque des revendications 1 à 6, dans lequel le composé de Formule (I) ou un sel, ester, stéréoisomère, polymorphe, solvate, N-oxyde ou composé isotopiquement marqué acceptable pharmaceutiquement de celui-ci est destiné à être administré en continu pendant au moins 3 jours, au moins 4 jours, au moins 5 jours, au moins 6 jours, au moins 7 jours, au moins 8 jours, au moins 9 jours, au moins 10 jours, au moins 11 jours, au moins 12 jours, au moins 13 jours, au moins 14 jours, au moins 15 jours, au moins 16 jours, au moins 17 jours, au moins 18 jours, au moins 19 jours, au moins 20 jours, au moins 21 jours, au moins 22 jours, au moins 23 jours, au moins 24 jours, au moins 25 jours, au moins 30 jours, au moins 35 jours, au moins 40 jours, au moins 45 jours, au moins 50 jours, au moins six mois, au moins 1 an, au moins 2 ans, au moins 3 ans, au moins 4 ans, ou au moins 5 ans.

8. Composé destiné à être utilisé selon l'une quelconque des revendications 1 à 7, dans lequel le composé de Formule (I) ou un sel, ester, stéréoisomère, polymorphe, solvate, N-oxyde ou composé isotopiquement marqué acceptable pharmaceutiquement de celui-ci est destiné à être administré pendant un ou plusieurs (par exemple, 1, 2, 3, 4, 5, 6, 7, 8, 9 ou 10) cycles de traitement, dans lequel chaque cycle de traitement dure au moins 3 jours, au moins 4 jours, au moins 5 jours, au moins 6 jours, au moins 7 jours, au moins 8 jours, au moins 9 jours, au moins 10 jours, au moins 11 jours, au moins 12 jours, au moins 13 jours, au moins 14 jours, au moins 15 jours, au moins 16 jours, au moins 17 jours, au moins 18 jours, au moins 19 jours, au moins 20 jours, au moins 21 jours, au moins 22 jours, au moins 23 jours, au moins 24 jours, au moins 25 jours, au moins 30 jours, au moins 35 jours, au moins 40 jours, au moins 45 jours ou au moins 50 jours ; et l'intervalle entre deux cycles de traitement est de 0, 1, 2, 3, 4, 5, 6, 7, 8, 9, 10 jours, deux semaines, trois semaines ou quatre semaines.

9. Composé destiné à être utilisé selon l'une quelconque des revendications 1 à 8, dans lequel le composé de Formule (I) ou un sel, ester, stéréoisomère, polymorphe, solvate, N-oxyde ou composé isotopiquement marqué acceptable pharmaceutiquement de celui-ci est destiné à être administré par injection (par exemple, injection intraveineuse, intra-artérielle, sous-cutanée, intrapéritonéale, intramusculaire, y compris goutte à goutte) ou par voie transdermique, ou est destiné à être administré par voie orale, buccale, nasale, transmuqueuse ou topique, sous forme de formulation ophtalmique ou par inhalation.

10. Composé destiné à être utilisé selon l'une quelconque des revendications 1 à 9, dans lequel le composé de Formule (I) ou un sel, ester, stéréoisomère, polymorphe, solvate, N-oxyde ou composé isotopiquement marqué acceptable pharmaceutiquement de celui-ci est destiné à être administré sous une forme posologique choisie dans le groupe constitué par un comprimé, une capsule, une pastille, un bonbon dur, une poudre, un vaporisateur, une émulsion, une crème, un baume, un suppositoire, un gel, une pâte, une lotion, une injection, une nanoformulation, un timbre, une suspension aqueuse, une solution, un élixir et un sirop.

11. Composé destiné à être utilisé selon l'une quelconque des revendications 1 à 10, dans lequel la toux est choisie dans le groupe constitué par une toux aiguë, une toux subaiguë, une toux chronique, une toux résistante au traitement, une toux chronique résistante au traitement, une toux chronique idiopathique, une toux post-virale, une toux iatrogène, une toux associée à un écoulement post-nasal, une toux associée au rhume, à une infections des voies respiratoires supérieures, à l'asthme, au cancer du poumon et/ou à la bronchopneumopathie chronique obstructive (BPCO), une toux associée à une maladie interstitielle, une toux associée à la fibrose pulmonaire, une toux associée au reflux gastro-œsophagien (GERD), une toux associée au tabagisme ou à une forme de bronchite, et l'hypersensibilité neuronale à l'origine d'une toux aiguë, subaiguë ou chronique ; de préférence, la toux est choisie dans le groupe constitué par une toux chronique, une toux résistante au traitement, une toux chronique résistante au traitement et une toux associée à la fibrose pulmonaire.
